(19) Europäisches Patentamt European Patent Office Office européen des brevets

(11) **EP 3 794 353 B1**

(12) **EUROPEAN PATENT SPECIFICATION**

(45) Date of publication and mention of the grant of the patent:
**09.11.2022 Bulletin 2022/45**

(21) Application number: **19727454.1**

(22) Date of filing: **14.05.2019**

(51) International Patent Classification (IPC):
**G01N 33/574** *(2006.01)*

(52) Cooperative Patent Classification (CPC):
**G01N 33/57415;** G01N 2800/52

(86) International application number:
**PCT/GB2019/051319**

(87) International publication number:
**WO 2019/220099 (21.11.2019 Gazette 2019/47)**

(54) **SPAG5 AS A BIOMARKER FOR BREAST CANCER TREATMENT**

SPAG5 ALS BIOMARKER ZUR BEHANDLUNG VON BRUSTKREBS

SPAG5 COMME BIOMARQUEUR POUR LE TRAITEMENT DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2018 GB 201807879**
**02.11.2018 GB 201817968**

(43) Date of publication of application:
**24.03.2021 Bulletin 2021/12**

(73) Proprietor: **Nottingham University Hospitals NHS Trust**
**Nottingham, Nottinghamshire NG5 1PB (GB)**

(72) Inventors:
• **ABDEL-FATAH, Tarek**
  **Nottingham NG5 1PB (GB)**
• **CHAN, Stephen**
  **Nottingham NG5 1PB (GB)**

(74) Representative: **Barker Brettell LLP**
**100 Hagley Road**
**Edgbaston**
**Birmingham B16 8QQ (GB)**

(56) References cited:
**US-A1- 2015 185 219**

• **ABDEL-FATAH TAREK M A ET AL: "SPAG5as a prognostic biomarker and chemotherapy sensitivity predictor in breast cancer: a retrospective, integrated genomic, transcriptomic, and protein analysis", THE LANCET ONCOLOGY, vol. 17, no. 7, 14 June 2016 (2016-06-14), pages 1004-1018, XP029625461, ELSEVIER, AMSTERDAM, NL ISSN: 1470-2045, DOI: 10.1016/S1470-2045(16)00174-1**
• **L-J YUAN ET AL: "SPAG5 upregulation predicts poor prognosis in cervical cancer patients and alters sensitivity to taxol treatment via the mTOR signaling pathway", CELL DEATH & DISEASE, vol. 5, no. 5, E1247, 1 May 2014 (2014-05-01), pages 1-8e1247, XP055607837, DOI: 10.1038/cddis.2014.222**

- **DATABASE EMBASE [Online] ELSEVIER SCIENCE PUBLISHERS, AMSTERDAM, NL; 1 May 2018 (2018-05-01), MOHAMED AHMED ABDEL-FATAH T ET AL: "Sperm associated antigen 5 (SPAG5) as a predictor and monitor for response and distant relapse risk (DRR) to endocrine (ET) and chemo-therapies (CT) in oestrogen receptor positive (ER+) breast cancer (BC)", XP002793140, Database accession no. EMB-625970468 & MOHAMED AHMED ABDEL-FATAH T ET AL: "Sperm associated antigen 5 (SPAG5) as a predictor and monitor for response and distant relapse risk (DRR) to endocrine (ET) and chemo-therapies (CT) in oestrogen receptor positive (ER+) breast cancer (BC)", JOURNAL OF CLINICAL ONCOLOGY 20180501 AMERICAN SOCIETY OF CLINICAL ONCOLOGY NLD, vol. 36, no. 15, Supplement 1, 20 May 2018 (2018-05-20), ISSN: 1527-7755**

**Description**

The present invention relates to methods for determining if an ER+ breast cancer is expected to respond to endocrine or chemotherapy treatment. Any references to methods of treatment by therapy or surgery are to be interpreted as references to compounds, pharmaceutical compositions and medicaments of the present invention for use in those methods, or to methods of selecting patients for said treatment.

Of the 1.38 million newly diagnosed breast cancer (BC) cases each year, 65-70% of them are oestrogen receptor positive (ER+). Although the single-agent endocrine therapy (ET), which aims to prevent ER signaling through either selective ER modulators (SERM) (e.g., tamoxifen) or aromatase inhibitors (AIs) has significantly extended survival of ER+BC patients, resistance to ET is common (up to 50%). To further extend treatment benefit and delay the development of ET resistance, a combination of ET with chemotherapy (CT) with or without other modulators e.g., mammalian target of rapamycin (mTOR) inhibitors has been proven to be an effective strategy. Offering cytotoxic chemotherapy (CT) in addition to ET can improve the clinical outcome in up to 30% of ER patients and therefore leaving the remainder both being over-treated and experiencing the associated burdens of side-effects.

Currently, there is no proven test which can predict response to either ET or CT accurately. The current practice is largely based on assessment of the recurrence risk (RR) and overall survival (OS) of the patient, using the traditional clinico-pathological prognostic factors (e.g., lymph node status (LN)) and the multi-gene tests (e.g., Oncotype DX® (Genomic Health, CA, USA), MammaPrint® (Agendia, CA, USA) and Prosigna® (Nanostring Technologies, WA, USA). Nevertheless, these tests do not predict if a patient will respond to the cancer therapy, i.e., ET or CT. This therefore makes it difficult for clinicians and the patients to determine the risk/benefit ratio associated with treating an individual with ET or CT. The "benefit" of the "risk/benefit ratio" being treatment of the cancer, whereas the "risk" is the possibility that it will not treat the cancer and the possibility of any side effects associated with using ET or CT. US 2015/185219 discloses SPAG5 as a biomarker for breast cancer treatment but not for choosing whether to select ET vs CT.

There is therefore a need for an improved means of determining if an individual is expected to respond to chemotherapy, endocrine therapy and/or other types of cancer therapy.

Disclosed herein but not claimed is a method for determining if a cancer of a subject is expected to respond to chemotherapy, the method comprising:

(i) determining if a cell in a sample of the cancer expresses granules of SPAG5 protein; and/or
(ii) determining if cells in a sample of the cancer overexpresses a *SPAG5* transcript;

wherein if the sample comprises a cell that expresses granules of SPAG5 protein and/or if the sample overexpresses the *SPAG5* transcript, the subject has a cancer that is expected to respond to chemotherapy;
wherein if the sample does not comprise a cell that expresses granules of SPAG5 protein and/or if cells in the sample does not overexpress the *SPAG5* transcript, the subject has a cancer that is not expected to respond to chemotherapy.

Surprisingly, when SPAG5 protein is overexpressed in a cancer, the SPAG5 displays intracytoplasmic granular (punctate) staining in the cancer cells.

Furthermore, cancer cells that comprise granules of SPAG5 protein and/or overexpress the SPAG5 transcript are surprisingly responsive to chemotherapy and resistant or less responsive to endocrine therapy (see Examples 14 and 37). It is believed that this is because cancers that overexpress SPAG5 protein and/or *SPAG5* transcript are rapidly growing and therefore respond better to non-specific therapies, such as chemotherapy which target cell division (growth). Since SPAG5 has an essential role in the progression of the cell cycle during the mitotic phase, the inventors believe that SPAG5 dysregulation could contribute to chromosome instability and aneuploidy, both of which are hallmarks of malignant cells and could confer vulnerability to chemotherapeutics drugs. SPAG5 protein is a microtubule-associated protein with dual localization to both centrosomes and kinetochores, and it is required for mitotic spindle formation and chromosome segregation. SPAG5 protein functions directly or indirectly in the microtubule-based vesicle transport that is essential for spindle formation during mitotic (M) phase. SPAG5 is upregulated in M-phase cells and binds to microtubules as a regulator of the timing of spindle organization and separation of sister chromatids. Thus, punctuate or granular staining pattern of SPAG5 protein may reflect over-production (overexpression or upregulation) of SPAG5-microtubule-based vesicle and excess spindle formation in M-phase leading to uncontrolled cell division.

The method may further comprise the step of determining if a cell in a sample of a tumour overexpresses HER2 protein or HER2 gene (HER2+). If a cell in a sample of the tumour is HER2+ and the sample comprises a cell that expresses granules of SPAG5 protein and/or the sample overexpresses the *SPAG5* transcript, the tumour is expected to respond to chemotherapy. Therefore, HER2 targeting agents, such as Trastuzumab, Pertuzumab and or Lapatinib may be deaccelerated or may not be needed. If the sample comprises a cell that is HER2+ and the sample does not comprise a cell that expresses granules of SPAG5 protein and/or does not overexpress the *SPAG5* transcript, the tumour is expected to respond to HER2 targeting agents and is not expected to respond to chemotherapy.

The method may further comprise the step of determining if a cell in a sample of a tumour expresses oestrogen receptor (ER+). If a cell in a sample of the tumour is ER+ and the sample comprises a cell that expresses granules of SPAG5 protein and/or cells in the sample overexpress the *SPAG5* transcript, the tumour is expected to respond to chemotherapy. If the sample comprises a cell that is ER+ and the sample does not comprise a cell that expresses granules of SPAG5 protein and/or the cells in the sample do not overexpress the *SPAG5* transcript, the tumour may be expected respond to endocrine therapy.

According to a first aspect of the invention, there is provided a method for determining if an ER+ breast cancer of a subject is expected to respond to endocrine therapy or chemotherapy, the method comprising:

(i) determining if a cell in a sample of the ER+ breast cancer expresses granular SPAG5 protein; or
(ii) determining if cells in a sample of the ER+ breast cancer overexpress a *SPAG5* transcript;

wherein if the sample does not comprise a cell that expresses granular SPAG5 protein and/or if cells in the sample do not overexpress *SPAG5* transcript, the subject has a cancer that may be expected to respond to endocrine therapy, and
wherein if the sample comprises a cell that expresses granular SPAG5 protein and/or if cells in the sample overexpress the *SPAG5* transcript, the subject has a cancer that may be expected to respond to chemotherapy, wherein the chemotherapy is one or more selected from the group consisting of: cisplatinum, taxane, cyclophosphamide, methotrexate, 5-fluorouracil, an anthracycline, epirubicin, doxorubicin and anthracycline-based combination chemotherapy,
wherein the endocrine therapy is tamoxifen and/or an aromatase inhibitor.

Advantageously, therefore, the method according to the first aspect is a fast and accurate means of determining whether an individual is likely to respond to endocrine therapy or chemotherapy.

Disclosed herein but not claimed is a method for determining if an ER+ cancer of a subject is expected to respond to endocrine therapy, the method comprising:

(i) determining if a cell in a test sample of the ER+ cancer expresses granular SPAG5 protein; or
(ii) determining if cells in the sample of the ER+ cancer overexpress a *SPAG5* transcript;

wherein if the sample does not comprise a cell that expresses granules of SPAG5 protein and/or if cells in the sample does not overexpress the *SPAG5* transcript, the subject has a cancer that may be expected to respond to endocrine therapy.

Disclosed herein but not claimed is a method for determining if an ER+ cancer of a subject may be expected to respond to chemotherapy, the method comprising:

determining if a cell in a sample of the ER+ cancer expresses granules of SPAG5 protein and/or determining if cells in the sample overexpress *SPAG5* transcript,
wherein if the sample comprises a cell that expresses granules of SPAG5 protein and/or if cells in the sample overexpress *SPAG5* transcript, the subject has a cancer that may be expected to respond to chemotherapy.

If the sample referred to herein does not comprise a cell that expresses granules of SPAG5 protein and/or the cells in sample do not overexpress the *SPAG5* transcript, the cancer may be expected to be resistant to chemotherapy. If the sample comprises a cell that expresses granules of SPAG5 protein and/or if cells in the sample overexpress the *SPAG5* transcript, the cancer may be expected to be resistant to endocrine therapy.

If the sample referred to herein comprises a cell that is ER+ but does not comprise a cell that expresses granules of SPAG5 protein or cells in the sample do not overexpress the *SPAG5* transcript, the sample may be taken from a cancer, which in combination with endocrine therapy, responds to a cancer therapy selected from the group comprising/consisting of: HER2 targeting therapy, such as Trastuzumab; taxane; and fluorouracil (5-FU). Preferably, the group does not comprise anthracycline, epirubicin or doxorubicin.

If the sample comprises a cell that is ER+ and comprises a cell that expresses granules of SPAG5 protein and/or if cells in the sample overexpress the *SPAG5* transcript, the tumour may be expected to respond to anthracycline-based combination chemotherapy and/or taxane. Anthracycline-based combination chemotherapy is chemotherapy which comprises epirubicin (E) or doxorubicin (A) and one or more other chemotherapy drugs, including cyclophosphamide (C) and fluorouracil (5-FU; F). Thus, Anthracycline-based combination chemotherapy may comprise AC, FAC, EC or FEC. If the sample comprises a cell that is ER+ and comprises a cell that expresses granules of SPAG5 protein and/or overexpresses the *SPAG5* transcript, the sample may be taken from a cancer, which in combination with chemotherapy, responds to a cancer therapy selected from the group consisting of: mTOR inhibitors, such as rapamycin and its analogs; AKT inhibitors, such as MK-2206 and GSK690693; PI3K inhibitors, such as LY294002; and CK4/6 inhibitors, such as LY2835219 and PD-0332991.

Surprisingly, ER+ cancers that are also devoid of cells that overexpress SPAG5 protein (i.e., granules of SPAG5 protein) or SPAG5 transcript are very responsive to endocrine therapy (and resistant or less responsive to chemotherapy). This may be because ER+ cancers that do not overexpress SPAG5 protein are slowly growing tumours and not as aggressive (as cancers that overexpress SPAG5 protein) and therefore respond better to hormonal therapies, which act via the oestrogen receptor (ER).

Advantageously, therefore, the method disclosed herein is a fast and accurate means of determining whether an individual is likely to respond or be resistant to chemotherapy and/or endocrine therapy. Consequently, clinicians are able to provide a more tailored treatment regime for individuals with an ER+ cancer because it takes the risk/benefit ratio of chemotherapy and endocrine therapy into consideration.

The method disclosed herein may not comprise the step of obtaining a sample from the subject. The method disclosed herein may be used to determine the most appropriate type of cancer therapy for a subject that has cancer.

Disclosed herein but not claimed is a method of determining the efficacy of treating a subject suffering from cancer with a cancer therapy, the method comprising:

(i) determining the percentage of cells that express granules of SPAG5 protein in a test sample of a cancer from a subject, and

(ii) comparing the percentage of cells that express granules of SPAG5 protein in the test sample with a percentage of cells that express granules of SPAG5 protein in a reference sample,

wherein if the percentage of cells that express granules of SPAG5 protein in the test sample is lower compared to the reference sample, it may be indicative of the efficacy of treating the subject with the cancer therapy, and

wherein if the percentage of cells that express granules of SPAG5 protein in the test sample is higher compared to the reference sample, it may be indicative of the inefficacy of treating the subject with the cancer therapy.

The reference sample may be a sample of the same cancer from the same subject at an earlier time point.

Preferably the determining step is performed using techniques such as flow cytometry, immunohistochemistry, Chromogenic in situ hybridization (CISH) or FISH (Fluorescence In-Situ Hybridization).

The cancer may be an ER+ cancer or a cancer that comprises cells that express the ER. The cancer may be a HER2+ cancer or a cancer that comprises cells that express the HER2.

Disclosed herein but not claimed is a method of determining the prognosis of a subject that has a cancer, the method comprising:

(i) determining if a cell in a sample from the cancer expresses granules of SPAG5 protein; and/or
(ii) determining if cells in a sample from the cancer overexpress a *SPAG5* transcript;

wherein if the sample comprises a cell that expresses granules of SPAG5 protein and/or if the sample overexpresses the *SPAG5* transcript, the subject has a poor prognosis.

The method may further comprise determining if a cell in the sample from the tumour of the subject is ER+, wherein if a cell in the sample from the tumour is ER+ and the cell expresses granules of SPAG5 protein and/or the cells in sample from the tumour overexpress the *SPAG5* transcript, the subject has a poor prognosis.

The method may further comprise determining if a cell in the sample from the tumour of the subject is HER2+, wherein if a cell in the sample from the tumour is HER2+ and the cell expresses granules of SPAG5 protein and/or the sample from the tumour overexpresses the *SPAG5* transcript, the subject has a poor prognosis.

A poor prognosis may be an increase in relapse risk at 5 or 10 years after receiving surgery and/or therapy for cancer compared to the relapse risk of a reference individual. The increased risk of relapse may be a 1.5-fold increase or greater, 2-fold increase or greater, or 3-fold increase or greater.

A poor prognosis may be an increased risk of death from cancer at 5 or 10 years after receiving cancer surgery and/or cancer therapy compared to the relapse risk of a reference individual. The increased risk of death may be a 1.5-fold increase or greater, 2-fold increase or greater, or 3-fold increase or greater.

A good prognosis may be a decrease in relapse risk at 5 or 10 years after receiving surgery and/or therapy for cancer compared to the relapse risk of a reference individual. The decreased risk of relapse may be a 1.5-fold decrease or greater, 2-fold decrease or greater, or 3-fold decrease or greater.

A good prognosis may be a decreased risk of death from cancer at 5 or 10 years after receiving cancer surgery and/or cancer therapy compared to the relapse risk of a reference individual. The decreased risk of death may be a 1.5-fold decreased or greater, 2-fold decreased or greater, or 3-fold decreased or greater.

The reference individual may be one or more individuals with a cancer that does not comprise granules of SPAG5 protein or that does not overexpress *SPAG5* transcript. The reference individual may be one or more individuals with a cancer that comprises granules of SPAG5 protein or that overexpress *SPAG5* transcript. Preferably the one or more reference individuals have the same form of cancer as the individual of referred to in the invention.

Disclosed herein but not claimed is a chemotherapy agent for use in treating cancer in a subject, wherein cells of the cancer comprise granules of SPAG5 protein or overexpress *SPAG5* transcript.

The subject may have a cancer that is ER+. The subject may have a cancer that is HER2+.

According to another aspect of the invention, there is provided an endocrine therapy for use in treating an ER+ breast cancer in a subject, wherein cancer cells of the cancer do not comprise granules of SPAG5 protein or do not overexpress *SPAG5* transcript, wherein the endocrine therapy is tamoxifen and/or an aromatase inhibitor.

Disclosed herein but not claimed is a method for determining if an HER2+ cancer of a subject is expected to respond to HER2 targeting therapy or to chemotherapy, the method comprising:

(i) determining if a cell in a test sample of the HER2+ cancer expresses granular SPAG5 protein; or

(ii) determining if the sample of the HER2+ cancer overexpresses a *SPAG5* transcript;

wherein if the sample does not comprise a cell that expresses granules of SPAG5 protein and/or if the sample does not overexpress the *SPAG5* transcript, the subject has a cancer that is expected to respond to HER2 targeting therapy and not to respond to chemotherapy.

Disclosed herein but not claimed is a method for determining if an HER2+ cancer of a subject is expected to respond to chemotherapy, the method comprising:

determining if a cell in a sample of the HER2 cancer expresses granules of SPAG5 protein and/or determining if the sample overexpresses *SPAG5* transcript,

wherein if the sample comprises a cell that expresses granules of SPAG5 protein and/or if the sample overexpresses *SPAG5* transcript, the subject has a cancer that is expected to respond to chemotherapy.

If a sample does not comprise a cell that expresses granules of SPAG5 protein and/or the sample does not overexpress the *SPAG5* transcript, the cancer may be expected to be resistant to chemotherapy. If the sample comprises a cell that expresses granules of SPAG5 protein and/or if the sample overexpresses the *SPAG5* transcript, the cancer may not be expected to respond to or benefit from adding HER2 target therapy in addition to chemotherapy.

If a sample comprises a cell that is HER2+ but does not comprise a cell that expresses granules of SPAG5 protein or the sample does not overexpress the *SPAG5* transcript, the sample may be taken from a cancer, which responds to HER2 targeting therapy, such as: Herceptin Pertuzumab and Lapatinib and/or taxane. The cancer may not respond to anthracycline, epirubicin or doxorubicin.

If the sample comprises a cell that is HER2+ and comprises a cell that expresses granules of SPAG5 protein and/or if the sample overexpresses the *SPAG5* transcript, the tumour may be expected to respond to anthracycline-based combination chemotherapy. Anthracycline-based combination chemotherapy is chemotherapy which comprises epirubicin (E) or doxorubicin (A) and one or more other chemotherapy drugs, including cyclophosphamide (C) and fluorouracil *(5-FU;* F). Thus, anthracycline-based combination chemotherapy may comprise AC, FAC, EC or FEC. If the sample comprises a cell that is HER2+, ER+ and comprises a cell that expresses granules of SPAG5 protein and/or overexpresses the *SPAG5* transcript, the sample may be taken from a cancer, which in combination with chemotherapy, responds to a cancer therapy selected from the group consisting of: mTOR inhibitors, such as rapamycin and its analogs; AKT inhibitors, such as MK-2206 and GSK690693; PI3K inhibitors, such as LY294002; and CK4/6 inhibitors, such as LY2835219 and PD-0332991.

Surprisingly, HER2+ cancers that are also devoid of cells that overexpress SPAG5 protein (i.e., granules of SPAG5 protein) or SPAG5 transcript are very responsive to HER2 target therapy (and resistant or less responsive to chemotherapy). This may be because HER2+ cancers that do not overexpress SPAG5 protein are slowly growing tumours and not as aggressive (as cancers that overexpress SPAG5 protein) and therefore respond better to HER2 target therapies, which act via the HER2 receptor.

Advantageously, therefore, the method disclosed herein is a fast and accurate means of determining whether an individual is likely to respond or be resistant to chemotherapy and/or HER2 targeting therapy. Consequently, clinicians are able to provide a more tailored treatment regime for individuals with an HER2+ cancer because it takes the risk/benefit ratio of chemotherapy and HER2 targeting therapy into consideration.

The method disclosed herein may not comprise the step of obtaining a sample from the subject. The method disclosed herein may be used to determine the most appropriate type of cancer therapy for a subject that has cancer.

Disclosed herein but not claimed is an HER2 target therapy for use in treating an HER2+ cancer in a subject, wherein cancer cells of the cancer do not comprise granules of SPAG5 protein or do not overexpress *SPAG5* transcript.

Disclosed herein but not claimed is a method of determining a prognosis of a subject diagnosed with a cancer that does not overexpress HER2 protein or HER2 gene (HER2-) after receiving neoadjuvant chemotherapy, the method comprising:

a) i) determining if a cell in a test sample of the cancer overexpresses a HER2 protein or a HER2 gene (HER2+) and the sample does not comprise a cell that expresses granular SPAG5 protein, or
ii) determining if a test sample of the cancer does not comprise a cell that overexpresses a HER2 protein or a HER2 gene (HER2-) and determining if the sample does not comprise a cell that expresses granular SPAG5 protein; and
b) i) wherein if a cell in a test sample of the cancer overexpresses a HER2 protein or HER2 gene (HER2+) and granular SPAG5 protein and the subject receives one year of HER2 targeting therapy, the subject is expected to have a poor prognosis, or

ii) wherein if a cell in a test sample of the cancer overexpresses HER2 protein or HER2 gene (HER2+) and the subject does not receive one year of adjuvant HER2 targeting therapy, the subject is expected to have a poor prognosis, or
iii) wherein if the test sample of the cancer does not comprise a cell that overexpresses a HER2 protein or a HER2 gene (HER2-) but does express granular SPAG5 protein, the subject is expected to have a poor prognosis.

Disclosed herein but not claimed is a method for determining a prognosis of a subject diagnosed with a cancer that does not overexpress HER2 protein or HER2 gene and does not express ER (HER2-/ER-) after receiving neoadjuvant chemotherapy and without receiving any further adjuvant therapy, the method comprising:

(i) determining if a test sample of the cancer does not comprise a cell that overexpresses HER2 protein or the HER2 gene (HER2-); and
(ii) determining if the test sample of the cancer from the same subject does not comprise a cell that expresses granular SPAG5 protein;

wherein if a cell in the sample of the cancer expresses granules of SPAG5 protein, the subject has a cancer that is expected to have a poor prognosis, or
wherein if the sample of the cancer does not comprise a cells that expresses granules of SPAG5 protein, the subject has a cancer that is expected to have a good prognosis.

Disclosed herein but not claimed is a method for determining a prognosis of a subject diagnosed with a cancer that does not overexpress HER2 protein or HER2 gene but expresses ER (HER2- /ER+) after receiving neoadjuvant chemotherapy followed by 5 years of endocrine therapy, the method comprising:

(i) determining if a test sample of the cancer does not comprise a cell that overexpresses HER2 protein or HER2 gene, and
(ii) determining if the test sample of the cancer from the same subject does not comprise a cell that expresses granular SPAG5 protein;

wherein if a cell in the sample of the cancer expresses granules of SPAG5 protein, the subject has a cancer that is expected to have a poor prognosis in spite of receiving 5 year endocrine therapy, or
wherein if the sample of the cancer does not comprise a cell that expresses granules of SPAG5 protein, the subject has a cancer that is expected to have a good prognosis after receiving 5 year endocrine therapy.

Disclosed herein but not claimed is a method for determining a prognosis of a subject diagnosed with a cancer that does overexpress HER2 protein or HER2 gene (HER2+) after receiving neoadjuvant chemotherapy with or without pre-operative HER2 target therapy and followed by one year of adjuvant single agent of HER2 targeting therapy and no need to add other HER2 target therapy (e.g., Pertuzumab), the method comprising:

(i) determining if a test sample of the residual cancer does not comprise a cell that overexpresses a HER2 protein or a HER2 gene (HER2-), or
(ii) determining if a cell in a test sample of the cancer after overexpresses a HER2 protein or a HER2 gene (HER2+) and determining if a cell in the sample does not express granular SPAG5 protein;

wherein if a cell in the sample of the cancer overexpresses a HER2 protein or a HER2 gene (HER2+) and granules of SPAG5 protein, the subject has a cancer that is expected to have a poor prognosis.

Disclosed herein but not claimed is a method for determining a prognosis of a subject diagnosed with a cancer that overexpresses HER2 protein or HER2 gene (HER2+) after receiving neoadjuvant chemotherapy and if they would benefit from shorter course of adjuvant HER2 target therapy (6 months vs., one year), the method comprising:

(i) determining if a test sample of the residual cancer does not comprise a cell that overexpresses a HER2 protein or the HER2 gene (HER2-), or
(ii) determining if a cell in a test sample of the cancer overexpresses a HER2 protein or the HER2 gene (HER2+) and does not express granular SPAG5 protein;

wherein if a cell in the test sample of the cancer overexpresses a HER2 protein or a HER2 gene (HER2+) and granules of SPAG5 protein, the subject has a cancer that is expected to have a poor prognosis and would benefit from one year course of combined HER2 target therapy (e.g., Herceptin plus Pertuzumab).

Disclosed herein but not claimed is a method for determining a prognosis of a subject diagnosed with a cancer that overexpresses HER2 protein or HER2 gene but does not express ER (HER2+ /ER-) after receiving neoadjuvant chemotherapy and one year of adjuvant HER2 target therapy and if they would benefit from adding other HER2 target agents or additional immunotherapy, the method comprising:

determining if a cell in a test sample of the cancer overexpresses the HER2 protein or the HER2 gene (HER2+) and expresses granules of SPAG5 protein;
wherein if the test sample of the cancer (i) does not comprise a cell that overexpresses the HER2 protein or the HER2 gene (HER2-), or (ii) comprises a cell that overexpresses the HER2 protein or the HER2 gene (HER2+) but does not express granular SPAG5 protein, the subject has a cancer that is expected to have a good prognosis.

A subject with a good prognosis may not require any other forms of treatment.

The sample or cell(s) according to the invention may be taken from a (residual) tumour of a subject who has received chemotherapy.

Disclosed herein but not claimed is a method for determining a prognosis of a subject diagnosed with a cancer that overexpresses HER2 protein or HER2 gene and expresses ER (HER2+/ER+) after receiving neoadjuvant chemotherapy followed by 5 years of adjuvant endocrine therapy in addition to one year of adjuvant HER2 target therapy, the method comprising:

(i) determining if a cell in a test sample of the cancer overexpresses a HER2 protein or a HER2 gene (HER2+), and
(ii) determining if a test sample of the cancer from the same subject does not comprise a cell that expresses granular SPAG5 protein; wherein if a cell in a test sample of the cancer expresses granules of SPAG5 protein, the subject has a cancer that is expected to have a poor prognosis in spite of receiving 5 year endocrine therapy and one year of HER2 target therapy.

The sample or cell(s) may be taken from a (residual) tumour of a subject who has received chemotherapy.

If the subject is expected to have a poor prognosis, they may benefit from receiving other therapeutic treatments, such as CDK4/6 and or adding other HER2-targeting agents.

Disclosed herein but not claimed is a method for stratification and predicting the risk of relapse of a cancer after receiving neoadjuvant therapy followed by (if eligible) adjuvant endocrine therapy for 5 years and or adjuvant HER2 Target therapy by re-valuating and retesting a number of radiological and histopathological parameters in a sample of the residual cancer after receiving neoadjuvant chemotherapy from the same individual including HER2, SPAG5, MRI volumetric measurements, lympho-vascular invasion, histopathological grade and chemotherapy related fibrotic reaction; the method comprising:

(i) determining if a sample of the cancer taken after neoadjuvant chemotherapy does not change HER2 expression (negative to positive or positive to negative),
(ii) determining if a sample of the cancer taken from the subject after receiving neoadjuvant chemotherapy does not express granules of SPAG5 protein,
(iii) determining by using MRI volumetric measurements before and after receiving neoadjuvant if the volume of the tumour of the subject has decreased by equal or more than 30%,
(iv) determining if the histopathological grade of a subject before starting the neoadjuvant chemotherapy is not high grade,
(v) determining if there is no the lympho-vascular invasion in the post chemotherapy residual tumour sample, and
(vi) determining if there is chemotherapy related histopathological fibrotic reaction in the post chemotherapy residual tumour sample.

Wherein if after receiving neoadjuvant chemotherapy i) there is no granules of SPAG5 protein expression in a cell of a sample of residual tumour, and ii) there is no Lymphovascular invasion in the residual cancer and (iii) there is a reduction in the tumour volume measured by MRI equal to or more than 30% and (iv) the tumour is of low or intermediate histological grade and (v) there is a chemotherapy related histological fibrotic reaction in the residual tissue, the subject has a residual cancer that is expected to have a low risk of relapse, or

wherein if after receiving neoadjuvant chemotherapy i) there is granules of SPAG5 protein expression in a cell of a sample of residual tumour, or ii) there is Lymphovascular invasion in the residual cancer or (iii) there is a reduction in the tumour volume measured by MRI less than 30% or (iv) the tumour is of low high histological grade or (v) there is no a chemotherapy related histological fibrotic reaction in the residual tissue, the subject has a residual cancer that is expected to have high risk of relapse.

Preferably, the repetition of step (i) to (vi) will occur after failure of disappearance of cancer cells after receiving neoadjuvant chemotherapy.

Disclosed herein but not claimed is a method of calculating the 5 year predicted risk of relapse of a tumour subject who has no changes in HER2 expression status after receiving neoadjuvant chemotherapy, optionally followed by adjuvant endocrine therapy for 5 years and or adjuvant HER2 Target therapy, the method comprising:

(i) determining the relapse risk of SPAG5 protein expression after the subject has been treated with the neoadjuvant cancer therapy and before starting the adjuvant systemic therapy by giving a score of 0 if there is an absence of granular SPAG5 protein expression in the post chemotherapy sample, or a score of 3 if there is a presence of granular SPAG5 protein expression in the post chemotherapy sample;

(ii) determining the relapse risk of MRI volumetric changes after the subject has been treated with the neoadjuvant cancer therapy and before starting the adjuvant systemic therapy by giving a score of 0 if the volume of a tumour is changed by less than 30%, or 1 if the volume a tumour is changed by more than 30%;

(iii) determining the relapse risk of the histopathological grade of a subject before starting the neoadjuvant chemotherapy by giving a score of 0 if the histopathological grade is low or intermediate, or 3 if histopathological grade is high;

(iv) determining the relapse risk of lympho-vascular invasion after the subject has been treated with the neoadjuvant cancer therapy and before starting the adjuvant systemic therapy by giving a score of 0 if there is an absence of lympho-vascular invasion in the post chemotherapy sample, or 3 if there is a presence of lympho-vascular invasion in the post chemotherapy sample;

(v) determining the relapse risk of chemotherapy related histopathological fibrotic reaction after the subject has been treated with the neoadjuvant cancer therapy and before starting the adjuvant systemic therapy, wherein a score of 0 is given if there is a fibrotic reaction in the post chemotherapy sample, or a score of 3 is given if there is an absence of a fibrotic reaction in the post chemotherapy sample;

(vi) adding the scores of (i) to (v) together to give a (sum) score that allows relapse risk to be predicted.

An excellent prognosis (relapse risk <10%) is given if the sum of the relapse risk is 0 to 3; a good prognosis (relapse risk >=10% up to 25%) is given if the sum of the relapse risk is 4 to 6; an intermediate prognosis (relapse risk >=25% up to 50%) is given if the sum of the relapse risk is 7 to 9; and a poor prognosis (relapse risk >50%) is given if the sum of the relapse risk is 10 to 11.

Advantageously, therefore, the method disclosed herein is a fast and accurate means of determining whether an individual is likely to benefit from HER2 targeting therapy and or endocrine therapy. Consequently, clinicians are able to provide a more tailored treatment regime for individuals with a cancer because it takes the risk/benefit ratio of chemotherapy, HER2 targeting therapy and endocrine therapy into consideration.

The method disclosed herein may not comprise the step of obtaining a sample from the subject. The method disclosed herein may be used to determine the most appropriate type of cancer therapy for a subject that has cancer.

Disclosed herein but not claimed is a method of determining the prognosis of a subject with patient, the method comprising determining if a test sample from a subject with a tumour, which has not been treated with systemic therapy, is HER2- and/or determining if a test sample of the tumour in a subject, after being treated with anthracycline based combination chemotherapy with or without Taxane, is HER2+,

wherein if the sample that has not been treated with systemic therapy is HER2- and after chemotherapy a cell in a sample expresses HER2, the tumour/subject is expected to have a good prognosis (the 5 year recurrence risk is less than 25%) if receiving HER2 targeting agents (e.g., Trastuzumab, Pertuzumab and or Lapatinib), or

wherein if the sample that has not been treated with systemic therapy is HER2- and after chemotherapy the sample does not express HER2 but comprises granules of SPAG5 protein, the tumour/subject is expected to have a poor prognosis (the 5 year recurrence risk is more than 50%),

wherein if a cell in a sample that has not been treated with systemic therapy is HER2- and after chemotherapy the sample does not express HER2 (HER2-) and or granules of SPAG5 protein, the tumour/subject is expected to have intermediate prognosis (the 5 year recurrence risk is more than 25% but less than 50%).

Disclosed herein but not claimed is a method determining the prognosis of a subject with cancer, the method comprising determining if a test sample of a tumour from a subject with a tumour, which has not received any systemic therapy, is HER2+ and determining if a test sample of the tumour in a subject after being treated with anthracycline based combination chemotherapy with or without Taxane with or without HER2 targeting agents (e.g., Trastuzumab, Pertuzumab and or Lapatinib), is converted into HER2-,

wherein if the test sample of the tumour that has not been treated with any therapy is HER2+ and after chemotherapy does not express HER2 (HER2-), the tumour/subject is expected to have an good prognosis (the 5 year recurrence risk is less than 25%) if receiving HER2 targeting agents (e.g., Trastuzumab, Pertuzumab and or Lapatinib), wherein if the test sample of the tumour that has not been treated with any therapy is HER2+ and after chemotherapy comprises a cell that overexpresses HER2 and has granules of SPAG5 protein, the tumour/subject is expected to have a poor prognosis (the 5 year recurrence risk is less than 50%) after receiving adjuvant HER2 targeting agents (e.g., Trastuzumab, Pertuzumab and or Lapatinib), wherein if the test sample of the tumour that has not been treated with any therapy is HER2+ and after chemotherapy therapy the sample comprises a cell that shows HER2 overexpression/amplification (HER2+) but has no granules of SPAG5 protein, the tumour/subject is expected to have a good prognosis.

Disclosed herein but not claimed is a method for determining if a HER2- cancer of a subject, prior to treatment with systemic therapy, is expected to benefit from adjuvant HER2 target therapy after having received neoadjuvant chemotherapy, the method comprising:

(i) re-testing HER2 status of a post neoadjuvant sample to determine if, after receiving neoadjuvant chemotherapy, a cell in a sample of the same cancer subject expresses HER2 protein (HER2+) or gene, wherein if the HER2 status of a sample taken prior neoadjuvant therapy changes from negative, to positive in a sample taken post neoadjuvant therapy, the subject has a cancer that is expected to benefit from additional adjuvant HER2 target therapy (Trastuzumab).

Disclosed herein but not claimed is a method for determining if a HER2- cancer of a subject is expected to have a good prognosis after receiving neoadjuvant chemotherapy, the method comprising:

determining if a cell in a sample taken from the same cancer subject after chemotherapy expresses HER2 protein or the HER2 gene and this subject subsequently receives one year of adjuvant HER2 target therapy (Trastuzumab); wherein if a cell in the sample of the same cancer subject expresses HER2 protein or the HER2 gene but does not subsequently receive adjuvant HER2 target therapy (Trastuzumab), the cancer subject is expected to have a poor prognosis.

Disclosed herein but not claimed is a method of determining if a subject with cancer, prior to treatment with systemic therapy HER2-, is expected to have a good prognosis after receiving neoadjuvant chemotherapy, the method comprising:

determining the HER2 status of a sample taken from a cancer subject after neoadjuvant chemotherapy, wherein if the sample does not express HER2 protein (HER2-) or the HER2 gene, determining if a sample taken from the subject after receiving neoadjuvant chemotherapy does not expresses granules of SPAG5 protein, wherein if the sample comprises a cell that expresses granules of SPAG5 protein, the subject is expected to have a poor prognosis.

A poor prognosis may be an increased risk of relapse at 5 or 10 years after receiving surgery and/or cancer therapy for cancer compared to the relapse risk of a reference individual, or wherein a poor prognosis may be an increased risk of death from cancer at 5 or 10 years after receiving cancer surgery and/or cancer therapy compared to the relapse risk of a reference individual.
The increased risk of relapse or the increased risk of death may be a 1.5-fold increase or greater, 2-fold increase or greater, or 3-fold increase or greater.
The reference individual may be one or more individuals with a cancer that has achieved complete disappearance of cancer cells in post chemotherapy samples after receiving neoadjuvant chemotherapy.
SPAG5 is an important oncogene that not only plays an essential role in the formation and progression of human cancers, but also determines their clinical behaviour by regulating mitosis, cell cycle checkpoint and apoptosis.

The sequence of the SPAG5 protein may be 1193 amino acids long (Q96R06), and is referred to herein as SEQ ID NO. 1, as follows:

```
MWRVKKLSLS   LSPSPQTGKP   SMRTPLRELT   LQPGALTNSG   KRSPACSSLT   PSLCKLGLQE
GSNNSSPVDF   VNNKRTDLSS   EHFSHSSKWL   ETCQHESDEQ   PLDPIPQISS   TPKTSEEAVD
PLGNYMVKTI   VLVPSPLGQQ   QDMIFEARLD   TMAETNSISL   NGPLRTDDLV   REEVAPCMGD

RFSEVAAVSE   KPIFQESPSH   LLEESPPNPC   SEQLHCSKES   LSSRTEAVRE   DLVPSESNAF
LPSSVLWLSP   STALAADFRV   NHVDPEEEIV   EHGAMEEREM   RFPTHPKESE   TEDQALVSSV
EDILSTCLTP   NLVEMESQEA   PGPAVEDVGR   ILGSDTESWM   SPLAWLEKGV   NTSVMLENLR
QSLSLPSMLR   DAAIGTTPFS   TCSVGTWFTP   SAPQEKSTNT   SQTGLVGTKH   STSETEQLLC
GRPPDLTALS   RHDLEDNLLS   SLVILEVLSR   QLRDWKSQLA   VPHPETQDSS   TQTDTSHSGI
TNKLQHLKES   HEMGQALQQA   RNVMQSWVLI   SKELISLLHL   SLLHLEEDKT   TVSQESRRAE
TLVCCCFDLL   KKLRAKLQSL   KAEREEARHR   EEMALRGKDA   AEIVLEAFCA   HASQRISQLE
QDLASMREFR   GLLKDAQTQL   VGLHAKQEEL   VQQTVSLTST   LQQDWRSMQL   DYTTWTALLS
RSRQLTEKLT   VKSQQALQER   DVAIEEKQEV   SRVLEQVSAQ   LEECKGQTEQ   LELENSRLAT
DLRAQLQILA   NMDSQLKELQ   SQHTHCAQDL   AMKDELLCQL   TQSNEEQAAQ   WQKEEMALKH
MQAELQQQQA   VLAKEVRDLK   ETLEFADQEN   QVAHLELGQV   ECQLKTTLEV   LRERSLQCEN
LKDTVENLTA   KLASTIADNQ   EQDLEKTRQY   SQKLGLLTEQ   LQSLTLFLQT   KLKEKTEQET
LLLSTACPPT   QEHPLPNDRT   FLGSILTAVA   DEEPESTPVP   LLGSDKSAFT   RVASMVSLQP
AETPGMEESL   AEMSIMTTEL   QSLCSLLQES   KEEAIRTLQR   KICELQARLQ   AQEEQHQEVQ
KAKEADIEKL   NQALCLRYKN   EKELQEVIQQ   QNEKILEQID   KSGELISLRE   EVTHLTRSLR
RAETETKVLQ   EALAGQLDSN   CQPMATNWIQ   EKVWLSQEVD   KLRVMFLEMK   NEKEKLMIKF
QSHRNILEEN   LRRSDKELEK   LDDIVQHIYK   TLLSIPEVVR   GCKELQGLLE   FLS
```

[SEQ ID NO. 1]

Therefore, the SPAG5 protein may comprise an amino acid sequence substantially as set out in SEQ ID No. 1, or a variant or a fragment thereof.

A sample as referred to herein may comprise one or more cells from a subject with cancer, preferably cancer cells (not healthy/non-cancer cells). The one or more cells of the sample may comprise granular SPAG5 protein (i.e., granules of SPAG protein) and/or SPAG5 protein in a diffuse or non-granular form, such that it would have a diffuse staining pattern if, for example, it was stained using immunohistochemistry. The SPAG5 protein may comprise an amino acid sequence substantially as set out in SEQ ID No. 1, or a variant or a fragment thereof.

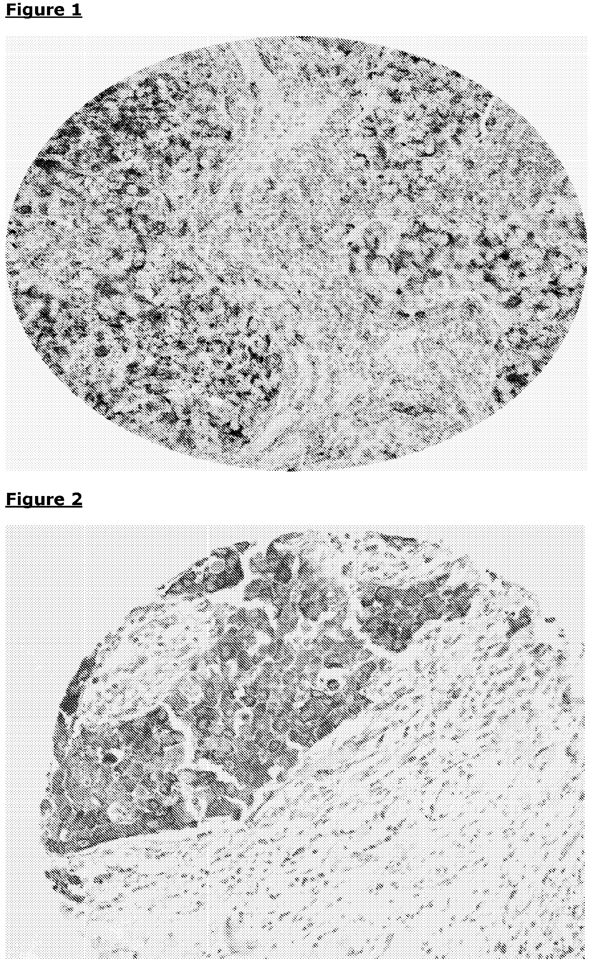

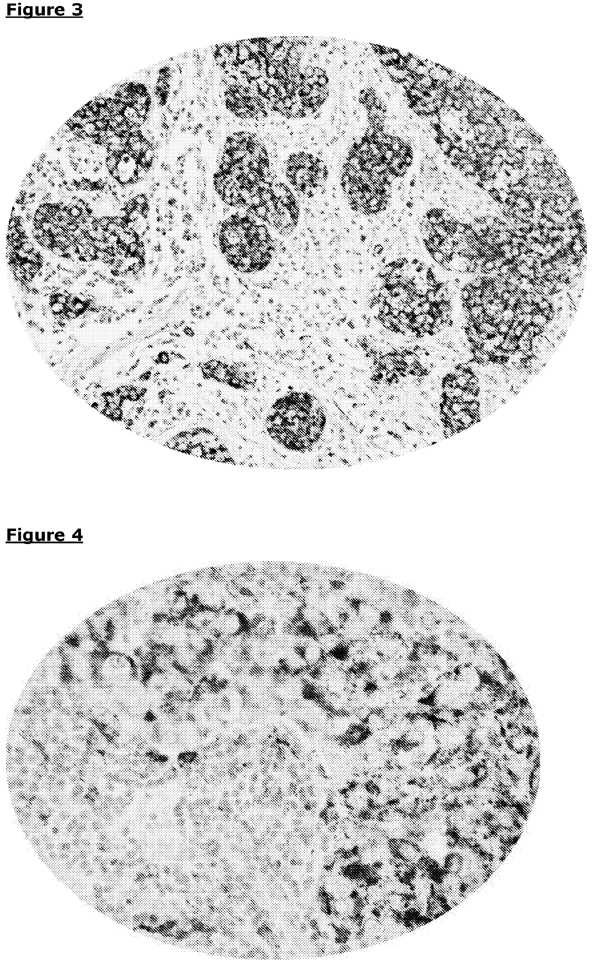

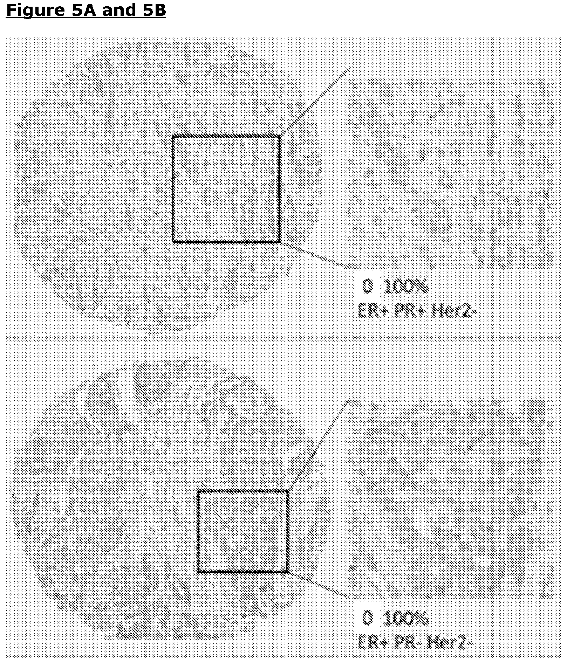

Granular SPAG5 protein refers to SPAG5 protein that displays a punctate intracytoplasmic staining pattern, such as spots or dots of SPAG5 protein. Granular SPAG5 protein may be one or more granules of SPAG5 protein. Granular SPAG5 protein does not occupy the entire cytoplasm of a cell. Granular SPAG5 protein only occupies a fraction of the cell's cytoplasm (see, for example, Figures 1, 2, 3 and 4). Non-granular SPAG protein is either a complete absence of any staining (no protein) or the presence of an evenly diffuse staining of SPAG5 protein throughout the cytoplasm of a cell (see, for example, Figures 5A and 5B). The cluster(s)/granules of SPAG5 protein may be any shape, including circular, elliptical or polygonal.

A cancer that is "ER+" or *"ER positive"* refers to a cancer that comprises cells which express the oestrogen receptor (ER). The sequence of the oestrogen receptor (ER) protein may be 595 amino acids long (P03372), and is referred to herein as SEQ ID NO. 2, as follows:

```
MTMTLHTKAS  GMALLHQIQG  NELEPLNRPQ  LKIPLERPLG  EVYLDSSKPA

VYNYPEGAAY  EFNAAAAANA  QVYGQTGLPY  GPGSEAAAFG  SNGLGGFPPL

NSVSPSPLML  LHPPPQLSPF  LQPHGQQVPY  YLENEPSGYT  VREAGPPAFY

RPNSDNRRQG  GRERLASTND  KGSMAMESAK  ETRYCAVCND  YASGYHYGVW

SCEGCKAFFK  RSIQGHNDYM  CPATNQCTID  KNRRKSCQAC  RLRKCYEVGM

MKGGIRKDRR  GGRMLKHKRQ  RDDGEGRGEV  GSAGDMRAAN  LWPSPLMIKR

SKKNSLALSL  TADQMVSALL  DAEPPILYSE  YDPTRPFSEA  SMMGLLTNLA

DRELVHMINW  AKRVPGFVDL  TLHDQVHLLE  CAWLEILMIG  LVWRSMEHPG

KLLFAPNLLL  DRNQGKCVEG  MVEIFDMLLA  TSSRFRMMNL  QGEEFVCLKS

IILLNSGVYT  FLSSTLKSLE  EKDHIHRVLD  KITDTLIHLM  AKAGLTLQQQ

HQRLAQLLLI  LSHIRHMSNK  GMEHLYSMKC  KNVVPLYDLL  LEMLDAHRLH

APTSRGGASV  EETDQSHLAT  AGSTSSHSLQ  KYYITGEAEG  FPATV
```

[SEQ ID NO. 2]

Therefore, the oestrogen receptor protein may comprise an amino acid sequence substantially as set out in SEQ ID No. 2, or a variant or a fragment thereof.

The cancer according to the invention is breast cancer. Other cancers disclosed but not claimed include lung cancer; ovarian cancer; gastric cancer; mesothelioma; Malignant Pleural Mesothelioma; uveal melanoma; melanoma; non-melanoma skin cancer; renal cancer; cholangiocarcinomas; cancer of the pleura; abdominal cancer; peritoneal cancer; cancer of the pericardium; head and neck cancers; brain cancer; liver cancer; biliary tract cancer; gastrointestinal cancers, including upper and lower tracts; urothelial cancer; prostate cancer; testicular cancer; cancer of the tunica vaginalis; cervical cancer, sarcoma; lymphoma; and leukaemia.

The cancer may or may not be metastatic.

The inventors have found that SPAG5 is not only a biomarker of proliferation but also an important genetic driver which is commonly amplified in highly proliferative "luminal B" and HER2+ BCs. Therefore, most preferably the cancer is breast cancer. More preferably, the breast cancer is a luminal B and/or HER2+ breast cancer.

A cancer that is *"HER2+"* or *"HER2 positive"* refers to a cancer that comprises cells that express the HER2 (Human Epidermal Growth Factor) receptor. The sequence of the HER2 receptor may be 1255 amino acids long (P04626), and is referred to herein as SEQ ID NO. 3, as follows:

MELAALCRWG LLLALLPPGA ASTQVCTGTD MKLRLPASPE THLDMLRHLY

QGCQVVQGNL ELTYLPTNAS LSFLQDIQEV QGYVLIAHNQ VRQVPLQRLR

IVRGTQLFED NYALAVLDNG DPLNNTTPVT GASPGGLREL QLRSLTEILK

GGVLIQRNPQ LCYQDTILWK DIFHKNNQLA LTLIDTNRSR ACHPCSPMCK

GSRCWGESSE DCQSLTRTVC AGGCARCKGP LPTDCCHEQC AAGCTGPKHS

DCLACLHFNH SGICELHCPA LVTYNTDTFE SMPNPEGRYT FGASCVTACP

YNYLSTDVGS CTLVCPLHNQ EVTAEDGTQR CEKCSKPCAR VCYGLGMEHL

REVRAVTSAN IQEFAGCKKI FGSLAFLPES FDGDPASNTA PLQPEQLQVF

ETLEEITGYL YISAWPDSLP DLSVFQNLQV IRGRILHNGA YSLTLQGLGI

SWLGLRSLRE LGSGLALIHH NTHLCFVHTV PWDQLFRNPH QALLHTANRP

EDECVGEGLA CHQLCARGHC WGPGPTQCVN CSQFLRGQEC VEECRVLQGL

PREYVNARHC LPCHPECQPQ NGSVTCFGPE ADQCVACAHY KDPPFCVARC

PSGVKPDLSY MPIWKFPDEE GACQPCPINC THSCVDLDDK GCPAEQRASP

LTSIISAVVG ILLVVLGVV FGILIKRRQQ KIRKYTMRRL LQETELVEPL

TPSGAMPNQA QMRILKETEL RKVKVLGSGA FGTVYKGIWI PDGENVKIPV

AIKVLRENTS PKANKEILDE AYVMAGVGSP YVSRLLGICL TSTVQLVTQL

MPYGCLLDHV RENRGRLGSQ DLLNWCMQIA KGMSYLEDVR LVHRDLAARN

VLVKSPNHVK ITDFGLARLL DIDETEYHAD GGKVPIKWMA LESILRRRFT

```
HQSDVWSYGV  TVWELMTFGA  KPYDGIPARE  IPDLLEKGER  LPQPPICTID

VYMIMVKCWM  IDSECRPRFR  ELVSEFSRMA  RDPQRFVVIQ  NEDLGPASPL

DSTFYRSLLE  DDDMGDLVDA  EEYLVPQQGF  FCPDPAPGAG  GMVHHRHRSS

STRSGGGDLT  LGLEPSEEEA  PRSPLAPSEG  AGSDVFDGDL  GMGAAKGLQS

LPTHDPSPLQ  RYSEDPTVPL  PSETDGYVAP  LTCSPQPEYV  NQPDVRPQPP

SPREGPLPAA  RPAGATLERP  KTLSPGKNGV  VKDVFAFGGA  VENPEYLTPQ

GGAAPQPHPP  PAFSPAFDNL  YYWDQDPPER  GAPPSTFKGT  PTAENPEYLG

LDVPV
```

[SEQ ID NO. 3]

Luminal tumour cells look like those of breast cancers that start in the inner (luminal) cells lining the mammary ducts. A *"luminal B"* cancer refers to a breast cancer that tends to be oestrogen-receptor positive. A *"luminal* B" cancer may be HER2+ or HER2-. A *"luminal A"* cancer refers to a breast cancer that is oestrogen-receptor positive and HER2 negative.

*"LN+"* or *"LN positive"* may mean that a cancer has spread to the lymph nodes, whereas "LN-" or "LN negative" means that a cancer has not spread to the lymph nodes.

The term *"respond"* or *"responsive"* means a cancer that can be treated by a particular cancer therapy, such as chemotherapy or endocrine therapy. A cancer may be considered treated if, for example, cancer cells have been killed, a tumour has been shrunk, replication of cancer cells has been halted, reduced or growth of a tumour has been stopped. The term *"resistant"* means a cancer that cannot be treated by a particular cancer therapy, such as chemotherapy or endocrine therapy.

The term *"express(es)"* or *"expression of"* can refer to the translation of a relevant DNA or RNA molecule into a polypeptide or protein sequence (such as SPAG5 protein or oestrogen receptor protein). The polypeptide or protein may be present within any compartment of the cell, may be present on a cell surface or may be secreted from a cell. This term may also refer to the transcription of DNA, which may be the production of transcripts, such as mRNA or cDNA, based on DNA. Thus, expression of a gene may be determined by measuring the amount of a transcript. *"Overexpresses",* as referred to herein, can refer to increased expression of a protein compared to the expression of the same protein (e.g. SPAG5) in normal (healthy/non-cancerous) individuals or it can refer to increased transcription of a gene into a transcript (e.g. mRNA or cDNA, such as that encoding SPAG5) compared the transcription of the same gene in normal (healthy/non-cancerous) individuals. *"Overexpresses"* may also refer to quantified expression, in a test sample, of the *SPAG5* gene, such that it has a value of greater than 1 after being normalised (with respect to relevant databases) and median centred (such that the values fall in a range of 0 to 1 with the media at the centre).

SPAG5 gene expression is normalised and median centred by:

1) calculating the arithmetic mean of *SPAG5* gene expression for samples recorded in the relevant databases;
2) calculating the standard deviation of *SPAG5* gene expression for samples recorded in the relevant databases; and
3) normalising, for each test sample, the *SPAG5* expression using the following equation:

$$\text{(Test sample SPAG5 gene expression value} - \text{the arithmetic mean of } SPAG5 \text{ gene expression)} / \text{the standard deviation of } SPAG5 \text{ gene expression.}$$

4) To median centre the normalised SPAG5 expression values, the following equation was used:

[Normalized SPAG5 expression value of the test sample -

minimal normalized value of SPAG5 among database samples] /

[maximum normalized expression value of SPAG5 among the database samples -

minimal normalized expression value of SPAG5 among the database samples]

The test sample according to the invention may be the sample from the subject referred to herein.

The relevant databases may be one or more of the following databases:

| Database | Platform | | | |
|---|---|---|---|---|
| GSE33143 | [HG_U95Av2] | AFFYMETRIX | GPL8300 |
| GSE20685 | HG-UA133-PLUS2 | AFFYMETRIX | GPL570 |
| GSE21653 | HG-UA133-PLUS2 | AFFYMETRIX | GPL570 |
| H_v2.1.1 55K | SWEGENE | | GPL5345 |
| GSE12093 | HG-UA133A | AFFYMETRIX | GPL96 |
| GSE31448 | HG-UA133-PLUS2 | AFFYMETRIX | GPL570 |
| GSE42568 | HG-UA133-PLUS2 | AFFYMETRIX | GPL570 |
| GSE45255 | HG-UA133A | AFFYMETRIX | GPL96 |
| GSE9893 | MLRG Human 21K V | Human Qiagen | GPL5049 |
| GSE22219 | Expression BeadC | ILLUMINA | GPL6098 |
| GSE10510 | OLIG0-ARRAY-35K | DKFZ | GPL6486 |
| GSE11121 | HG-UA133A | AFFYMETRIX | GPL96 |
| GSE7390 | HG-UA133A | AFFYMETRIX | GPL96 |
| GSE2034 | HG-UA133A | AFFYMETRIX | GPL96 |
| GSE6532-GSE9195 | HG-UA133-PLUS2 | AFFYMETRIX | GPL570 |

| Database | Platform |
|---|---|
| GSE34138 | (HumanWG-6 v3.0- ILLUMINA; GPL6884) |
| GSE22226 | (Agilent-014850-Agilent; GPL1708) |
| GSE6861 | (HG-U133_X3P-Affymetrix; GPL1352) |
| GSE41998 | (HG-U133A_2-Affymetrix; GPL571) |
| GSE32646 | (HG-U133_Plus_2-Affymetrix; GPL570) |
| GSE66399 | (HG-U133_Plus_2- Affymetrix; GPL570) |
| GSE50948 | (HG-U133_Plus_2 Affymetrix; GPL570) |
| GSE20271 | (HG-U133A-Affymetrix; GPL96) |
| GSE42822 | (HG-U133A-Affymetrix; GPL96) |
| GSE37946 | (HG-U133A-Affymetrix; GPL96) |
| GSE20194 | (HG-U133A-Affymetrix; GPL96) |
| GSE25066 | (HG-U133A-Affymetrix; GPL96) |

Preferably, the first 15 databases are used for determining the association between SPAG5 transcript and the distant relapse risk (DRR) after receiving adjuvant systemic therapy.

Preferably, the last 12 databases are used for determining the association between SPAG5 transcript expression and pathological complete response (pCR). More preferably, the last 12 databases are used for determining the association between SPAG5 transcript expression and pathological complete response (pCR) after receiving pre-operative cancer treatment. Most preferably, the preoperative cancer treatment is anthracycline based combination chemotherapy with or without Taxane and with or without Herceptin®.

SPAG5 gene expression data of each database were converted to a common scale (median equal to 0 and standard deviation equal to 1) in order to merge all of the study data that used the same platform and to create combined cohorts. To convert to a common scale each sample in the data was normalised to a mean of 0 and standard deviation of 1. Then the data was median centred for each gene whereby median of each gene is 0 (see steps 1 to 4 above). Databases using same platform have been merged and the median expression was calculated. The median expression of SPAG5 transcript for each platform has been calculated and values equal to or higher than the median coded as +1 (overexpression). Values of less than the median have been coded 0 or low SPAG5.

Overexpression of a gene may be an increase in expression of the gene in a sample in comparison to a "normal" or reference concentration. The increase may be an increase of 10% or more, 20% or more, 30% or more, 40% or more, 50% or more, 60% or more, 70% or more, 80% or more, 90% or more or 100% or more.

The "normal" or reference concentration may be obtained from control samples (i.e., samples from subject who do not suffer from a cancer). The control samples may be taken from one or more relevant databases. Preferably, the number of control samples is statistically significant.

Detection of transcript overexpression may be performed using any of the following techniques, which are known in the art: polymerase chain reaction (PCR); northern blotting; hybridisation-based detection techniques; or gene expression array.

"Chemotherapy", as referred to herein, can refer to any chemical drug that can be used to treat or prevent cancer, by, for example, killing cancer cells, shrinking a tumour, stopping or reducing replication of cancer cells or stopping or reducing growth of a tumour. Thus, chemotherapy may refer to a chemotherapeutic drug that inhibits mitosis or cellular division of cancer cells and/or induces cell death. The drug may be non-specific. Chemotherapy as referred to herein does not include endocrine therapy or targeted therapy.

Chemotherapy agents according to the invention may therefore include a selection from the group comprising/consisting of: cisplatinum, taxane, cyclophosphamide, methotrexate, 5-fluorouracil, an anthracycline, epirubicin, doxorubicin and anthracycline based combination chemotherapy.

Anthracycline based combination chemotherapy comprises epirubicin (E) or doxorubicin (A) and one or more chemotherapy drugs, which may include cyclophosphamide (C) and fluorouracil (5-FU; F).

"Endocrine therapy" (or "hormonal therapy"), as referred to herein, can refer to any drugs that can be used to treat or prevent cancer by preventing ER signalling through either selective oestrogen receptor modulators (SERM) (e.g., tamoxifen) or aromatase inhibitors (AIs). The endocrine therapy may therefore be a hormone, a hormone receptor antagonist or an enzyme inhibitor (such as an aromatase inhibitor). The hormone or hormone receptor antagonist may, for example, act via the endocrine receptor. Endocrine therapy according to the invention may therefore include: tamoxifen and/or an aromatase inhibitor.

"Neoadjuvant chemotherapy", as referred to herein, can refer to chemotherapy that is administered to a subject/patient before surgical removal of a tumour.

"Systemic therapy", as referred to herein, can refer to chemotherapy, HER2 target therapy and/or endocrine therapy received either before or after surgical removal of the tumour.

"HER2 status" may refer to the expression the human epidermal growth factor receptor 2, preferably using the American Society of Clinical Oncology guidelines.

The term "determining" can refer, but is not limited, to the use of any one of the following conventional assays for detecting the presence of one or more of the biomarkers (such as SPAG5 or the oesterogen receptor), or variants or fragments thereof, in a sample: flow cytometry; immunoassays, such as enzyme-linked immunosorbent assays (ELISAs), enzyme immunoassays (EIAs), radioimmunoassay (RIAs), Western Blots, immuo-precipitation or immunohistochemistry (IHC); immuno-electron microscopy; chromogenic (enzyme activity) assays; fluorometric imaging plate reader (FLIPR) assays; or high performance liquid chromatography (HPLC) tandem mass spectrometry (MS/MS). Preferably, immunohistochemistry or immuno-electron microscopy is used to detect granular SPAG5 protein.

Advantageously, IHC and immuno-electron microscopy allow stained cancer tissues that comprise cells containing granules of SPAG5 protein to be identified easily, i.e., by simply looking at the cells under a microscope.

The term "sample" refers to a specimen taken from the body of a subject. The sample may comprise blood, plasma, serum, spinal fluid, urine, sweat, saliva, tears, breast aspirate, prostate fluid, seminal fluid, vaginal fluid, stool, cervical scraping, cytes, intraocular fluid, animal tissue, cell lysates, tumour tissue, skin, bone marrow, cartilage, or combinations thereof. The sample may be contained within the subject, which maybe an experimental animal (e.g., a mouse or rat) or a human, wherein the method is an in vivo based test. Alternatively, the sample may be an ex vivo sample or an in vitro sample. Therefore, the cells being tested may be in a tissue sample (for ex vivo based tests) or the cells maybe grown in culture (an in vitro sample). Preferably, the biological sample is an ex vivo sample. The sample may be a (biological) tissue. Preferably the tissue is a solid tissue, such as breast tissue, bone tissue, lung tissue or liver tissue. Most preferably, the sample comprises tumour tissue. The sample may be any sample that comprises one or more cancer cells.

The "subject" may be a vertebrate, mammal or domestic mammal. Hence, the methods according to the invention may be used to treat any animal, for example, pigs, cats, dogs, horses, sheep or cows. Preferably, the subject is a human being.

"A cell" may be one or more cells. Preferably, it means two or more cells, or three or more cells.

It will be appreciated that the invention relates to or extends to any nucleic acid or peptide or variant, derivative or analogue thereof, which comprises substantially the amino acid or nucleic acid sequences of any of the sequences referred to herein, including variants or fragments thereof. The terms *"substantially the amino acid/nucleotide/peptide sequence"*, *"variant"* and *"fragment"*, can be a sequence that has at least 40% sequence identity with the amino acid/nucleotide/peptide sequences of any one of the sequences referred to herein, for example 40% identity with the polypeptide identified as SEQ ID No. 1, and so on.

Amino acid/polynucleotide/polypeptide sequences with a sequence identity which is greater than 50%, more preferably greater than 65%, 70%, 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90%, 92%, 95%, 97%, 98%, and most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants. Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al, 1994, Nucleic Acids Research, 22, 4673-4680; Thompson et al, 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment. Preferably, calculation of percentage identities between two amino acid/polynucleotide/polypeptide sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula:- Sequence Identity = (N/T)*100. Alternative methods for identifying similar sequences will be known to those skilled in the art. For example, a substantially similar nucleotide sequence will be encoded by a sequence which hybridizes to any sequences referred to herein or their complements under stringent conditions. By stringent conditions, it is meant that the nucleotide hybridises to filter-bound DNA or RNA in 3x sodium chloride/sodium citrate (SSC) at approximately 45°C followed by at least one wash in o.2x SSC/o.1% SDS at approximately 20-65°C. Alternatively, a substantially similar polypeptide may differ by at least 1, but less than 5, 10, 20, 50 or 100 amino acids from the sequences shown in SEQ ID No.1.

Due to the degeneracy of the genetic code, it is clear that any nucleic acid sequence described herein could be varied or changed without substantially affecting the sequence of the protein encoded thereby, to provide a variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example, small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

All of the features described herein (including the accompanying claims, abstract and drawings) and/or all of the steps of any method or process so disclosed, may be combined with any of the above aspects in any combinations, except combinations where at least some of such features and/or steps are mutually exclusive.

For a better understanding of the invention, and to show how embodiments of the same may be carried into effect, reference will now be made, by way of example, to the accompanying Figures, in which:-

Figure 1 is an image of breast cancer tissue that has been stained using immunohistochemistry in order to identify SPAG5. SPAG5 overexpression (high SPAG5) was defined as the presence of granular cytoplasmic staining of SPAG5 in cells of the cancer tissue (Example 1);

Figure 2 is an image of breast cancer tissue that has been stained using immunohistochemistry in order to identify SPAG5. SPAG5 overexpression (high SPAG5) was defined as the presence of an intracytoplasmic granular (i.e., punctate) staining pattern in cells of the cancer tissue (Example 1);

Figure 3 is an image of breast cancer tissue that has been stained using immunohistochemistry in order to identify SPAG5. SPAG5 overexpression (high SPAG5) was defined as the presence of a granular staining pattern of SPAG5 protein in the cytoplasm of cancer cells in the cancer tissue (Example 1);

Figure 4 is an image of breast cancer tissue that has been stained using immunohistochemistry in order to identify SPAG5. SPAG5 overexpression (high SPAG5) was defined as the presence of intracytoplasmic granular staining pattern of SPAG5 protein in cells of the cancer tissue (Example 1);

Figures 5A and 5B are images of breast cancer tissue that has been stained using immunohistochemistry in order to identify negative or low expression of SPAG5 protein, which is defined as either complete absence of any staining or the presence of evenly diffuse staining for SPAG5 protein (Example 2);

Figure 6 is a Kaplan-Meier plot showing the predicted probability of breast cancer specific survival of breast cancer patients that express low and high levels of SPAG protein in their tumour tissues at the time of surgery (Nottingham University Hospitals Early-Stage Breast cancer (NUH-ESBC) patients (Example 3);

Figure 7 is a Kaplan-Meier plot showing the predicted probability of breast cancer specific survival of breast cancer patients that express low and high levels of SPAG protein in their tumour tissues at the time of surgery (Queensland Follow Up Early Stage Breast cancer (QFU-ESBC) patients) (Example 3);

Figure 8 is a Kaplan-Meier plot showing the predicted probability of breast cancer specific survival of ER+ breast cancer patients that express low and high levels of SPAG protein in their tumour tissues at the time of surgery (ER+ NUH ESBC Cohort)(Example 4);

Figure 9 is a Kaplan-Meier plot the predicted probability of breast cancer specific survival of ER+ breast cancer patients that express low and high levels of SPAG protein in their tumour tissues at the time of surgery (ER+ QFU ESBC Cohort) (Example 4);

Figure 10 is a Kaplan-Meier plot showing the risk of distant relapse in ER+ breast cancer patients that express low and high levels of SPAG protein (NUH ESBC Cohort - see Example 6);

Figure 11 is a Kaplan-Meier plot showing the risk of distant relapse in ER+LN-breast cancer patients that express low and high levels of SPAG protein (NUH ESBC Cohort - see Example 7);

Figure 12 is a Kaplan-Meier plot showing the risk of distant relapse in ER+LN-breast cancer patients that express low and high levels of SPAG protein after being treated with Tamoxifen (NUH ESBC Cohort - see Example 8);

Figure 13 is a Kaplan-Meier plot showing the risk of distant relapse in ER+LN-breast cancer patients that express low and high levels of SPAG protein after being treated with adjuvant AC therapy with or without taxane chemotherapy in addition to Tamoxifen (NUH ESBC Cohort - see Example 9);

Figure 14 is a Kaplan-Meier plot showing the risk of distant relapse in ER+LN+ breast cancer patients that express low and high levels of SPAG protein after being treated with Tamoxifen (NUH ESBC Cohort - see Example 10);

Figure 15 is a Kaplan-Meier plot showing the risk of distant relapse in ER+LN+ breast cancer patients that express low and high levels of SPAG protein after being treated with CMF therapy and Tamoxifen (NUH ESBC Cohort - see Example 11);

Figure 16 is a Kaplan-Meier plot showing the risk of distant relapse in ER+LN+ breast cancer patients that express low and high levels of SPAG protein after being treated with Ac therapy with or without taxane in addition to Tamoxifen (NUH ESBC Cohort - see Example 8);

Figure 17A is a bar chart showing the relationship between SPAG5 protein expression and pathological complete response (pCR) to preoperative anthracycline based combination therapy with or without taxane in all patients and ER- patients, ER+ patients, HER2 negative patients and HER2+ patient subgroups (Example 14-18):

Figure 17B is a bar chart showing the relationship between SPAG5 protein expression and pathological complete response (pCR) to preoperative anthracycline based combination therapy alone (AC), with taxane (AC+T) or with taxane and Herceptin® (AC+T+H) in All patients (Example 19-21);

Figure 17C is bar chart showing the relationship between SPAG5 protein expression and pathological complete response (pCR) to preoperative anthracycline based combination therapy alone (AC), with taxane (AC+T) or with taxane and Herceptin® (AC+T+H) in ER+ patients (Example 22-24);

Figure 18 is a Kaplan-Meier plot showing the predicted probability of risk of relapse in ER+LN- breast cancer patients who have not received any systemic therapy and have tumours that overexpress SPAG mRNA (SPAG5+) or do not overexpress SPAG5 mRNA (SPAG5-) (Example 26);

Figure 19 is a Kaplan-Meier plot showing the predicted probability of risk of relapse in ER+ LN- breast cancer patients that overexpress SPAG mRNA (SPAG5+) or do not overexpress SPAG5 mRNA (SPAG5-) and are treated with tamoxifen alone (Example 27);

Figure 20 is a Kaplan-Meier plot showing the predicted probability of risk of relapse in ER+ LN- breast cancer patients that do not overexpress mRNA (SPAG5-) and are not treated with adjuvant therapy, are treated with tamoxifen and AC therapy or treated with tamoxifen only (Examples 28 and 29);

Figure 21 is a Kaplan-Meier plot showing the predicted probability of risk of relapse in ER+ LN- breast cancer patients that do overexpress mRNA (SPAG5+) and are not treated with systemic adjuvant therapy, are treated with tamoxifen only or treated with tamoxifen and AC (Examples 30 and 31);

Figure 22 is a Kaplan-Meier plot showing the predicted probability of risk of relapse in ER+ LN+ breast cancer patients that overexpress mRNA (SPAG5+) or do not overexpress SPAG5 mRNA (SPAG5-) and are treated with tamoxifen only (Example 32);

Figure 23 is a Kaplan-Meier plot showing the predicted probability of risk of relapse in ER+ LN+ breast cancer patients that overexpress mRNA (SPAG5+) and are treated with tamoxifen and AC therapy (Example 33);

Figure 24 is a Kaplan-Meier plot showing the predicted probability of risk of relapse in ER+ LN+ breast cancer patients that overexpress mRNA (SPAG5+) and are treated with tamoxifen only or are treated with Tamoxifen and AC-therapy (Example 34);

Figure 25 is a Kaplan-Meier plot showing the predicted probability of risk of relapse in ER+LN+ breast cancer patients that do not overexpress mRNA (SPAG5-) and are treated with tamoxifen only or treated with tamoxifen and AC therapy (Example 35);

Figure 26 shows the relationship between SPAG5 transcript expression changes using gene expression array and the changes in the volume of tumour mass measured by ultrasound during 3-month course of neoadjuvant endocrine therapy: (A) boxplots diagram showing the changes of SPAG5 mRNA during neoadjuvant endocrine therapy (pre-treatment vs., after 2weeks vs 3 months). (B): boxplots diagram showing the levels of SPAG5 mRNA in responder vs non responder after 3 months of neoadjuvant endocrine therapy;

Figure 27 is a is bar chart showing the relationship between SPAG5 transcript expression and pathological complete response (pCR) to preoperative anthracycline based combination therapy alone (AC), with taxane (AC+T) or with Taxane and Herceptin® (AC+T+H) in ER+ breast cancer patients (mRNA expression) - Example 38;

Figure 28 is a Kaplan-Meier plot showing the predicted probability of risk of relapse in ovarian cancer patients that overexpress SPAG5 mRNA (low) or do not overexpress SPAG5 mRNA (high) (Example 44);

Figure 29 is an image that shows the intracytoplasmic granular (punctate) staining pattern of SPAG5 protein in ovarian cancer tissue that has been stained using immunohistochemistry (Example 45);

Figure 30 is a Kaplan-Meier plot showing the predicted probability of risk of death in ovarian cancer patients that overexpress SPAG5 protein (high) or do not overexpress SPAG5 protein (low) and are treated with cisplatinum and taxane (Example 46);

Figure 31 is a Kaplan-Meier plot showing the predicted probability of risk of relapse in ovarian cancer patients that overexpress SPAG5 protein (high) or do not overexpress SPAG5 protein (low) and are treated with cisplatinum and taxane (Example 47);

Figure 32 is a Kaplan-Meier plot showing the predicted probability of risk of relapse in gastric cancer patients that overexpress SPAG5 mRNA (high) or do not overexpress SPAG5 mRNA (low) and are treated with cisplatinum (Example 48);

Figure 33 is a Kaplan-Meier plot showing the predicted probability of risk of relapse in gastric cancer patients that overexpress SPAG5 mRNA (high) or do not overexpress SPAG5 mRNA (low) and are treated with 5-flurouracil (Example 49);

Figure 34 is an image that shows the intracytoplasmic granular (punctate) staining pattern of SPAG5 protein in gastric cancer tissue that has been stained using immunohistochemistry (Example 50);

Figure 35 is a Kaplan-Meier plot showing the predicted probability of risk of death in gastric cancer patients that overexpress SPAG5 protein (high) or do not overexpress SPAG5 protein (low) and are treated with cisplatinum (Example 51);

Figure 36 is a Kaplan-Meier plot showing the predicted probability of risk of relapse in gastric cancer patients that overexpress SPAG5 protein (high) or do not overexpress SPAG5 protein (low) and are treated with cisplatinum (Example 51);

Figure 37 is a Kaplan-Meier plot showing the predicted probability of risk of death in lung cancer patients that overexpress SPAG5 mRNA (high) or do not overexpress SPAG5 mRNA (low) (Example 52);

Figure 38 is a Kaplan-Meier plot showing the predicted probability of risk of recurrence in lung cancer patients that overexpress SPAG5 mRNA (high) or do not overexpress SPAG5 mRNA (low) (Example 53);

Figure 39 is an image that shows granular SPAG5 protein in lung cancer tissue that has been stained using immunohistochemistry (Example 54);

Figure 40 is a Kaplan-Meier plot showing the predicted probability of risk of death in lung cancer patients that overexpress SPAG5 protein or do not overexpress SPAG5 protein (low) (Example 55);

Figure 41 is a Kaplan-Meier plot showing the predicted probability of risk of relapse in liver cancer patients that overexpress SPAG5 mRNA (high) or do not overexpress SPAG5 mRNA (low) (Example 56);

Figure 42 is a Kaplan-Meier plot showing the predicted probability of risk of relapse in colon cancer patients that overexpress SPAG5 protein (high) or do not overexpress SPAG5 protein (low) and are treated with cisplatin (Example 57);

Figure 43 is a Kaplan-Meier plot showing the predicted probability of risk of death in rectal cancer patients who did not receive systemic therapy and their tumours overexpress SPAG5 protein (high) or do not overexpress SPAG5 protein (low) (Example 58);

Figure 44 is a Kaplan-Meier plot showing the predicted probability of risk of distant metastases in HER2+ patients who did not receive HER2 target therapy and anthracycline based chemotherapy and their tumours overexpress SPAG5 protein (high) or do not overexpress SPAG5 protein (low) (Example 59).

Figure 45 is a Kaplan-Meier plot showing the predicted probability of risk of distant metastases in HER2+ patients who did receive anthracycline based chemotherapy but no HER2 target therapy and their tumours overexpress SPAG5 protein (high) or do not overexpress SPAG5 protein (low) (Example 60);

Figure 46 is a Kaplan-Meier plot showing the predicted probability of risk of distant metastases in HER2+ patients who did receive anthracycline based chemotherapy plus HER2 target therapy (Trastuzumab) and their tumours overexpress SPAG5 protein (high) or do not overexpress SPAG5 protein (low) (Example 61);

Figure 47 is a Kaplan-Meier plot showing the predicted probability of risk of distant metastases in HER2+ patients who did not receive HER2 target therapy and anthracycline based chemotherapy and their tumours overexpress SPAG5 transcript (high) or do not overexpress SPAG5 transcript (low) (Example 63);

Figure 48 is a Kaplan-Meier plot showing the predicted probability of risk of distant metastases in HER2+ patients who received anthracycline based chemotherapy but no HER2 target therapy and their tumours overexpress SPAG5 transcript (high) or do not overexpress SPAG5 transcript (low) (Example 64); and

Figure 49 is a Kaplan-Meier plot showing the predicted probability of risk of distant metastases in HER2+ patients who received anthracycline based chemotherapy plus HER2 target therapy and their tumours overexpress SPAG5 transcript (high) or do not overexpress SPAG5 transcript (low) (Example 65).

Figure 50 is a Kaplan-Meier plot showing the predicted probability of risk of relapse of the pre systemic therapy HER2 negative samples who received anthracycline based combination chemotherapy with or without taxane stratified according to HER2 status in their paired post chemotherapy tumours samples (Example 67).

Figure 51 is a Kaplan-Meier plot showing the predicted probability of risk of relapse of the paired post neoadjuvant chemotherapy HER2+ samples stratified according to if they received adjuvant HER2 target therapy (Trastuzumab) or not (Example 68).

Figure 52 is a Kaplan-Meier plot showing the predicted probability of risk of relapse of the pre systemic therapy HER2 negative samples who remained HER2- in the paired post neoadjuvant chemotherapy samples stratified according to the presence of granules of SPAG5 protein expression in the paired post chemotherapy tumours samples (Example 69).

Figure 53 is a Kaplan-Meier plot showing the predicted probability of risk of relapse of the pre systemic therapy HER2 negative/ ER negative samples who remained HER2- in the paired post neoadjuvant chemotherapy samples stratified according to the presence of granules of SPAG5 protein expression in the paired post chemotherapy tumours samples (Example 70).

Figure 54 is a Kaplan-Meier plot showing the predicted probability of risk of relapse of the pre systemic therapy HER2 negative/ ER positive samples who remained HER2- in the paired post neoadjuvant chemotherapy samples stratified according to the presence of granules of SPAG5 protein expression in the paired post chemotherapy tumours samples (Example 71). All patients have received adjuvant endocrine therapy for 5 years.

Figure 55 is a Kaplan-Meier plot showing the predicted probability of risk of relapse of the pre systemic therapy HER2 positive samples who received anthracycline based combination chemotherapy with taxane with or without HER2 target neoadjuvant therapy followed by HER2 target therapy for one year stratified according to HER2 status in their paired post chemotherapy residual tumours samples (Example 72).

Figure 56 is a Kaplan-Meier plot showing the predicted probability of risk of relapse of the pre systemic therapy HER2 positive samples who remained HER2+ in the paired post neoadjuvant chemotherapy samples stratified according to if they did or did not receive adjuvant HER2 target therapy (Example 73).

Figure 57 is a Kaplan-Meier plot showing the predicted probability of risk of relapse of the pre systemic therapy HER2 positive samples that remained HER2+ in the paired post neoadjuvant chemotherapy samples and received adjuvant HER2 target therapy for one year stratified according to the presence or absence of granular SPAG5 protein expression (Example 74).

Figure 58 is a Kaplan-Meier plot showing the predicted probability of risk of relapse of the pre systemic therapy HER2 positive/ ER positive samples who remained HER2+ in the paired post neoadjuvant chemotherapy samples and received adjuvant HER2 target therapy for one year in addition to 5- year adjuvant endocrine therapy stratified according to the presence or absence of granular SPAG5 protein expression (Example 74).

Figure 59 is a Kaplan-Meier plot showing the predicted probability of risk of relapse of the pre systemic therapy HER2 positive/ER negative samples who remained HER2+ in the paired post neoadjuvant chemotherapy samples and received adjuvant HER2 target therapy for one year stratified according to the presence or absence of granular SPAG5 protein expression (Example 74).

## Examples

## Materials and Methods

*SPAG5 protein expression (i.e. Examples 1 to 25, 46 to 48, 51, 52, 55, 56, 58 and 59)*

Immunohistochemistry (IHC) and tissue microarray (TMA) analysis of SPAG5 protein expression in breast cancer was investigated by IHC analysis at two independent centres:

## 1) Nottingham University Hospitals (NUH), UK

Tumours were arrayed in tissue microarrays (TMAs) constructed with two duplicates of 0.6mm cores from the periphery of the tumours. The TMAs were immunohistochemically profiled for SPAG5. Immunohistochemical staining was performed using the Thermo Scientific Shandon Sequenza chamber system (REF: 72110017), in combination with the Novolink Max Polymer Detection System (RE7280-K: 1250 tests), and the Leica Bond Primary Antibody Diluent (AR9352), each used according to the manufacturer's instructions (Leica Microsystems). The tissue slides were deparaffinised with xylene and then rehydrated through five decreasing concentrations of alcohol; 100%, 90%, 70%, 50% and 30% for two minutes each. Pre-treatment antigen retrieval was performed on the TMA sections using sodium citrate buffer (pH 6.0) and heated for 20 minutes at 95°C in a microwave (Whirpool JT359 Jet Chef 1000W). A set of slides were incubated with the primary anti-SPAG5 antibody (Sigma HPA022479), at a dilution of 1:50 for 30 minutes. Negative and positive (by omission of the primary antibody and IgG-matched serum) controls were included in each run. The negative control ensured that all the staining was produced from the specific interaction between antibody and antigen.
The individual tissue cores were scored by two experienced pathologists, according to the intensity of tumour cell staining: punctate cytoplasmic staining was recorded as 1+ (if it is of mild/moderate granular intensity) or 2+ (if it is with strong granular intensity). No staining, faint or diffuse staining was recorded as 0 (negative or week). Using the maximum score of duplicate tissue cores for each case, the percentage of each category was estimated (0-100%). A score (range 0-200) was calculated by multiplying intensity of staining and percentage staining. A median score of $\geq 1$ was taken as the cut-off for high SPAG5 cytoplasmic expression. Not all cores within the TMA were suitable for IHC analysis as some cores were missing or lacked tumour (<15% tumour). The expression of HER2, ER and PR was re-assessed according to the American Society of Clinical Oncology/College of American Pathologists (ASCO/CAP) guidelines. To validate the use of TMAs for immuno-phenotyping, full-face sections of 40 cases were stained and the protein expression levels were compared. The concordance between TMAs and full-face sections was excellent using Cohen's kappa statistical test for categorical variables (kappa=0.8).
The clinicopathological and molecular characteristics of SPAG5 protein expression as well as its association with death, relapse and or distal relapse risks were analysed in the in the following cohorts:

a) **Nottingham University Hospital Early Stage Breast cancer (NUH-ESBC) cohort.**
This cohort of BC include 3196 patients (age>71 years) who were diagnosed and treated uniformly between 1986 and 2006 at the Nottingham City Hospital (NCH), Nottingham, UK. Patients within the good prognosis group (Nottingham Prognostic Index (NPI) <3.4) did not receive systemic adjuvant therapy. Pre-menopausal patients within the moderate and poor prognosis groups were candidates for CMF chemotherapy (cyclophosphamide 750 mg m$^{-2}$, methotrexate 50 mg m$^{-2}$ and 5-fluorouracil 1 g m$^{-2}$, on day 1 of a 21-day cycle.). Conversely, postmenopausal ER-positive patients with moderate or poor NPI were offered hormonal therapy, whereas ER-negative patients received CMF chemotherapy. Additionally, none of the human epidermal growth factor receptor-2 (HER2) overexpression patients received trastuzumab. Clinical data were maintained on a prospective basis with a median follow-up of 143 months (Inter-quartile range (IQR) = 114-174). Survival data were maintained on a prospective basis. Breast cancer specific survival (BCSS) was defined as the number of months from diagnosis to the occurrence of breast cancer related death. Survival was censored if the patient was still alive, lost to follow-up, or died from other causes. Relapse free survival (RFS) was defined as the number of months from diagnosis to relapse either locally, regionally or distantly. Distant relapse free survival (DRFS) was defined as the number of months from diagnosis to distant metastases relapse.

**b) ER- Nottingham University Hospital Early-Stage breast cancer cohort (ER- NUH- ESBC; n=697)**

To evaluate the survival benefit of SPAG5-protein expression, its expression was analysed in a consecutive series of 697 early-stage ER- BC's who had been diagnosed and managed at NCH between 1999 and 2007. This series included: 1) The ER-negative BC patients of Nottingham historical earl stage BC cohort (n=332) who managed before 2000 and treated either with no chemotherapy or with adjuvant CMF and 2) the new ER-negative early-stage BC patients (n=365) who managed after 2000 and received either no chemotherapy or adjuvant anthracycline-based combination chemotherapy.

**c) Nottingham anthracycline based Neo-Adjuvant Chemotherapy cohort (Nottingham AC-Neo-ACT; n=450)**

The relationship between SPAG5-protein expression and response to chemotherapy was evaluated by investigating its expression in pair-matched pre-chemotherapy core biopsies and post-chemotherapy surgical specimens, from 450 female patients with locally-advanced primary BC (LAP-BC) (stage IIIA-C) that had been treated with anthracycline-based Neo-ACT (AC-Neo-ACT) [18] at NCH between 1996 and 2015. Sixty three percent of patients received six cycles of an anthracycline-based therapy (FEC: 5-fluorouracil (5-FU) 500 mg m$^{-2}$, epirubicin 75-100 mg m$^{-2}$, cyclophosphamide 500 mg m$^{-2}$, on day 1 of a 21-day cycle), whereas 37% of patients received FEC plus Taxane. All patients underwent mastectomy or breast-conserving surgery and axillary dissection, followed by adjuvant radiation therapy. Patients with ER+ BCs were offered 5 years of adjuvant endocrine therapy. The median follow-up time was 67 months (IRQ 27-81).

**Pathological response rate (pCR):** To assess SPAG5 protein expression as a predictive biomarker for response to combination cytotoxic chemotherapy, the association with pCR was analysed.

**2) University of Queensland, Australia**

The IHC of SPAG5 protein has been externally validated at University of Queensland centre using Queensland breast cancer follow-up (QFU) cohort, which comprises TMAs of 547 invasive breast carcinomas (sampled in duplicate at a minimum) with associated clinical data, including breast cancer-specific survival up to 35 years post-diagnosis (median follow-up 14.1 years, range 0.03 -41.75 years). The samples were collected from the Royal Brisbane and Women's Hospital (RBWH) between 1987 and 1994. Pathological and clinical data for these patients were obtained from a combination of clinical diagnostic pathology reports (Pathology Queensland), the Queensland Cancer Registry and internal histopathology review (SRL). The use of samples and clinical data for this study were approved by human research ethics committees of the University of Queensland and RBWH.

Four pm TMA sections were processed in a decloaker for antigen retrieval in sodium citrate buffer (pH 6.0) for 20 mins, and then IHC was performed using an anti-SPAG5 antibody (Sigma HPA022479; 1:50), and the Mach 1 Universal HRP-Polymer Detection kit (Biocare Medical). Haematoxylin-counterstained, mounted sections were then scanned at 40 x magnification on an Aperio AT Turbo slide scanner (Leica Biosystems). Digital images of individual tissue cores were scored by three experienced molecular pathologists according to the intensity of tumour cell staining: punctate cytoplasmic staining was recorded as 1+ (moderate) or 2+ (strong), and multiplied by the percentage of tumour cells stained to derive a final score ranged between o to 200. Faint, diffuse staining was recorded as 0. Using the maximum score of duplicate tissue cores for each case, associations between SPAG5 expression and clinicopathologic variables were investigated.

Statistical analysis: Statistical analyses were performed using STATISTICA (Stat Soft Ltd, Tulsa, USA) and SPSS (version 17, Chicago, USA) by the authors who were blinded to the clinical data. Where appropriate, Pearson's chi-squared; student's t-test and ANOVA tests were used. Positivity for SPAG5 protein both pre- and post-¬chemotherapy was calculated and compared using McNemar's test. Cumulative survival probabilities and 10-year BCSS and DRFS were estimated using the univariate Cox proportional hazards models and the Kaplan-Meier plot method where appropriate, and differences between survival rates were tested for significance using the log-rank test. Multivariable analysis for survival was performed using the Cox proportional hazard model. The proportional hazards assumption was tested using standard log-log plots. Hazard ratios (HR) and 95% confidence intervals (95% CI) were estimated for each variable. All tests were two-sided with a 95% CI and a p value <0.05 was considered to be indicative of statistical significance. The interaction between SPAG5 and chemotherapy was tested in Cox proportional hazard model. For multiple comparisons, p values were adjusted according to Benjamini-Hochberg method. Ethical approval was obtained from the Institutes Research Ethics Committees.

Also, immunohistochemistry (IHC) and tissue microarray (TMA) analysis of SPAG5 protein expression in other tumours types was investigated by IHC analysis at Nottingham City Hospital including:

1) Ovarian cancer cohort (Nottingham-OVC): Investigation of the expression of SPAG5 protein in ovarian epithelial cancer was carried out on tissue microarrays of 195 consecutive ovarian epithelial cancer cases treated at NUH between 2000 and 2007. All patients treated with surgery followed cisplatinum. Patients were comprehensively staged as per International Federation of Obstetricians and Gynecologists (FIGO) Staging System for Ovarian Cancer. Survival was calculated from the operation date until 1st of October 2015 when any remaining survivors were censored. Platinum resistance was defined as patients who had progression during first-line platinum chemotherapy or relapse within 6 months after treatment.

2) Gastric cancer cohort (Nottingham-Gastric Cancer): This cohort included two sets of cases. The first set consisted of 142 gastric/gastro-oesophageal cancer cases not exposed to neoadjuvant chemotherapy. The second set we consisted of 103 gastric/gastro-oesophageal cancer cases exposed to preoperative platinum-based chemotherapy. Tissue was obtained from patients treated at Nottingham University Hospitals (NUH) between 2001 and 2008. Survival was calculated from the date of diagnosis until 13 January 2015, when any remaining survivors were censored. During the study period, patients in the neoadjuvant arm with adenocarcinomas were treated with either neoadjuvant ECF (epirubicin (50 mg m$^{-2}$), cisplatin (60 mg m$^{-2}$) and continuous infusional 5-FU (200 mg m$^{-2}$ per day)) or ECX (epirubicin (50 mg m$^{-2}$), cisplatin (60 mg m$^{-2}$) and capecetabine (625 mg m$^{-2}$ p.o. b.d continuously)) chemotherapy up to three cycles before surgery. Patients with squamous cell carcinoma were treated with CF (cisplatin (80 mg m$^{-2}$) and infusional 5-FU (1000 mg m$^{-2}$ daily for 4 days)) chemotherapy up to two cycles before surgery. The conduct of this study was approved by the ethics committee of Nottingham University Hospitals.

3) Lung cancer cohort (Nottingham-Lung cancer): 220 non-small cell lung cancers (January 1996-July 2006; median follow-up, 36 months; censored May 2013; none of the patients received chemotherapy prior to surgery but 11 patients received radiotherapy and 9 patients received at least 1 cycle of adjuvant chemotherapy postsurgery) obtained from patients undergoing elective surgical resection of a histologically proven cancer at Nottingham or Derby University Hospitals (Derby, United Kingdom).

4) Colorectal cancer: Tumour samples from 462 patients who underwent elective surgery to resect a primary colorectal cancer between 1994 and 2000 (mean follow-up of 75 months) were assembled in tissue microarray format. Clinico-pathological data including tumour grade, stage, and vascular invasion status along with disease specific survival data has been collected prospectively. Patients with lymph node-positive disease routinely received adjuvant chemotherapy with 5-flurouracil/folinic acid).

*SPAG5 transcript expression*

*A) Breast Cancer - Assessment death and distant relapse risks after surgery and receiving adjuvant systemic therapy if eligible in Early-stage breast cancer (i.e. Examples 26 to 37, 45, 49, 50, 53, 54 and 57)*

The inventors evaluated the prognostic utility of SPAG5-mRNA expression in a large combined breast cancer (BC) cohort which was sourced from 15 publically-available, global datasets (n=3538) using the following access numbers.

| DATABASE | PLATFORM | | |
|---|---|---|---|
| GS33143 | [HG_U95Av2] | AFFYMETRIX | GPL8300 |
| GSE20685 | HG-UA133-PLUS2 | AFFYMETRIX | GPL570 |
| GSE21653 | HG-UA133-PLUS2 | AFFYMETRIX | GPL570 |
| H_v2.1.1 55K | SWEGENE | | GPL5345 |
| GSE12093 | HG-UA133A | AFFYMETRIX | GPL96 |
| GSE31448 | HG-UA133-PLUS2 | AFFYMETRIX | GPL570 |
| GSE42568 | HG-UA133-PLUS2 | AFFYMETRIX | GPL570 |
| GSE45255 | HG-UA133A | AFFYMETRIX | GPL96 |
| GSE9893 | MLRG Human 21K V | Human Qiagen | GPL5049 |
| GSE22219 | Expression BeadC | ILLUMINA | GPL6098 |
| GSE10510 | OLIG0-ARRAY-35K | DKFZ | GPL6486 |
| GSE11121 | HG-UA133A | AFFYMETRIX | GPL96 |
| GSE7390 | HG-UA133A | AFFYMETRIX | GPL96 |
| GSE2034 | HG-UA133A | AFFYMETRIX | GPL96 |
| GSE6532-GSE9195 | HG-UA133-PLUS2 | AFFYMETRIX | GPL570 |

All the databases had been downloaded and processed. The gene expression data were converted to a common scale (median equal to 0 and standard deviation equal to 1) in order to merge all of the studies data and create combined cohorts. Each sample in the data was standardised to a mean of 0 and standard deviation of 1. Then the data was median centred for each gene whereby median of each gene is 0.

In addition, the SPAG5-trancript analysis was validated using the Molecular Taxonomy of BC International Consortium [METABRIC (n=1980; median follow-up time in years (MFUT) (inter-quantile range (IQR)): 9.1(5.2-12.9)) and The Cancer Genome Atlas-Breast Cancer project13 (TCGA-BRCA; n=709; MFUT (IQR): 1.9 (1.7-3.6).

In these cohorts, oestrogen receptor positive (ER+) and/or lymph-node negative (LN-) patients did not receive adjuvant chemotherapy, whereas ER negative (ER-) and/or lymph-node positive (LN+) patients received adjuvant chemotherapy. Additionally, none of the human epidermal growth factor receptor-2 (HER2) overexpression (+) patients received trastuzumab.

Cut-off determination:

SPAG5 gene expression data of each database were converted to a common scale (median equal to 0 and standard deviation equal to 1) in order to merge all of the studies data that used the same platform and create combined cohorts. To convert to a common scale each sample in the data was standardised to a mean of 0 and standard deviation of 1. Then the data was median centred for each gene whereby median of each gene is 0. Databases using same platform have been merged and the median expression was calculated. The median expression of SPAG5 transcript for each platform has been calculated and values equal to or higher than the median coded as +1 (overexpression). Values of less than the median have been coded 0 or low SPAG5.

For HG-U133A-Affymetrix; GPL96 platform: SPAG5 median was 0.3858. For HG-U133_Plus_2 -Affymetrix; GPL570 SPAG5 median was 0.4205 For [HG_U95Av2] AFFYMETRIX GPL8300 platform: SPAG5 median was 0.1259 For H_v2.1.1 55K SWEGENE GPL5345 platform: SPAG5 median was 0.4163 For MLRG Human 21K V-Human QiagenGPL5049 platform: SPAG5 median was 0.4012 For OLIG0-ARRAY-35K-DKFZ GPL6486 platform: SPAG5 median was 0.6963 For Expression BeadC-ILLUMINA GPL6098 platform: SPAG5 median was 0.3535

*B) Breast Cancer - Assessment of the clinical dynamic response after receiving preoperative endocrine therapy (aromatase inhibitor) (i.e., Example 38)*

The association between SPAG5 mRNA expression and clinical response after receiving preoperative therapy has been analysed in a total of 101 patients from a consecutive series of 255 postmenopausal women who presented to Western General Hospital in Edinburgh with large primary histologically confirmed estrogen receptor (ER) -rich (Allred scores 6 to 8) invasive breast cancer and who were recruited between 2003 and 2011 fulfilled the requirements to be included in this. All patients gave informed consent and the study was approved by the local regional ethics committee (2001/8/80 and 2001/8/81). Patients were treated with letrozole (Femara, 2.5 mg; Novartis Pharma AG, Basel, Switzerland) daily for at least 3 months. The clinical response was assessed by three-dimensional ultrasound measurements using dynamic (serial) changes in tumor volumes. Tumours with < 50% reduction in volume were classified as non-responders. SPAG5 mRNA expression data were retrieved from the publically available gene expression microarray datasets at the National Center for Biotechnology Information Gene Expression Omnibus under access numbers (GSE59515, GSE55374, and GSE20181).

*Gene expression analysis and data processing*

For "GSE59515" and "GSE55374" cases, RNA was extracted, labelled, and hybridized to HumanHT-12 v4 Illumina BeadChips (GPL10558) according to the standard protocol for NuGEN amplified samples. For GSE20181 cases, samples were processed on Affymetrix U133A gene chip (GPL96). There were no significant differences in clinical or pathologic features of patients or tumours from the three datasets. Illumina and Affymetrix data were independently normalized (quantile using the lumi Bioconductor package21 and the log scale robust multi-array analysis expression measure was implemented with the affy packages) and mapped to Ensembl gene identifiers using re-annotation and mapping for oligonucleotide array technologies and custom chip definition file, respectively, before detection filtering and batch correction with cross-platform normalization. Biopsies were taken from the same subjects at three time-points: pre-treatment, after 10-14 days Letrozol (2.5 mg/day, oral), and after 90 days Letrozol (2.5 mg/day, oral).

*C) Assessment of complete pathological response (pCR; complete disappearance of tumour cells in breast and lymph node) after receiving pre-operative anthracycline-based combination chemotherapy therapy for ER+ locally advanced breast cancer patients (i.e., Examples 39 to 44)*

The association between *SPAG5* mRNA expression and pCR was evaluated in 2208 patients with locally advanced BC who received anthracycline based combination chemotherapy (E, AC, or FEC) with (+) or without (-) taxane (T). SPAG5 mRNA expression data has been retrieved from 11 publically available gene expression microarray datasets at the National Centre for Biotechnology Information Gene Expression Omnibus using the following access numbers.

| Database | Platform |
|----------|----------|
| GSE34138 | (HumanWG-6 v3.0- ILLUMINA; GPL6884) |
| GSE22226 | (Agilent-014850-Agilent; GPL1708) |
| GSE6861 | (HG-U133_X3P-Affymetrix; GPL1352) |
| GSE41998 | (HG-U133A_2-Affymetrix; GPL571) |
| GSE32646 | (HG-U133_Plus_2-Affymetrix; GPL570) |
| GSE66399 | (HG-U133_Plus_2- Affymetrix; GPL570) |
| GSE50948 | (HG-U133_Plus_2 Affymetrix; GPL570) |
| GSE20271 | (HG-U133A-Affymetrix; GPL96) |
| GSE42822 | (HG-U133A-Affymetrix; GPL96) |
| GSE37946 | (HG-U133A-Affymetrix; GPL96) |
| GSE20194 | (HG-U133A-Affymetrix; GPL96) |
| GSE25066 | (HG-U133A-Affymetrix; GPL96) |

The patients had received the following pre-operative chemotherapy.

| Treatment | No of patients |
|-----------|----------------|
| AC | 192 |
| AC-T | 262 |
| AC-T-H | 14 |
| ET | 59 |
| FEC | 195 |
| FEC-T | 595 |
| T-FEC | 522 |
| T-FEC-H | 176 |
| T-FEC-H-LAP | 22 |
| T-FEC-LAP | 31 |

| Treatment | No of patients |
|-----------|----------------|
| AC | 192 |
| AC-T | 262 |
| AC-T-H | 14 |
| ET | 59 |
| FEC | 195 |
| FEC-T | 595 |
| T-FEC | 522 |
| T-FEC-H | 176 |
| T-FEC-H-LAP | 22 |
| T-FEC-LAP | 31 |
| Total | 2208 |

Cut-off determination:

SPAG5 gene expression data of each database were converted to a common scale (median equal to 0 and standard deviation equal to 1) in order to merge all of the studies data that used the same platform and create combined cohorts. To convert to a common scale each sample in the data was standardised to a mean of 0 and standard deviation of 1. Then the data was median centred for each gene whereby median of each gene is 0. Databases using same platform have been merged and the median expression was calculated. The median expression of SPAG5 transcript for each platform has been calculated and values equal to or higher than the median coded as +1 (overexpression). Values of less than the median have been coded 0 or low SPAG5.

For HumanWG-6 v3.0- ILLUMINA; GPL6884 platform: SPAG5 median was 0.356 For HG-U133A_2-Affymetrix; GPL571 platform: SPAG5 median was 0.5505 For HG-U133_X3P-Affymetrix; GPL1352 platform: SPAG5 median was 0.3102 For Agilent-014850-Agilent; GPL1708 platform: SPAG5 median was 0.3373 For HG-U133A-Affymetrix; GPL96 platform: SPAG5 median was 0.5075 For HG-U133_Plus_2 - Affymetrix; GPL570 SPAG5 median was 0.4368 Ovarian Cancer: Assessment death and distant relapse risks after surgery and receiving adjuvant systemic therapy if eligible in ovarian epithelial cancer. The inventors evaluated the prognostic utility of SPAG5-mRNA expression in a large combined ovarian (OVC) cohort which was sourced from 14 publically-available, global datasets (n=1434) using the following access numbers: GSE14764, GSE15622, GSE18520, GSE19829, GSE23554, GSE26193, GSE26712, GSE27651, GSE30161, GSE3149, GSE51373, GSE63885, GSE9891 and TCGA. Data download and processed as mentioned before in breast cancer. Median cut off was 0.4

Lung cancer: Assessment death and distant relapse risks after surgery and receiving adjuvant systemic therapy if eligible in lung cancer. The inventors evaluated the prognostic utility of SPAG5-mRNA expression in a large combined lung cohort which was sourced from 14 publically-available, global datasets (n=1926) using the following access numbers: CAARRAY, GSE8894, GSE50081, GSE4573, GSE43580, GSE37745, GSE31908, GSE3141, GSE231210, GSE30219, GSE29013, GSE519188, GSE14814, and TCGA. Data download and processed similar to breast cancer.

Gastric cancer: Assessment death and distant relapse risks after surgery and receiving adjuvant systemic therapy if eligible in lung cancer. The inventors evaluated the prognostic utility of SPAG5-mRNA expression in a large combined lung cohort which was sourced from 6 publically-available, global datasets (n=876) using the following access numbers: GSE14216, GSE15459, GSE22377, GSE29272, GSE51105 and GSE62254. Data download and processed similar to breast cancer.

Liver cancer: The relationship between SPAG5 mRNA using SPAG5 RNA sequence ID 10615 and survival was analysed in 364 patients.

Immunohistochemistry (IHC) and tissue microarray (TMA) analysis of SPAG5 protein expression in HER2+ breast cancer was investigated by IHC analysis as four independent cohorts:

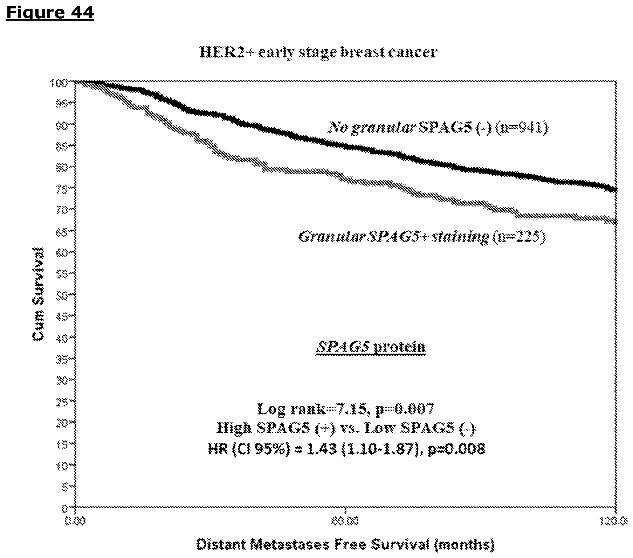

A) **Nottingham University Hospital Early Stage HER2 + Breast cancer (NUH-ES-HER2+ BC) cohort (Example 59 and figure 44).**

This cohort of HER2+ BC include patients (age>71 years) who were diagnosed and treated uniformly between 1986 and 2006 at the Nottingham City Hospital (NCH), Nottingham, UK. Patients within the good prognosis group (Nottingham Prognostic Index (NPI) <3.4) did not receive systemic adjuvant therapy. Pre-menopausal patients within the moderate and poor prognosis groups were candidates for CMF chemotherapy. Conversely, postmenopausal ER-positive patients with moderate or poor NPI were offered hormonal therapy, whereas ER-negative patients received CMF chemotherapy. None of the patients received trastuzumab. Clinical data were maintained on a prospective basis with a median follow-up of 143 months (Inter-quartile range (IQR) = 114-174). Survival data were maintained on a prospective basis. Breast cancer specific survival (BCSS) was defined as the number of months from diagnosis to the occurrence of breast cancer related death. Survival was censored if the patient was still alive, lost to follow-up, or died from other causes. Relapse free survival (RFS) was defined as the number of months from diagnosis to relapse either locally, regionally or distantly. Distant relapse free survival (DRFS) was defined as the number of months from diagnosis to distant metastases relapse.

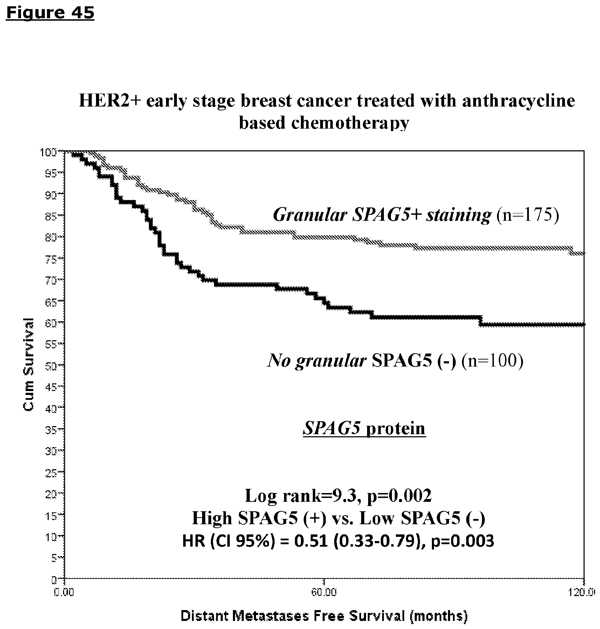

B) **Nottingham University Hospital Early Stage HER2+ anthracycline based combination chemotherapy treated breast cancer cohort (NUH- ES-HER2+ ACT-BC; n=275; Example 60 and figure 45)**

To evaluate the survival benefit of SPAG5-protein expression, its expression was analysed in a consecutive series of 275 early stage HER2+ BC's who had been diagnosed and managed at NCH between 2000 and 2006 who received adjuvant anthracycline-based combination chemotherapy. None of the patients received trastuzumab.

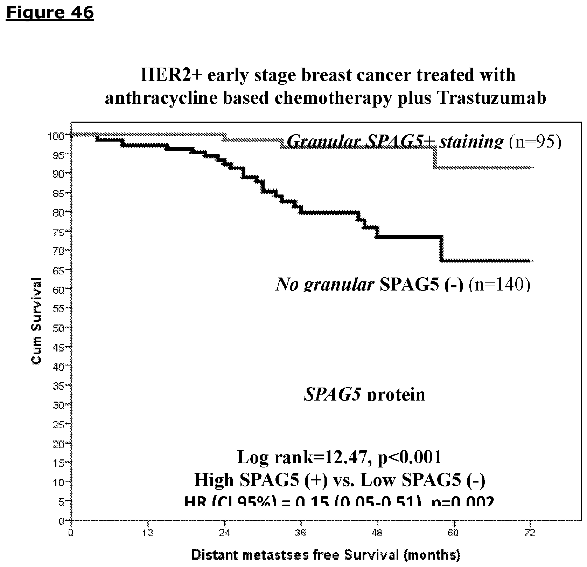

C) **Nottingham University Hospital Early Stage HER2+ Trastuzumab treated breast cancer cohort (NUH- ES-HER2+ HERCEPTIN-BC; n=235; Example 61 and figure 46)**

To evaluate the survival benefit of SPAG5-protein expression, its expression was analysed in a consecutive series of 235 early stages HER2+ BC's who had been diagnosed and managed at NCH between 2006 and 2010 who received adjuvant Trastuzumab with and without anthracycline-based combination chemotherapy and or Taxane.

**D) Nottingham University Hospital HER2+ Locally advanced BC (NUH-HER2+ LABC; n=162); Example 62**

The relationship between SPAG5-protein expression and response to chemotherapy was evaluated by investigating its expression in pair-matched pre-chemotherapy core biopsies and post-chemotherapy surgical specimens, from 162 female patients with HER2+ locally-advanced primary BC (HER2+ LAP-BC) (stage IIIA-C) that had been treated with anthracycline-based Neo-ACT (AC-Neo-ACT) with or without Taxane and with or without Trastuzumab at NCH between 1996 and 2015. Twenty-eight percent of patients received six cycles of an anthracycline-based therapy (FEC: 5-fluorouracil (5-FU) 500 mg m$^{-2}$, epirubicin 75-100 mg m$^{-2}$, cyclophosphamide 500 mg m$^{-2}$, on day 1 of a 21-day cycle), whereas 27% of patients received FEC plus Taxane. Forty five percent of patients have received Trastuzumab in addition to FEC plus Taxane. All patients underwent mastectomy or breast-conserving surgery and axillary dissection, followed by adjuvant radiation therapy. All patients offered one years of adjuvant Trastuzumab. Patients with HER2+ ER+ BCs were offered additionally 5 years of adjuvant endocrine therapy. The median follow-up time was 67 months (IRQ 27-81).

### SPAG5 transcript expression

**A) Adjuvant HER2+ Breast Cancer transcript cohort** - Assessment death and distant relapse risks after surgery and receiving adjuvant systemic therapy if eligible in early stage HER+ breast cancer (i.e., Examples 63, 64 and 65)

The inventors evaluated the prognostic utility of SPAG5-mRNA expression in a combined HER2+ breast cancer (BC) cohort which was sourced from 8 publically-available, global datasets (n=446) using the following access numbers.

| DATABASE | PLATFORM | | |
|---|---|---|---|
| GSE17907 | HG-UA133-PLUS2 | AFFYMETRIX | GPL570 |
| GSE21653 | HG-UA133-PLUS2 | AFFYMETRIX | GPL570 |
| GSE31448 | HG-UA133-PLUS2 | AFFYMETRIX | GPL570 |
| GSE45255 | HG-UA133A | AFFYMETRIX | GPL96 |
| GSE65092 | HG-UA219 | AFFYMETRIX | GPL13667 |
| GSE10510 | OLIG0-ARRAY-35K | DKFZ | GPL6486 |
| GSE16391 | AGILENT-014693 | AGILENT | GPL9128 |
| GSE19615 | AGILENT-014693 | AGILENT | GPL9128 |

All the databases had been downloaded and processed. The gene expression data were converted to a common scale (median equal to 0 and standard deviation equal to 1) in order to merge all of the studies data and create combined cohorts. Each sample in the data was standardised to a mean of 0 and standard deviation of 1. Then the data was median centred for each gene whereby median of each gene is 0. No systemic therapy has been prescribed for 132/436 of patients (30%) while 35% (155/436) of patients have received FEC plus Trastuzumab. FEC alone has been offered to 142/436 (33%) while CMF was given to 7 patients. Endocrine therapy has been offered to ER+ HER2+ patient (144/444).

**B) Assessment of complete pathological response** (pCR; complete disappearance of tumour cells in breast and lymph node) after receiving pre-operative anthracycline based combination chemotherapy therapy for HER2+ locally advanced breast cancer patients (i.e., Example 66)

The association between SPAG5 mRNA expression and pCR was evaluated in 476 patients with locally advanced BC who received anthracycline based combination chemotherapy (E, AC, or FEC) with (+) or without (-) taxane (T). Fifty one percent of patients (244/476) have offered FEC plus Taxane plus Trastuzumab while 43% (205/476) has received FEC plus Taxane. Only 6% (27/476) has been prescribed FEC.

SPAG5 mRNA expression data has been retrieved from 10 publically available gene expression microarray datasets at the National Centre for Biotechnology Information Gene Expression Omnibus using the following access numbers.

| Database | Platform |
|---|---|
| GSE41998 | (HG-U133A_2-Affymetrix; GPL571) |
| GSE32646 | (HG-U133_Plus_2-Affymetrix; GPL570) |
| GSE66399 | (HG-U133_Plus_2- Affymetrix; GPL570) |
| GSE50948 | (HG-U133_Plus_2 Affymetrix; GPL570) |
| GSE20271 | (HG-U133A-Affymetrix; GPL96) |

(continued)

| Database | Platform |
| --- | --- |
| GSE37946 | (HG-U133A-Affymetrix; GPL96) |
| GSE20194 | (HG-U133A-Affymetrix; GPL96) |
| GSE25066 | (HG-U133A-Affymetrix; GPL96) |
| GSE42827 | (HG-U133A-Affymetrix; GPL96) |
| GSE22226 | (agilent-014850; GPL1708) |

**Advanced Breast Cancer**

SPAG5 Immunohistochemistry (IHC) and HER2 protein and gene expression in locally advanced breast cancer was investigated by IHC analysis in three independent cohorts (examples 67-74).

The histopathological and radiological alterations of tumor characteristics after receiving neoadjuvant chemotherapy (NACT) was evaluated and the clinical significance of the changes of adjuvant therapy based on these findings was determined. Methods: A pathological assessment of tumor features including ER, PR, HER2 and proliferation markers (Ki67 and SPAG5) status in pre and post neoadjuvant tumors tissues was centrally evaluated in Nottingham University Hospital (NUH; n=465). Since 2013 any change in the ER and HER2 status from negative (-) [in the pre NACT biopsies] to positive (+) [in the post neoadjuvant surgical specimens] received additional adjuvant therapy (Endocrine therapy (ET) for ER+ and Trastuzumab for HER2+ cases). The primary end point was disease free survival (DFS). The relationship between SPAG5-protein expression and response to chemotherapy was evaluated by investigating its expression in pair-matched pre-chemotherapy core biopsies and post-chemotherapy surgical specimens. Twenty-eight percent of patients received six cycles of an anthracycline-based therapy (FEC: 5-fluorouracil (5-FU) 500 mg m$^{-2}$, epirubicin 75-100 mg m$^{-2}$, cyclophosphamide 500 mg m$^{-2}$, on day 1 of a 21-day cycle), whereas 27% of patients received FEC plus Taxane. Forty five percent of patients received Trastuzumab in addition to FEC plus Taxane. All patients underwent mastectomy or breast-conserving surgery and axillary dissection, followed by adjuvant radiation therapy. All patients offered one years of adjuvant Trastuzumab if HER2+. Patients with ER+ BCs were offered additionally 5 years of adjuvant endocrine therapy. The median follow-up time was 67 months (IRQ 27-81).

Example 1 - The intracytoplasmic granular (punctate) staining pattern of SPAG5 protein in early-stage breast cancer

Granules of SPAG5 protein have been detected in the cytoplasm of cells of breast cancer tissue stained using immunohistochemistry (see Figures 1 to 4). The formation of granules of SPAG5 is believed to be due to the overexpression of SPAG5 protein.

Example 2 - The negative or low SPAG5 protein expression

The absence of staining or presence of faint or diffuse intracytoplasmic SPAG5 staining have been detected in the cytoplasm of breast cancer tissues stained using immunohistochemistry (see Figures 5A-5B)

Example 3 - Granular SPAG5 protein expression in early-stage breast cancer (Clinical outcome measurement: risk of death)

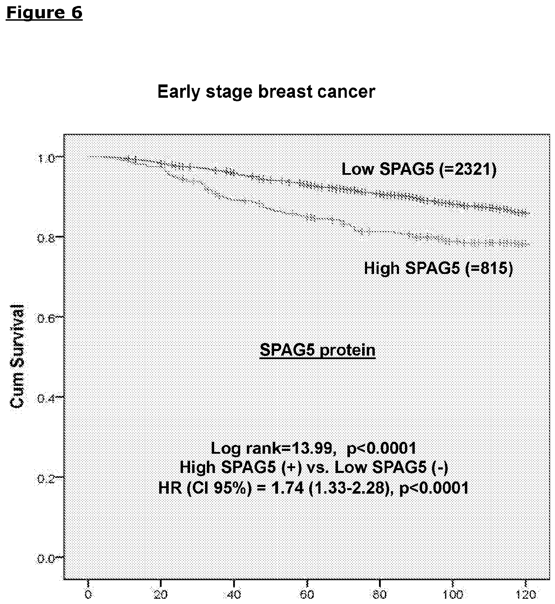

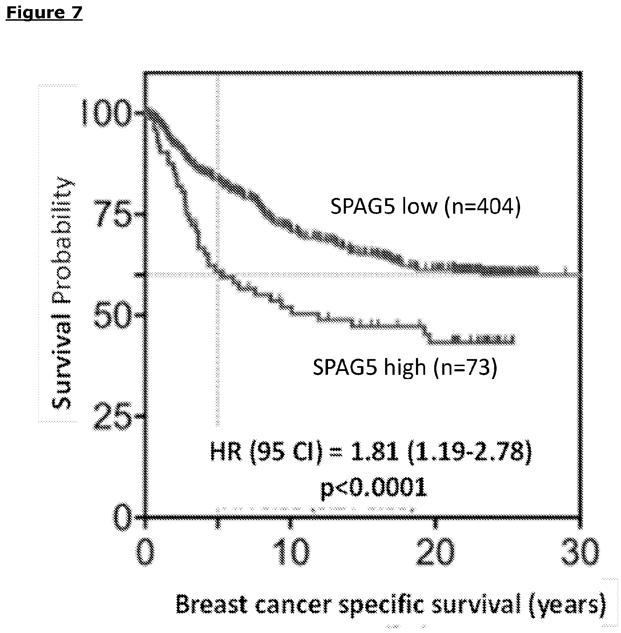

Early-stage breast cancer patients with SPAG5 overexpression in their cancerous tissue at the time of surgery had 2-fold increase in the risk of death from BC at 10-year of follow up after the time of surgery compared to those with no SPAG5 protein overexpression (i.e., no expression of granular SPAG5 protein) (see Figures 6 and 7).

Example 4 - Granular SPAG5 protein expression in early-stage ER+ breast cancer (Clinical outcome measurement: risk of death)

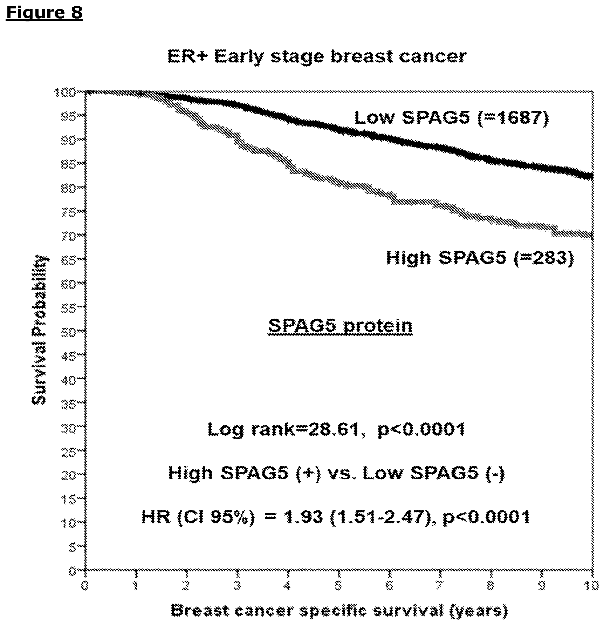

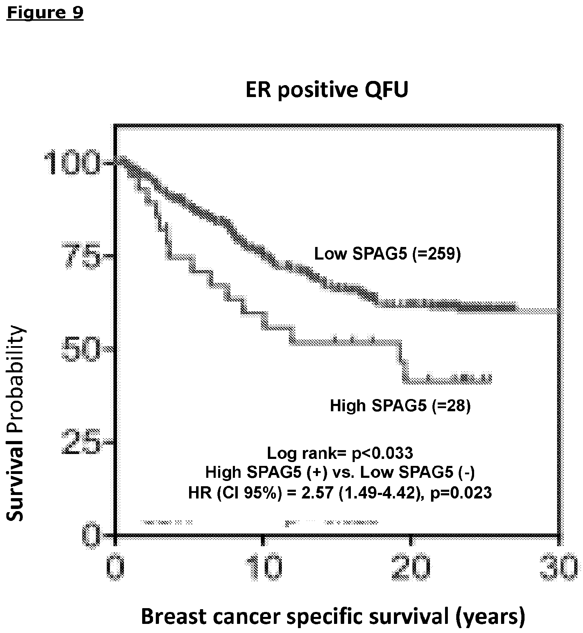

ER+ Early-stage breast cancer patients with SPAG5 overexpression in their cancerous tissue at the time of surgery had 2-fold increase in the risk of death from BC at 10-year of follow up after the time of surgery compared to those with low SPAG5 protein overexpression (i.e., no staining or faint or diffuse staining (no intracytoplasmic punctate expression of SPAG5 protein) (see Figures 8 and 9).

Example 5 - Granular SPAG5 protein expression in early stage breast cancer (Clinical outcome measurements & interaction analysis with other prognostic factors)

In early stage BC, the interaction analysis (using multivariate Cox regression model) of SPAG5 protein with other validated prognostic factors confirmed that SPAG5+ protein overexpression was independently associated with 2 fold increase in the risk of death from breast cancer at 10 year of follow up after the time of surgery compared to those with low SPAG5 protein expression (i.e. no staining or faint or diffuse staining (no intracytoplasmic punctate expression of SPAG5 protein) (see Table 1).

**TABLE 1 - QFU cohort cox regression analysis**

| Parameters | Univariate analysis (Log-rank or Gehan-Breslow Wilcoxon*) | | | | Multivariate analysis (Stepwise cox proportional-hazards) | | |
|---|---|---|---|---|---|---|---|
| | No death/no survivor | Rate | Differential | p | HR | 95% CI | p |
| **Lymph Node Status** Negative | 100/133 | 0.25 | | <0.0001 | | | 0.0001 |
| Positive | 40/108 | 0.63 | 2.54 | | 2.69 | 1.63 -4.46 | |
| **HER2 Status** Negative Overexpression | 291/445 17/57 | 0.35 0.70 | 2.03 | <0.0001 | 2.73 | 1.51 -4.93 | 0.0009 |
| **Histological Grade** G1 G2 G3 | 60/74 152/249 97/171 | 0.19 0.39 0.43 | 2.06 | <0.0001 | | | NS |
| **Lymphovascular Invasion** Absent Present | 274/413 51/109 | 0.34 0.53 | 1.58 | <0.0001 | | | NS |
| **SPAG5 Expression** Low Overexpression | 262/404 34/73 | 0.35 0.53 | 1.52 | <0.0001 | 1.92 | 1.11 -3.35 | 0.021 |
| **Tumour Size** <= 1.5 cm > 1.5 | 42/156 90/208 | 0.27 0.43 | 1.29 | 0.0005 | 2.4 | 1.25-4.6 | 0.009 |
| **Ki67 expression** Low High | 259/409 33/64 | 0.37 0.48 | 1.32 | 0.0005 | | | NS |
| **ER status** Negative Positive | 114/195 204/319 | 0.42 0.36 | 1.15 | 0.0015* | | | NS |
| **Triple negativ (TN) status** Non TN TN | 257/411 60/101 | 0.37 0.41 | 1.08 | 0.014* | | | NS |
| Gehan-Breslow-Wilcoxon p values are shown for parameters with most prognostic significance within the first 5 years post-diagnosis | | | | | | | |

Example 6 - Granular SPAG5 protein expression in early-stage ER+ breast cancer (Clinical outcome measurement: risk of distant relapse)

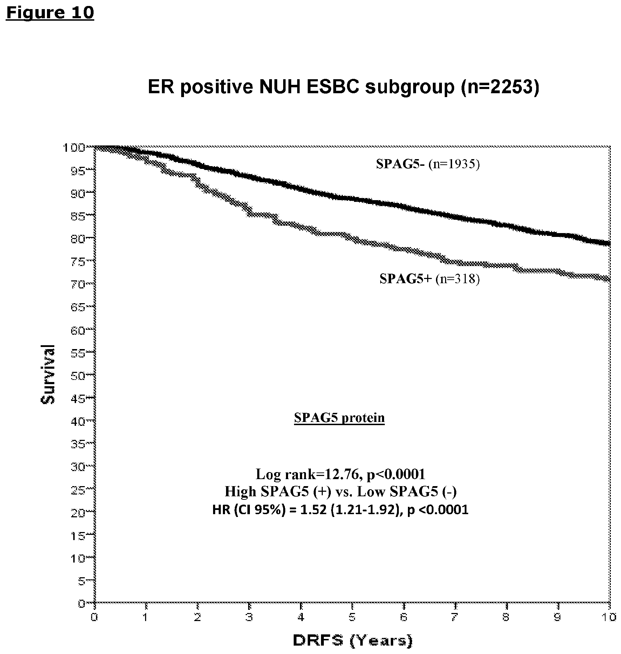

ER+ Early-stage breast cancer patients with SPAG5 overexpression in their cancerous tissue at the time of surgery had 1.5-fold increases in the risk of distant relapse at 10-year of follow up after the time of surgery compared to those with low SPAG5 protein expression (i.e., no staining or faint or diffuse staining (no intracytoplasmic punctate expression of SPAG5 protein) (see Figure 10).

Example 7 - Granular SPAG5 protein expression in early stage ER+LN- breast cancer that has not been treated with any systemic therapy (Clinical outcome measurement: risk of distant relapse)

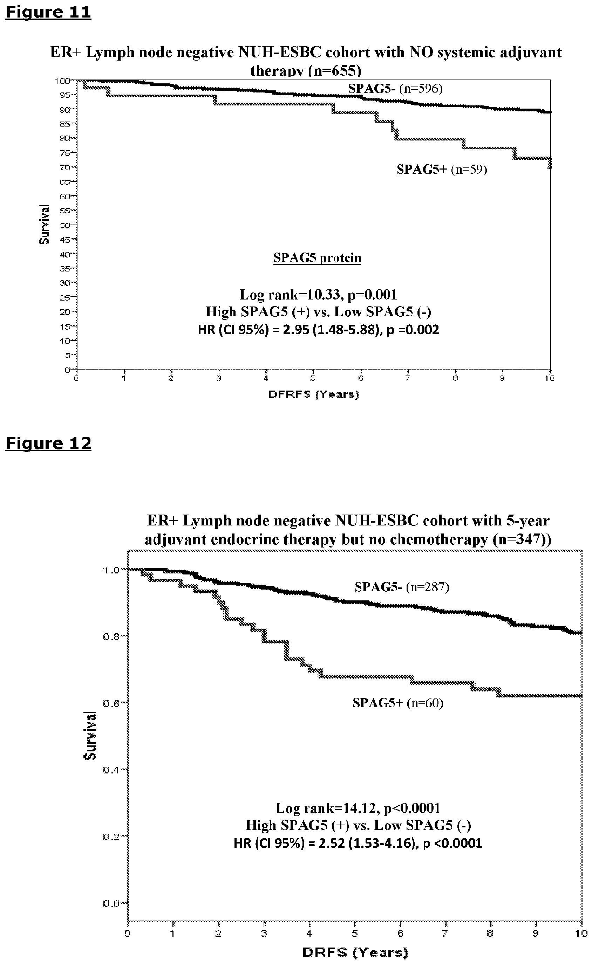

In ER+ LN- early stage BC patients (i.e. patients that have a breast cancer that has not spread to the lymph nodes (LN-) but contains cells that express the oestroegen receptor (ER+)) who did not receive any systemic therapy, SPAG5 protein overexpression in the cancerous tissue at the time of surgery was associated with a three-fold increase of distant relapse risk at 5 years follow up compared to low SPAG5 protein expression (i.e. no staining or faint or diffuse staining (no intracytoplasmic punctate expression of SPAG5 protein) (see Figure 11).

Example 8 - Granular SPAG5 protein overexpression in early-stage ER+LN- breast cancer that has been treated with Tamoxifen therapy only (Clinical outcome measurement: risk of distant relapse)

In ER+ LN- early-stage BC patients who received 5-year adjuvant endocrine therapy (Tamoxifen) alone, SPAG5 protein overexpression in the cancerous tissue at the time of surgery was associated with 2.5-fold increase of distant relapse risk at 5 years follow up compared to low SPAG5- protein expression (i.e., no staining or faint or diffuse staining (no intracytoplasmic punctate expression of SPAG5 protein) (see Figure 12).

Example 9 - SPAG5 protein overexpression in early-stage ER+LN- breast cancer that has been treated with adjuvant AC therapy with or without taxane chemotherapy in addition to Tamoxifen therapy Clinical outcome measurement: risk of distant relapse)

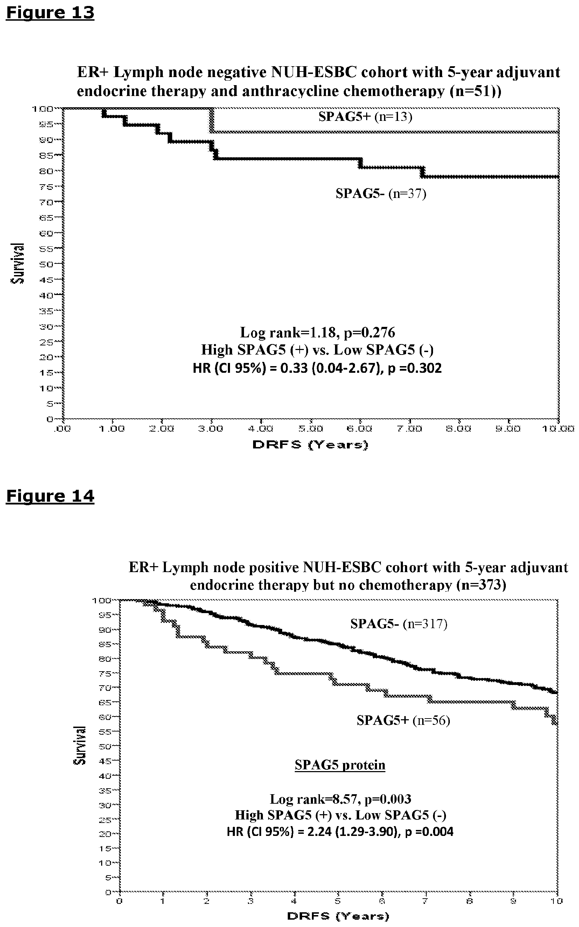

In ER+LN- early stage BC patients who received adjuvant anthracycline based combination (AC) therapy with or without taxane chemotherapy in addition to adjuvant 5-year endocrine therapy (Tamoxifen), SPAG5 protein overexpression in the cancerous tissue at the time of surgery had a similar distant relapse risk at 5 years follow up after surgery to low SPAG5 protein expression (i.e. no staining or faint or diffuse staining (no intracytoplasmic punctate expression of SPAG5 protein) (see Figure 13).

Example 10 - Granular SPAG5 protein expression in early-stage ER+LN+ breast cancer that has been treated with Tamoxifen therapy only (Clinical outcome measurement: risk of distant relapse)

In ER+ LN+ early-stage BC patients who received 5-year adjuvant endocrine therapy (Tamoxifen) alone, SPAG5 protein overexpression in the cancerous tissue at the time of surgery was associated with 2-fold increase of distant relapse risk at 5 years follow up after surgery compared to low SPAG5 protein expression (i.e., no staining or faint or diffuse staining (no intracytoplasmic punctate expression of SPAG5 protein) (see Figure 14).

Example 11 - Granular SPAG5 protein expression in early-stage ER+LN+ breast cancer that has been treated with CMF chemotherapy therapy and Tamoxifen (Clinical outcome measurement: risk of distant relapse)

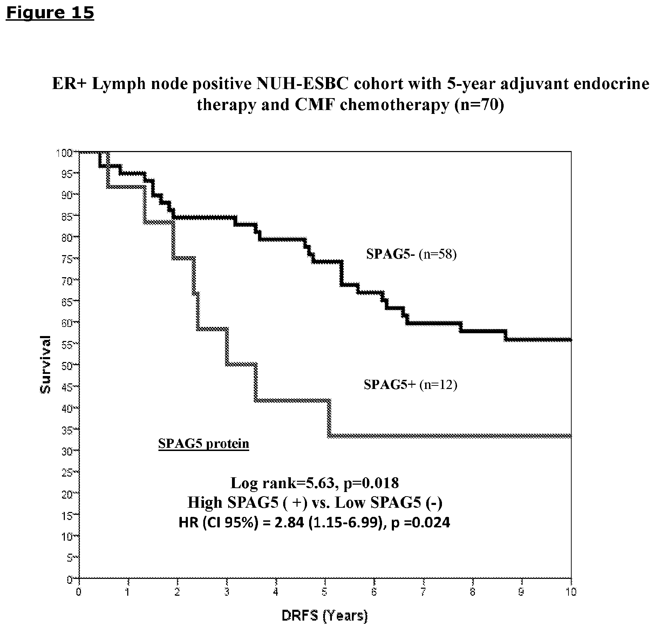

In ER+LN+ early stage BC patients who received adjuvant CMF (cyclophosphamide, methotrexate and 5-fluorouracil) chemotherapy in addition to adjuvant 5-year endocrine therapy (Tamoxifen), SPAG5 protein overexpression in the cancerous tissue at the time of surgery was associated with 3-fold increase of distant relapse risk at 5 years follow up after surgery compared to low SPAG5 protein expression (i.e. no staining or faint or diffuse staining)(see Figure 15).

Example 12 - Granular SPAG5 protein expression in early stage ER+LN+ breast cancer that has been treated with AC therapy with or without taxane chemotherapy in addition to Tamoxifen therapy (Clinical outcome measurement: risk of distant relapse)

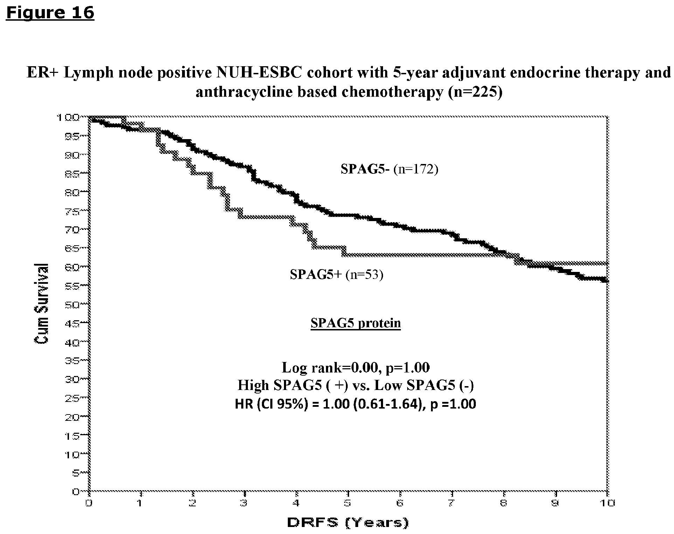

In ER+LN+ early-stage BC patients who received adjuvant anthracycline based combination with or without taxane chemotherapy in addition to adjuvant 5-year endocrine therapy (Tamoxifen), SPAG5 protein overexpression in the cancerous tissue at the time of surgery had similar distant relapse risk at 5 years of follow up after surgery to low SPAG5 protein expression (i.e., no staining or faint or diffuse staining (no intracytoplasmic punctate expression of SPAG5 protein) (see Figure 16).

Example 13 - Granular SPAG5 protein expression in early-stage breast cancer (Clinical outcome measurements & interaction analysis with systemic therapy and other prognostic factors)

In early stage BC, the interaction analysis (using multivariate Cox regression model) of SPAG5 protein expression with systemic therapy and other validated prognostic factors confirmed that SPAG5+ protein overexpression was independently associated with 2 fold increase in the risk of distant relapse at 5 year of follow up after the time of surgery compared to those with low SPAG5 protein expression (i.e. no staining or faint or diffuse staining (no intracytoplasmic punctate expression of SPAG5 protein) (see Table 2 below).

**Table 2:** Multivariable Cox regression models analysis for 5 year distance relapse risk (DRR) in ER positive NUH ESBC cohort

| Variables | HR | 95·0% CI | | P value |
| --- | --- | --- | --- | --- |
| | | Lower | Upper | |
| **SPAG5 protein expression (positive)** | 1·68 | 1·18 | 2·93 | **0·004**\* |
| **Ki67 protein expression** | 0·71 | 0·47 | 1·06 | 0·093 |
| **TOP2A protein expression** | 0·91 | 0·65 | 1·27 | 0·57 |
| **Tumour size (continuous)** | 1·59 | 1·23 | 2·07 | **<0·0001** \* |
| **Lymph node (LN) status** Negative Positive | 1 1·72 | 1-44 | 2·05 | **<0·0001**\* |
| **Histological grade** Low/intermediate High | 1 2·63 | 2·10 | 3·30 | **<0·0001**\* |
| **HER2 overexpression (positive)** | 1·68 | 1·22 | 2·32 | **0·004**\* |
| **Anthracycline Chemotherapy (Yes)** | 0·68 | 0·48 | 0·94 | **0·022**\* |
| **Endocrine therapy (Yes)** | 0·76 | 0·56 | 1·03 | **0·079**\* |
| \*Statistically significant at p<0·05. | | | | |

SPAG5; Sperm-associated antigen, ER; Oestrogen receptor; HER2; Human epidermal growth factor receptor 2.

Example 14 - Pathological complete response (pCR) of locally advanced breast cancer (with granular SPAG5 protein expression at the time of diagnosis) to preoperative treatment with AC therapy alone (AC), AC with taxane (AC+T) and AC+T with Herceptin® (AC+T+H) (SPAG5 protein analysis)

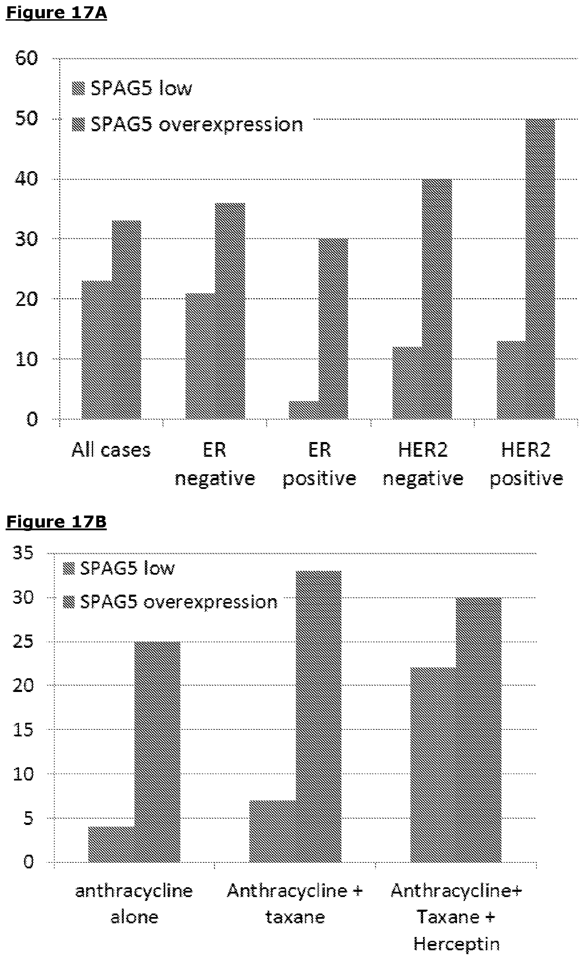

In locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 2-fold higher rate of complete disappearance of the cancer cells in the breast and lymph node (i.e., pathological complete response) in the surgically removed specimen after receiving preoperative anthracycline based combination chemotherapy (AC) with or without taxane (36% (45/126) vs., 21% (16/77); odd ratio (OR) (CI 95%) = 5.78 (3.47-9.63); p<0.0001) (see Figure 17A).

Example 15 - Pathological complete response (pCR) of locally advanced ER- breast cancer (with increased granular SPAG5 protein expression at time of surgery) to preoperative treatment with AC therapy with or without Taxane (SPAG5 protein analysis)

In ER negative locally advanced breast cancer, compared to low SPAG5 protein expression (no intracytoplasmic punctate expression of SPAG5 protein)in the tumour biopsy that had been taken from patients at the time of the diagnosis and before receiving any treatment, SPAG5 protein overexpression (i.e. The intracytoplasmic granular (punctate) staining pattern of SPAG5 protein) predicted 6-fold higher rate of complete disappearance of the cancer cells in the breast and lymph node (i.e., pathological complete response) in the surgically removed specimen after receiving preoperative anthracycline based combination chemotherapy (AC) with or without taxane (33% (73/219) vs., 8% (23/289); odd ratio (OR) (CI 95%) = 2.12 (1.10-4.10); p=0.024) (see Figure 17A).

Example 16 - Pathological complete response (pCR) of locally advanced ER+ breast cancer (with increased granular SPAG5 protein expression at time of surgery) to preoperative treatment with AC therapy with or without Taxane (SPAG5 protein analysis)

In ER positive locally advanced breast cancer, compared to low SPAG5 protein expression (no intracytoplasmic punctate expression of SPAG5 protein)in the tumour biopsy that had been taken from patients at the time of the diagnosis and before receiving any treatment, SPAG5 protein overexpression (i.e. The intracytoplasmic granular (punctate) staining pattern of SPAG5 protein) predicted 13-fold higher rate of complete disappearance of the cancer cells in the breast and lymph node (i.e., pathological complete response) in the surgically removed specimen after receiving preoperative anthracycline based combination chemotherapy (AC) with or without taxane (30% (28/93) vs., 3% (7/212); odd ratio (OR) (CI 95%) = 12.62 (5.26-30.23); p<0.0001)(see Figure 17A).

Example 17 - Pathological complete response (pCR) of locally advanced HER2-breast cancer (with increased granular SPAG5 protein expression at time of surgery) to preoperative treatment with AC therapy with or without Taxane (SPAG5 protein analysis)

In HER2 negative locally advanced breast cancer, compared to low (no intracytoplasmic punctate expression of SPAG5 protein) SPAG5 protein expression in the tumour biopsy that had been taken from patients at the time of the diagnosis and before receiving any treatment, SPAG5 protein overexpression (i.e. The intracytoplasmic granular (punctate) staining pattern of SPAG5 protein) predicted 6-fold higher rate of complete disappearance of the cancer cells in the breast and lymph node (i.e., pathological complete response) in the surgically removed specimen after receiving preoperative anthracycline based combination chemotherapy (AC) with or without taxane (27% (40/149) vs., 6% (12/199); odd ratio (OR) (CI 95%) = 5.72 (2.88-11.37); p<0.0001) (see Figure 17A).

Example 18 - Pathological complete response (pCR) of locally advanced HER2+ breast cancer (with increased granular SPAG5 protein expression at time of surgery) to preoperative treatment with AC therapy with or without Taxane (SPAG5 protein analysis)

In HER2 positive locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 7-fold higher rate of complete disappearance of the cancer cells in the breast and lymph node (i.e., pathological complete response) in the surgically removed specimen after receiving preoperative anthracycline based combination chemotherapy (AC) with or without taxane (50% (30/60) vs., 13% (10/80); odd ratio (OR) (CI 95%) = 7.00 (3.04-16.11); p<0.0001) (see Figure 17A).

Example 19 - Pathological complete response (pCR) of locally advanced breast cancer (with increased granular SPAG5 protein expression at time of surgery) to preoperative treatment with AC therapy (SPAG5 protein analysis)

In locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 9-fold higher rate of complete disappearance of the cancer cells in the breast and lymph node (i.e., pathological complete response) in the surgically removed specimen after receiving preoperative anthracycline based combination chemotherapy (AC) alone (28% (25/89) vs., 4% (4/93); odd ratio (OR) (CI 95%) = 8.70 (2.88-26.20); p<0.0001) (see Figure 17B).

Example 20 - Pathological complete response (pCR) of locally advanced breast cancer (with increased granular SPAG5 protein expression at time of surgery) to preoperative treatment with taxane and AC therapy (SPAG5 protein analysis)

In locally advanced breast cancer, compared to low SPAG5 protein expression in the tumour biopsy that had been taken from patients at the time of the diagnosis and before receiving any treatment, SPAG5 protein overexpression predicted 7-fold higher rate of complete disappearance of the cancer cells in the breast and lymph node (i.e., pathological complete response) in the surgically removed specimen after receiving preoperative taxane in addition to anthracycline based combination chemotherapy (33% (34/103) vs., 7% (11/160); odd ratio (OR) (CI 95%) = 6.70 (3.19-13.95); p<0.0001) (see Figure 17B).

Example 21 - Pathological complete response (pCR) of locally advanced HER2+ breast cancer (with increased granular SPAG5 protein expression at time of surgery) to preoperative treatment with trastuzumab, taxane and AC therapy (SPAG5 protein analysis)

In HER2 positive locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 4-fold higher rate of complete disappearance of the cancer cells in the breast and lymph node (i.e., pathological complete response) in the surgically removed specimen after receiving preoperative trastuzumab and taxane in addition to anthracycline based combination chemotherapy (52% (14/27) vs., 22% (8/36); odd ratio (OR) (CI 95%) = 3.80 (1.27-11.21); p=0.015) (see Figure 17B).

Example 22 - Pathological complete response (pCR) of locally advanced ER+ breast cancer (with increased granular SPAG5 protein expression at time of surgery) to preoperative treatment with AC therapy (SPAG5 protein analysis)

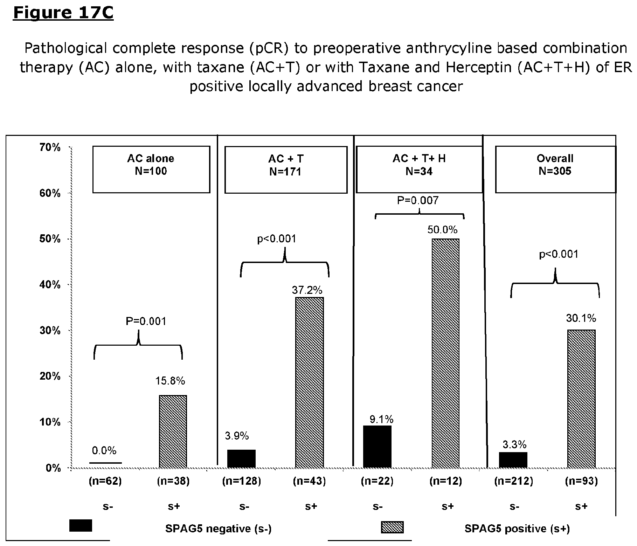

In ER positive locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 16 fold higher rate of complete disappearance of the cancer cells in the breast and lymph node (i.e., pathological complete response) in the surgically removed specimen after receiving preoperative anthracycline based combination chemotherapy (AC) alone (16% (6/38) vs., 0% (0/62); p=0.001) (see Figure 17C).

Example 23 - Pathological complete response (pCR) of locally advanced ER+ breast cancer (with increased granular SPAG5 protein expression at time of surgery) to preoperative treatment with taxane and AC therapy. (SPAG5 protein analysis)

In ER positive locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 15-fold higher rate of complete disappearance of the cancer cells in the breast and lymph node (i.e., pathological complete response) in the surgically removed specimen after receiving preoperative taxane in addition to anthracycline based combination chemotherapy (37% (16/43) vs., 4% (5/128); odd ratio (OR) (CI 95%) = 14.60 (4.92-43.24); p<0.0001) (see Figure 17C).

Example 24 - Pathological complete response (pCR) of locally advanced ER+HER2+ breast cancer (with increased granular SPAG5 protein expression at time of surgery) to preoperative treatment with taxane and AC therapy (SPAG5 protein analysis)

In ER positive/Her2 positive locally advanced breast cancer, compared to low SPAG5 protein expression (no intracytoplasmic punctate expression of SPAG5 protein) in the tumour biopsy that had been taken from patients at the time of the diagnosis and before receiving any treatment, SPAG5 protein overexpression (i.e. The intracytoplasmic granular (punctate) staining pattern of SPAG5 protein) predicted 10-fold higher rate of complete disappearance of the cancer cells in the breast and lymph node (i.e., pathological complete response) in the surgically removed specimen after receiving preoperative taxane in addition to anthracycline based combination chemotherapy (37% (16/43) vs., 4% (5/128); odd ratio (OR) (CI 95%) = 10.00 (1.60-63.10); p=0.0007) (see Figure 17C).

In summary SPAG5 protein overexpression is associated with higher pCR regardless the molecular subclass of breast cancer after receiving either Anthracycline based combination chemotherapy alone or with Taxane or with Taxane and Herceptin®.

Example 25- SPAG5 transcript expression in early-stage breast cancer

SPAG5+ mRNA expression was observed in 43% of ER+ early-stage BC tissue at the time of surgery. SPAG5 transcript overexpression is more common in PAM-50 luminal B, PAM-50 HER2+ breast cancer than in luminal A, TP53 mutation, BRCA2 mutation, highly genomic instable integrative clusters (1, 2, 5, 6, 9 and 10), Luminal genomic complex and 17q12 genomic patterns and 8qcis-acting/20q amplification.

Example 26 - SPAG5 transcript expression in early-stage ER+ breast cancer (Clinical outcome measurement: risk of distant relapse)

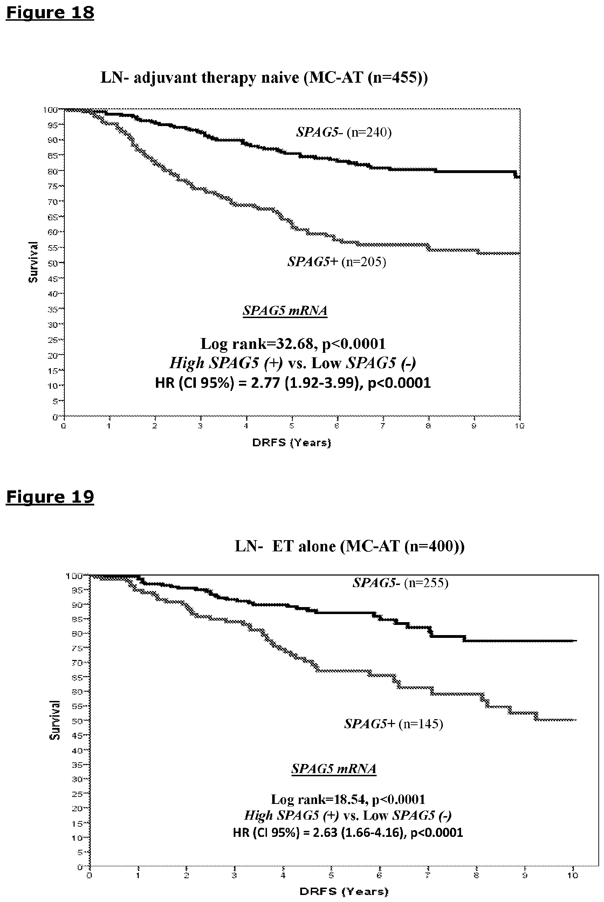

In ER+ early-stage breast cancer, at 10 years of follow up after surgically removing the primary breast cancer regardless of the other risk factors and did not receive adjuvant systemic therapy, SPAG5 overexpression in breast cancer tissue at the time of surgery was associated with 2-fold increase in the distant relapse risk compared to low SPAG5 mRNA expression (see Figure 18).

Example 27 - SPAG5 transcript expression in early-stage ER+LN- breast cancer that has been treated with Tamoxifen therapy only (Clinical outcome measurement: risk of distant relapse)

In ER+ early-stage BC patients with lymph node negative, after receiving 5-year of adjuvant endocrine therapy (Tamoxifen), breast cancers that exhibited overexpression of SPAG5+ mRNA at the time of surgery had 3-fold increase of distant relapse risk compared to those with low SPAG5 (see Figure 19).

Example 28 - SPAG5 transcript over-expression in early-stage ER+LN- breast cancer that has been treated with AC therapy followed by Tamoxifen therapy vs no systemic therapy (Clinical outcome measurement: risk of distant relapse)

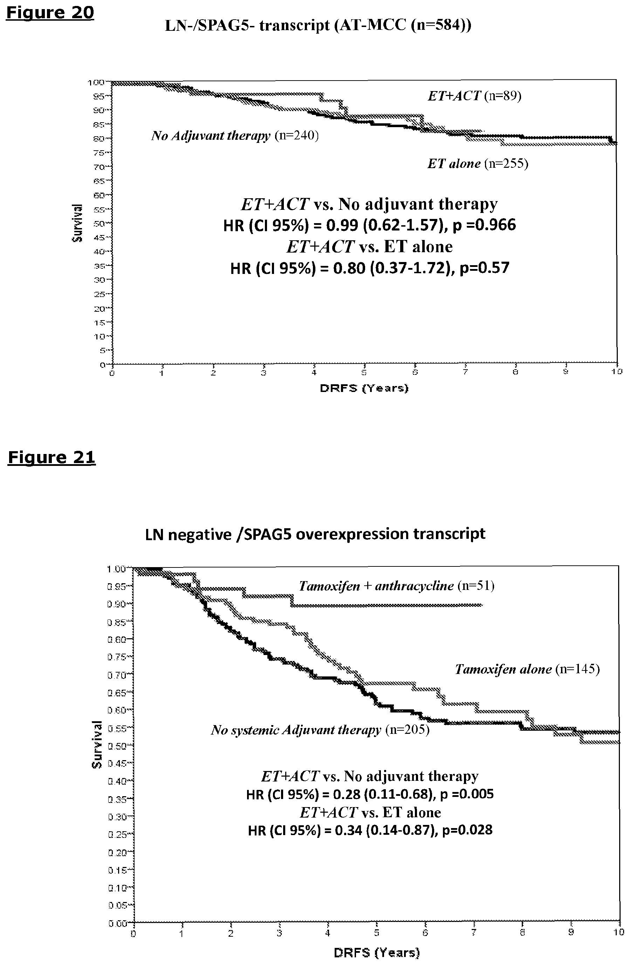

In ER+ early-stage BC patients with lymph node negative, after receiving adjuvant anthracycline combination chemotherapy followed by 5-year-adjuvant endocrine therapy (Tamoxifen), breast cancers that exhibited overexpression of SPAG5+ mRNA at the time of surgery had similar distant relapse risk compared to those who did not receive any systemic therapy (see Figure 20).

Example 29 - SPAG5 transcript overexpression in early-stage ER+LN- breast cancer that has been treated with Tamoxifen and anthracycline vs tamoxifen alone (Clinical outcome measurement: risk of distant relapse)

In ER+ early stage BC patients with lymph node negative and low SPAG5 mRNA expression at the time of surgery, receiving 5-year-adjuvant endocrine therapy (Tamoxifen) alone had the similar distant relapse risk of receiving adjuvant anthracycline combination chemotherapy in addition to the 5-year-adjuvant endocrine therapy (Tamoxifen) (13% vs 15%)(see Figure 20).

Example 30 - SPAG5 transcript overexpression in early-stage ER+LN- breast cancer patients that has been treated with AC and tamoxifen therapy vs tamoxifen only (Clinical outcome measurement: risk of distant relapse)

In ER+ early stage BC patients with lymph node negative and SPAG5+ mRNA overexpression at the time of surgery, receiving adjuvant anthracycline combination chemotherapy in addition to the 5-year-adjuvant endocrine therapy (Tamoxifen), had reduced the distant relapse risk by 3-fold (11%) compared to receiving the 5-year-adjuvant endocrine therapy (Tamoxifen) alone (33%)(see Figure 21).

Example 31 - SPAG5 transcript expression in early-stage ER+LN- breast cancer that has been treated with AC and tamoxifen therapy vs no systemic therapy (Clinical outcome measurement: risk of distant relapse)

In ER+ early-stage BC patients with lymph node negative and SPAG5+ mRNA overexpression at the time of surgery, receiving adjuvant anthracycline combination chemotherapy in addition to 5-year-adjuvant endocrine therapy (Tamoxifen), had reduced the distant relapse risk (11%) by 4-fold compared to receiving no systemic therapy (39%) (see Figure 21).

Example 32- SPAG5 transcript expression in early-stage ER+LN+ breast cancer that has been treated with tamoxifen therapy only (Clinical outcome measurement: risk of distant relapse)

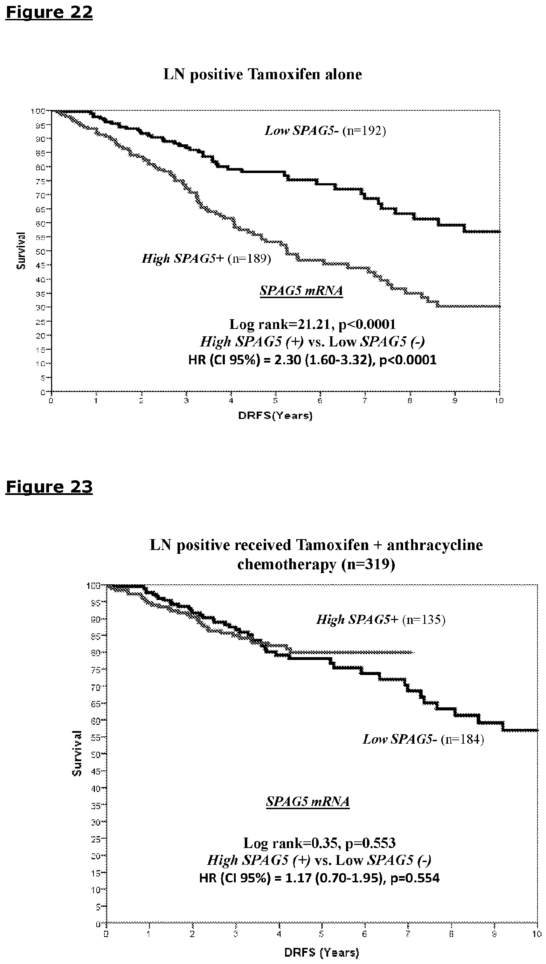

In ER+ early-stage BC patients with lymph node positive (LN+), after receiving 5-year-Adjuvant endocrine therapy (Tamoxifen) alone, breast cancers that exhibited overexpression of SPAG5+ mRNA at the time of surgery had a two-fold higher distant relapse risk compared to those with low SPAG5 (see Figure 22).

Example 33 - SPAG5 transcript expression in early-stage ER+LN+ breast cancer that has been treated with AC and tamoxifen therapy (Clinical outcome measurement: risk of distant relapse)

In ER+ early-stage BC patients with lymph node positive (LN+) after receiving adjuvant anthracycline combination chemotherapy in addition to 5-year-adjuvant endocrine therapy (Tamoxifen), tumours that exhibited overexpression of SPAG5+ mRNA had a similar distant relapse risk compared to those with low SPAG5 mRNA expressing (see Figure 23).

Example 34 - SPAG5 transcript over-expression in early-stage ER+LN+ breast cancer that has been treated with AC and tamoxifen therapy (Clinical outcome measurement: risk of distant relapse)

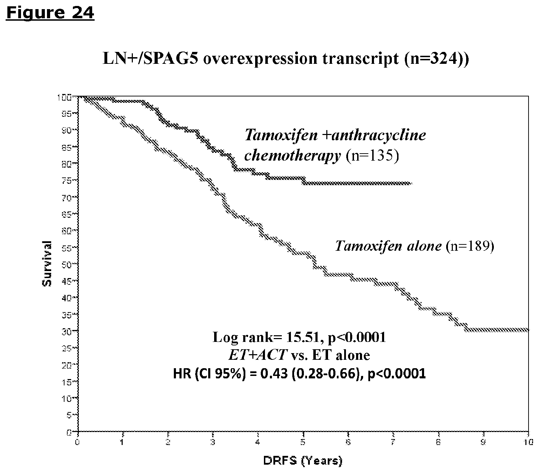

In ER+ early-stage BC patients with lymph node positive (LN+) and SPAG5+ mRNA overexpression at the time of surgery, receiving adjuvant anthracycline combination chemotherapy in addition to the 5-year-adjuvant endocrine therapy (Tamoxifen), had reduced the distant relapse risk by 2-fold (24%) compared to receiving the-5-year-adjuvant endocrine therapy (Tamoxifen) alone (46%) (see Figure 24).

Example 35 - SPAG5 transcript expression in early-stage ER+LN+ breast cancer that has been treated with tamoxifen therapy only (Clinical outcome measurement: risk of distant relapse)

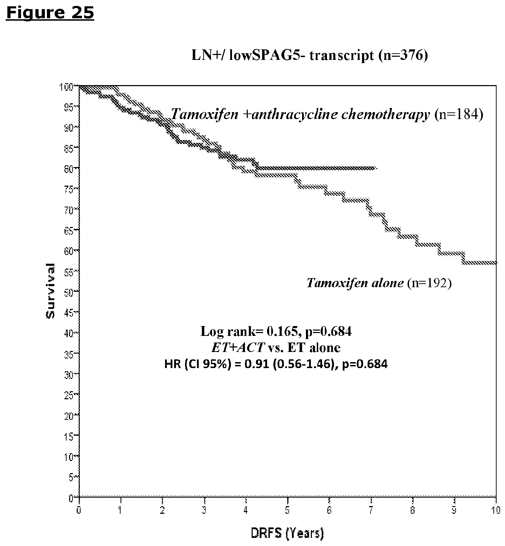

In ER+ early-stage BC patients with lymph node positive (LN+) and low SPAG5+ mRNA at the time of surgery, receiving 5-year-adjuvant endocrine therapy (Tamoxifen) alone had similar distant relapse risk of receiving adjuvant anthracycline combination chemotherapy in addition to the 5-year-adjuvant endocrine therapy (Tamoxifen), (22% vs 20%) (see Figure 25).

Example 36 - SPAG5 transcript expression in early-stage breast cancer (Clinical outcome measurements & interaction analysis with systemic therapy and other prognostic factors)

In ER+ early-stage BC, the interaction analysis (using multivariate Cox regression model) of SPAG5 mRNA with systemic therapy and other validated prognostic factors confirmed that SPAG5+ mRNA expression was a better independent biomarker for predicting poor 5-year distant relapse risk.

In ER+ early-stage BC, the interaction analysis (using multivariate Cox regression model) between SPAG5 mRNA with anthracycline based combination chemotherapy therapy has confirmed that SPAG5 mRNA is both a prognostic and predictive biomarker 5-year distant relapse risk.

In ER+ early-stage BC, the interaction analysis (using multivariable cox regression) of SPAG5 mRNA with Nottingham Prognostic Index (NPI) has revealed that SPAG5+ mRNA was a better independent biomarker for predicting 5-year distant relapse risk.

In ER+ early-stage BC, the interaction analysis (using multivariable cox regression) of SPAG5 mRNA with 72-proliferation-gene-signature has revealed that SPAG5+ mRNA was a better independent biomarker for predicting 5-year distant relapse risk.

In ER+ early-stage BC, the interaction analysis (using multivariable cox regression) of SPAG5 mRNA with 76-gene prognostic signature (Veridex) has revealed that SPAG5+ mRNA was a better independent biomarker for predicting 5-year distant relapse risk.

In ER+ early-stage BC, the interaction analysis (using multivariable cox regression) of SPAG5 mRNA with PAM-50-molecular subgroups has revealed that SPAG5+ transcript was a better independent biomarker for predicting 5-year distant relapse risk (Table 3).

**Table 3:** Multivariable Cox regression models analysis for 5 year distance relapse risk (DRR) in in the multicentre adjuvant therapy (MC-AT) cohort

| Multicentre adjuvant therapy (MC-AT) cohort (Transcript) | | | | |
|---|---|---|---|---|
| **A. Overall Model** | | | | |
| **Variables** | **HR** | **95·0% CI** | | **P value** |
| | | **Lower** | **Upper** | |
| *SPAG5* mRNA (high) | 2.59 | 1·69 | 3·97 | **<0·0001 *** |
| *MKI67* mRNA (high) | 0.99 | 0.49 | 1.71 | 0·77 |
| **Lymph node status (positive)** | 1·81 | 1·01 | 1·29 | **0·001*** |
| **Tumour size** <br> **< = 2cm** <br> **>2cm** | <br> 1 <br> 1.46 | <br> <br> 1.04 | <br> <br> 2.05 | **0·029*** |
| **Progesterone receptor (positive)** | 0·63 | 0·43 | 0·91 | **0·013*** |
| **Histologic grade** | | | | **0·005*** |
| Low <br> Intermediate <br> High | 1·00 <br> 2·59 <br> 3·18 | <br> 1·34 <br> 1·59 | <br> 5·01 <br> 6·36 | |
| **Size** | 1·01 | 1.007 | 1·015 | **<0·0001 *** |
| **Endocrine and Chemotherapies** <br> No systemic therapy <br> Endocrine therapy alone <br> Endocrine therapy + Chemotherapy | <br> 1·00 <br> 0·57 <br> 0·29 | <br> 0·27 <br> 0·13 | <br> 1·18 <br> 0·66 | **0·004*** |
| **Anthracycline Chemotherapy***SPAG5* | 0·33 | 0·17 | 0·67 | **0·002*** |
| **B. *SPAG5* vs NPI** | | | | |
| *SPAG5* mRNA (high +) | 4·72 | 1.97 | 11·28 | **<0·0001*** |
| **NPI (continuous)** | 1·78 | 0·71 | 4·45 | 0·22 |
| **C. *SPAG5* vs 76-gene prognostic signature (Veridex)** | | | | |
| **76-gene prognostic signature (Veridex)[13]** | 0·66 | 0·.5 | 1·24 | 0·20 |
| *SPAG5* mRNA (high +) | 4·02 | 1·63 | 9·90 | **0·002*** |
| **D. *SPAG5* vs PAM-50 Molecular classes** | | | | |
| **PAM-50 Molecular subclasses[28]** <br> PAM50-LumA <br> PAM50-LumB <br> PAM50-HER2 <br> PAM50-Basal-like <br> PAM50-Normal-like | <br> 1 <br> 1·58 <br> 1-41 <br> 0·86 <br> 1·18 | <br> <br> 0·91 <br> 0·85 <br> 0·62 <br> 0·85 | <br> <br> 2-72 <br> 2·34 <br> 1·18 <br> 1·64 | 0·085 |
| *PAG5* mRNA (high +) | | | | |
| 1·85 | 1·26 | 2·70 | **0·002*** | |
| *Statistically significant at p<0·05. | | | | |

Example 37 - SPAG5 transcript expression and clinical response to endocrine therapy neoadjuvant therapy

The expression level of SPAG5 mRNA in a tumour biopsy that has been taken from BC patients with an ER+ tumour after 2 weeks of receiving pre-operative endocrine therapy was significantly downregulated in 72% (68/92) of cases compared to the expression level in the biopsy that has been taken before the starting of the treatment I ($p < 0 \cdot 00001$, Wilcoxon-test).

There was a significant further reduction in the level of SPAG5 mRNA expression in the tumour biopsy that has been taken from patients after 12 weeks of receiving pre-operative endocrine therapy compared to the expression level in the biopsy that has been taken after 2 weeks of receiving pre-operative endocrine therapy ($p = 0 \cdot 023$, Wilcoxon).

In 76% of tumours that have shown reduction in the tumour volume by 50% after receiving preoperative endocrine therapy for 2 weeks (i.e., responding tumours), a significant down-regulation of SPAG5 mRNA expression occurred in the tumour biopsy that has been taken from patients after 2 weeks of receiving pre-operative endocrine therapy ($p < 0 \cdot 00001$, Wilcoxon-test).

In tumours that have not shown reduction in the tumour volume by 50% after receiving preoperative endocrine therapy for either 2 or 12 weeks (i.e. non-responding tumours), there was no significant change in SPAG5 mRNA expression level in the tumour biopsy that has been taken from patients either after 2 or 12 weeks of receiving pre-operative endocrine therapy compared to the expression level in the biopsy that has been taken before the starting of the treatment ($p = 0.265$ and $0.563$; respectively, Wilcoxon-test).

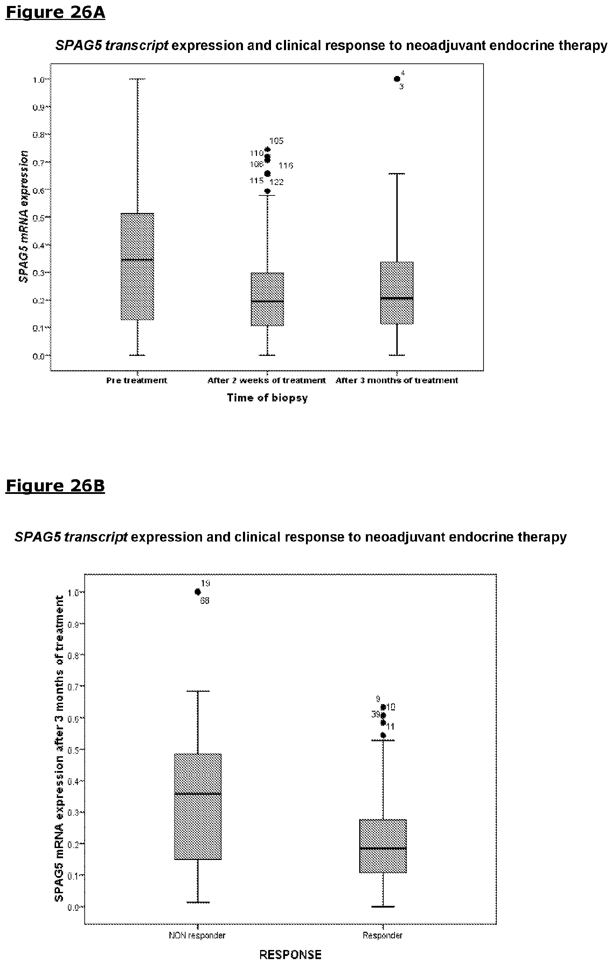

After 12 weeks of receiving pre-operative endocrine therapy, tumours that did not show a reduction in the tumour volume by 50% (i.e., non-responding tumours) expressed a higher level of SPAG5 mRNA level compared to those that showed reduction in the tumour volume by 50% (i.e., responding tumours) ($p = 0.014$, Mann-Whitney); Figures 26A and 26B.

Example 38 - Pathological complete response (pCR) to pre-operative anthracycline-based chemotherapy (AC) in ER+ locally advanced breast cancer (SPAG5 transcript analysis)

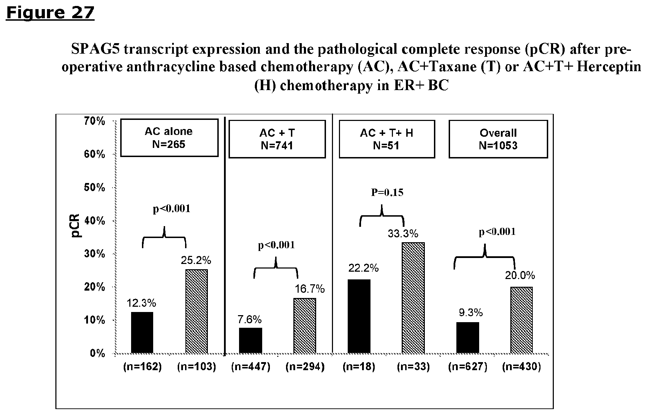

In ER+ locally advanced breast cancer, compared to low SPAG5 mRNA expression in the tumour biopsy that had been taken from patients at the time of the diagnosis and before receiving any treatment, SPAG5 mRNA overexpression predicted 2-fold higher rate of complete disappearance of the cancer cells in the breast and lymph node (i.e., pathological complete response) in the surgically removed specimen after receiving preoperative anthracycline based combination chemotherapy alone (25% vs 12%)); $p < 0.0001$; see Figure 27.

Example 39 - Pathological complete response (pCR) to pre-operative AC + taxane (T) chemotherapy in ER+ locally advanced breast cancer (SPAG5 transcript analysis)

In ER+ locally advanced breast cancer, compared to low SPAG5 mRNA expression in the tumour biopsy that had been taken from patients at the time of the diagnosis and before receiving any treatment, SPAG5 mRNA overexpression predicted 2-fold higher rate of complete disappearance of the cancer cells in the breast and lymph node (i.e., pathological complete response) in the surgically removed specimen after receiving preoperative taxane in addition to anthracycline based combination chemotherapy (17% vs 8%); $p < 0.0001$.

Example 40 - Pathological complete response (pCR) to pre-operative AC + taxane (T) chemotherapy in ER+HER2- locally advanced breast cancer (SPAG5 transcript analysis)

In ER+HER2- locally advanced breast cancer, compared to low SPAG5 mRNA expression in the tumour biopsy that had been taken from patients at the time of the diagnosis and before receiving any treatment, SPAG5 mRNA overexpression predicted 2.5 fold higher rate of complete disappearance of the cancer cells in the breast and lymph node (i.e., pathological complete response) in the surgically removed specimen after receiving preoperative taxane in addition to anthracycline based combination chemotherapy [17% (57/328) vs., 8% (43/552); OR (CI 95%): 2.49 (1.63-3.80); $p = 0 \cdot 00001$].

Example 41 - Pathological complete response (pCR) to pre-operative Trastuzumab, AC and taxane (T) chemotherapy in ER+HER2+ locally advanced breast cancer (SPAG5 transcript analysis)

In ER+HER2+ locally advanced breast cancer, compared to low SPAG5 mRNA expression in the tumour biopsy that had been taken from patients at the time of the diagnosis and before receiving any treatment, SPAG5 mRNA overexpression predicted a trend of higher rate of complete disappearance of the cancer cells in the breast and lymph node (i.e., pathological complete response) in the surgically removed specimen after receiving preoperative Trastuzumab and taxane in addition to anthracycline based combination chemotherapy (33% vs 22%; p=0.15).

Example 42 - Pathological complete response (pCR) to pre-operative AC with or without taxane (T) chemotherapy in ER+ locally advanced breast cancer (SPAG5 transcript analysis)

Overall, in ER+ locally advanced breast cancer, compared to low SPAG5 mRNA expression in the tumour biopsy that had been taken from patients at the time of the diagnosis and before receiving any treatment, SPAG5 mRNA overexpression predicted 2.5-fold higher rate of complete disappearance of the cancer cells in the breast and lymph node (i.e., pathological complete response) in the surgically removed specimen after receiving preoperative anthracycline based combination chemotherapy with or without taxane (20% (86/430) vs., 9% (58/627); odd ratio (OR) (CI 95%) = 2.5 (1.71-3.51); p<0.0001).

Example 43 - SPAG5 transcript expression in early-stage breast cancer (Clinical outcome measurements & interaction analysis with systemic therapy and other prognostic factors)

The interaction analysis of SPAG5 mRNA with systemic therapy and other validated prognostic factors (using multivariate logistic regression model) confirmed that SPAG5 mRNA overexpression in the tumour biopsy that had been taken from patients at the time of the diagnosis and before receiving any treatment, predicted 2-fold higher rate of complete disappearance of the cancer cells in the breast and lymph node (i.e., pathological complete response) in the surgically removed specimen after receiving anthracycline based combination preoperative chemotherapy regardless HER2 status or receiving additional taxane or Trastuzumab (OR (CI 95%): 1.92 (1.01-3.64); p=0·047; Table 4A; MC-Neo-Adj-CT cohort).

**Table 4:** Multivariable logistic regression models analysis for pathological complete response (pCR) after neo-adjuvant chemotherapy

**Table 4:** Multivariable logistic regression models analysis for pathological complete response (pCR) after neo-adjuvant chemotherapy

| A. Multivariable logistic regression models analysis for pathological complete response (pCR) in Multicentre neoadjuvant chemotherapy (MC-NACT) cohort (n=1073) | | | | |
|---|---|---|---|---|
| **Variables** | **OR** | **95.0% CI** | | **P value** |
| | | **Lower** | **Upper** | |
| *SPAG5* **mRNA expression (high)** | 1·92 | 1·01 | 3·64 | **0·047***  |
| **HER2 expression (overexpression)** | 3·03 | 1·34 | 6·88 | **0·008** |
| **Histological Grade (G1/2 vs G3)** | 2·33 | 1·25 | 4·34 | **0·008** |
| **PAM-50 Molecular subclasses**[28] PAM50-LumA PAM50-LumB PAM50-HER2 PAM50-Basal-like PAM50-Normal-like | 1 0·24 0·97 0·72 0·32 | 0·07 0·23 0·15 0·10 | 0·82 4·03 3·40 1·09 | **0·014*** |
| **Trastuzumab NACT (Yes)** | 0·66 | 0·20 | 2·16 | **0·49** |
| B. Multivariable logistic regression models analysis for pathological complete response (pCR) in the ER+NUH-LABC neo-adjuvant cohort | | | | |
| **SPAG5 protein (high)** | 23·03 | 7·26 | 73·02 | **<0·001*** |
| **Histological grade G1/G2 vs G3** | 2·37 | 1·15 | 4·89 | **0·020*** |
| **Age of patients (continuous)** | 0·99 | 0·96 | 1·01 | 0·26 |
| **HER2 (+)** | 5·83 | 1·97 | 17·22 | 0·001 |
| **Progesterone receptor (positive)** | 0·23 | 0·88 | 0·69 | 0·009 |

(continued)

| B. Multivariable logistic regression models analysis for pathological complete response (pCR) in the ER+NUH-LABC neo-adjuvant cohort | | | | |
|---|---|---|---|---|
| Taxane neoadjuvant (Yes) | 1·52 | 0-40 | 5·85 | 0·54 |
| Age (years) | 1·04 | 0·96 | 1·05 | 0·89 |

Example 44 - SPAG5 mRNA expression in ovarian cancer

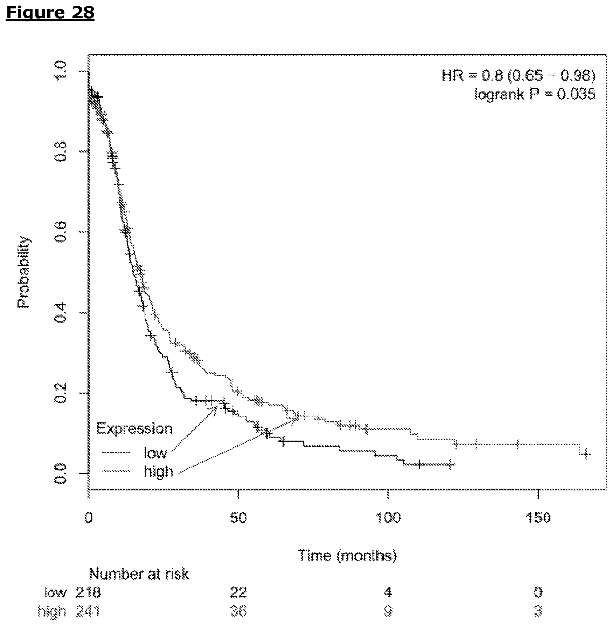

In ovarian cancer, SPAG5 mRNA overexpression in ovarian cancer tissue at the time of surgery was associated with 20% reduction in the risk of relapse after receiving cisplatinum and taxane at 5 years of follow up after surgically removing the primary ovarian cancer compared to low SPAG5 mRNA expression (HR (95% CI) = 0.80 (0.65-0.98); p=0.035 (see Figure 28).

Example 45 - Granular SPAG5 protein expression in ovarian cancer

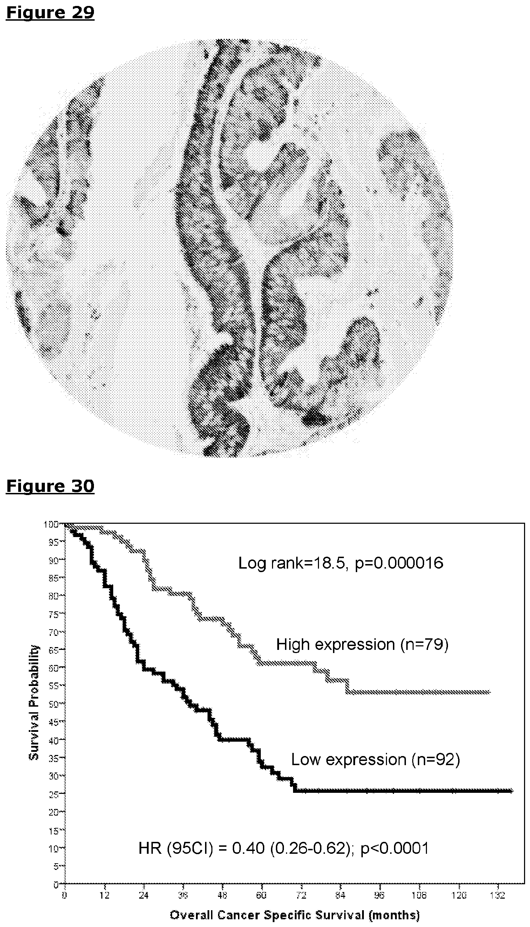

Granular SPAG5 protein is expressed in ovarian (see Figure 29).

Example 46 - Cisplatinum and taxane treatment of ovarian cancer that expresses granular SPAG5 protein (Clinical outcome measurement: risk of death)

In ovarian cancer, SPAG5 protein overexpression in ovarian cancer tissue at the time of surgery was associated with 60% reduction in the risk of death after receiving cisplatinum and taxane at 10 years of follow up after surgically removing the primary ovarian cancer compared to low SPAG5 protein expression (HR (95% CI) = 0.40 (0.26-0.62); p<0.0001 (see Figure 30).

Example 47 - Cisplatinum and taxane treatment of ovarian cancer that expresses granular SPAG5 protein (Clinical outcome measurement: risk of relapse)

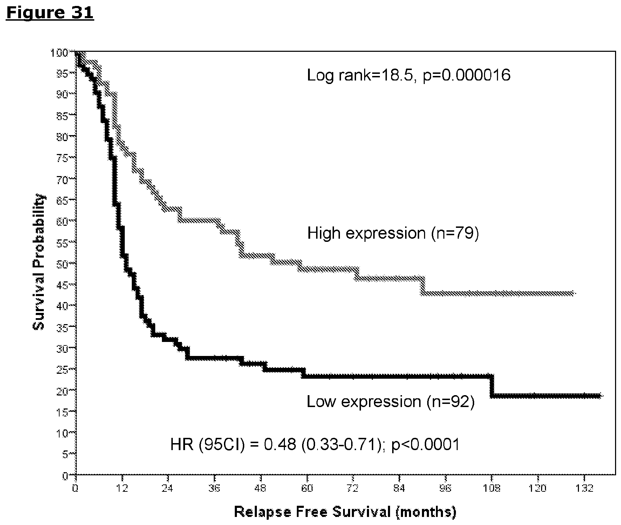

In ovarian cancer, SPAG5 protein overexpression in ovarian cancer tissue at the time of surgery was associated with 52% reduction in the risk of relapse after receiving cisplatinum and taxane at 10 years of follow up after surgically removing the primary ovarian cancer compared to low SPAG5 protein expression (HR (95% CI) = 0.48 (0.33-0.71); p<0.0001 (see Figure 31).

Example 48 - Cisplatinum treatment of gastric cancer that overexpresses SPAG5 mRNA (Clinical outcome measurement: risk of relapse)

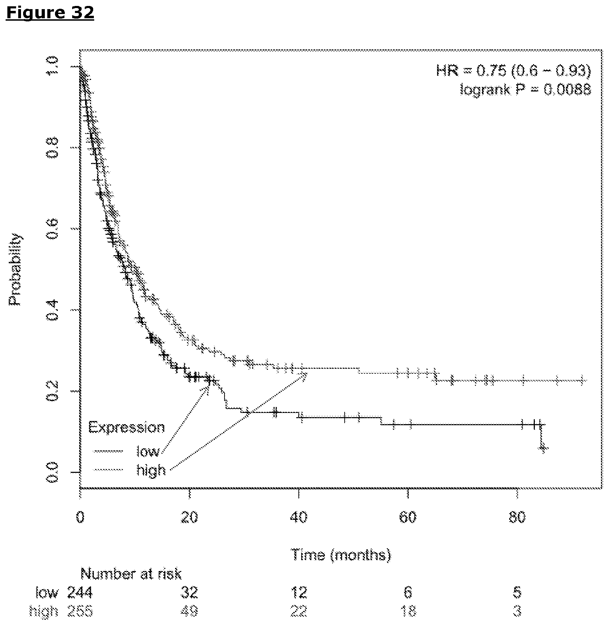

In gastric cancer, SPAG5 mRNA in gastric cancer tissue at the time of surgery was associated with 25% reduction in the risk of relapse after receiving preoperative cisplatinum followed by surgery compared to low SPAG5 mRNA expression at 5 years of follow up after surgically removing the primary gastric cancer, (HR (95% CI = 0.75 (0.60-0.93); p=0.0088) (n = 499) (see Figure 32).

Example 49- Treatment of gastric cancer that overexpresses SPAG5 mRNA with 5-Flu adjuvant therapy (Clinical outcome measurement: risk of relapse)

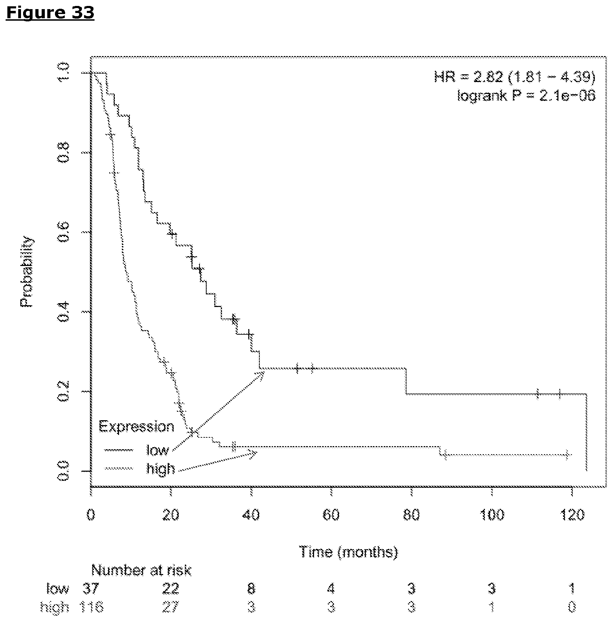

In gastric cancer, SPAG5 mRNA overexpression in gastric cancer tissue at the time of surgery was associated with three times higher risk of relapse after receiving 5-flu adjuvant therapy at 5 years of follow up after surgically removing the primary gastric cancer compared to low SPAG5 mRNA expression (HR (95% CI) = 2.82 (1.81-4.39); p<0.0001) (n = 499) (see Figure 33).

Example 50 - Granular SPAG5 protein expression in gastric cancer

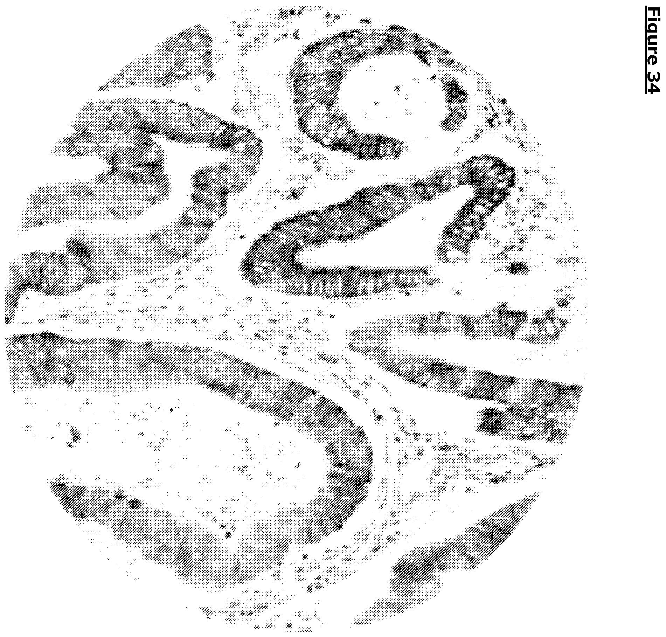

Granular SPAG5 protein is expressed in gastric cancer (see Figure 34; n = 140).

Example 51 - Cisplatinum treatment of gastric cancer that expresses granular SPAG5 protein (Clinical outcome measurement: risk of relapse)

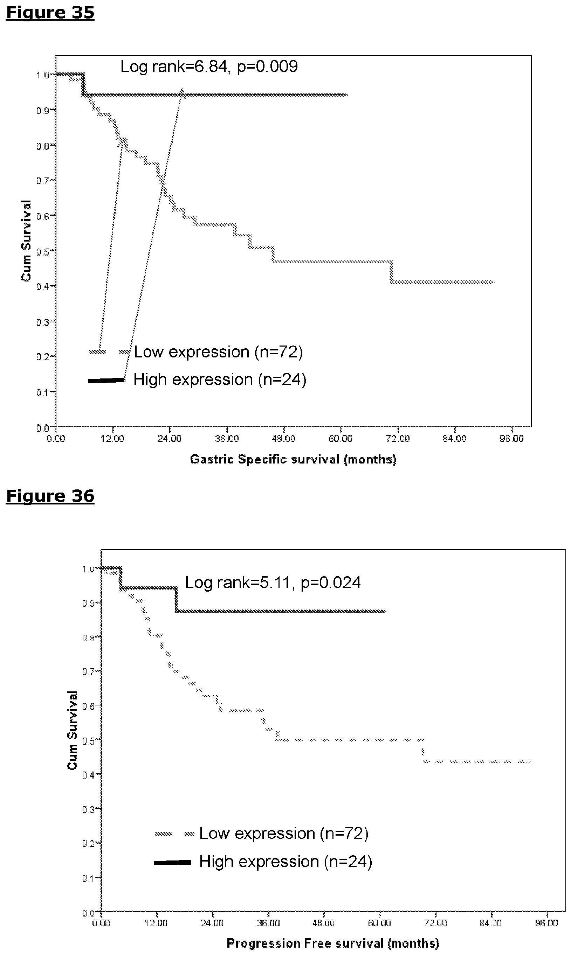

In gastric cancer, SPAG5 protein overexpression in gastric cancer tissue at the time of surgery was associated with 40% reduction in the risk of death (see Figure 35) and relapse (see Figure 36) after receiving preoperative cisplatinum followed by surgery at 5 years of follow up after surgically removing the primary gastric cancer compared to low SPAG5 protein expression.

Example 52 - SPAG5 mRNA overexpression in lung cancer (Clinical outcome measurement: risk of death)

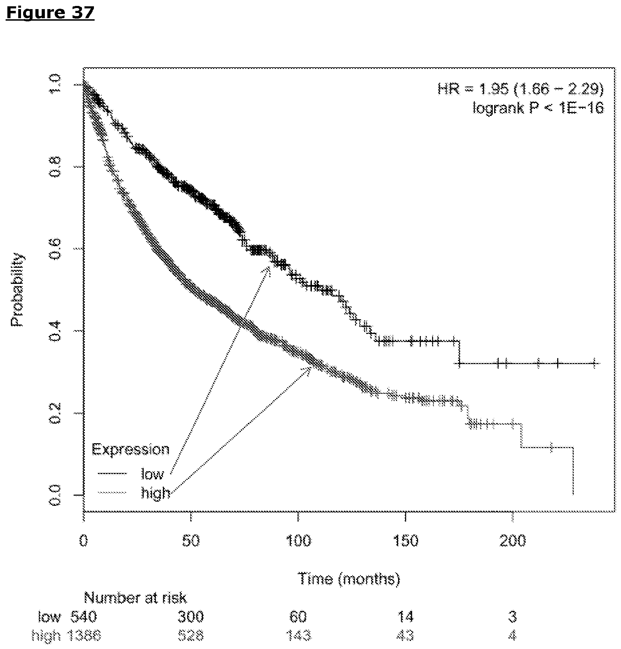

In lung cancer, SPAG5 mRNA overexpression in lung cancer tissue at the time of surgery was associated with 2-fold increase in the risk of death at 10 years of follow up after surgically removing the primary lung cancer compared to low SPAG5 mRNA expression (HR (95% CI): 1.95 (1.66-2.29), p<0.0001; n = 1926, expression level of SPAG5 probe = 26 - 5014, median cut-off expression level= 254) (see Figure 37).

Example 53 - SPAG5 mRNA overexpression in lung cancer (Clinical outcome measurement: risk of relapse)

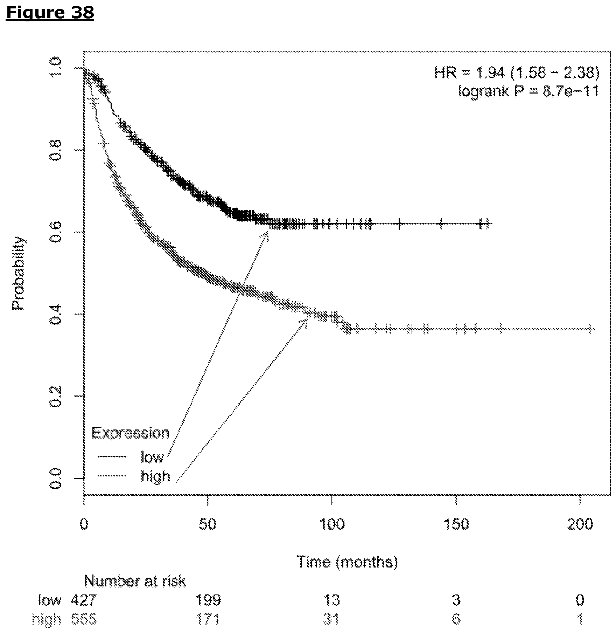

In lung cancer, SPAG5 mRNA overexpression in lung cancer tissue at the time of surgery was associated with 2-fold increase in the risk of relapse at 10 years of follow up after surgically removing the primary lung cancer compared to low SPAG5 mRNA expression (HR (95% CI): 1.94 (1.58-2.38), p<0.0001; n = 1926, expression level of SPAG5 probe = 26 - 5014, median cut-off expression level= 254) (see Figure 38).

Example 54 - Granular SPAG5 protein expression in lung cancer

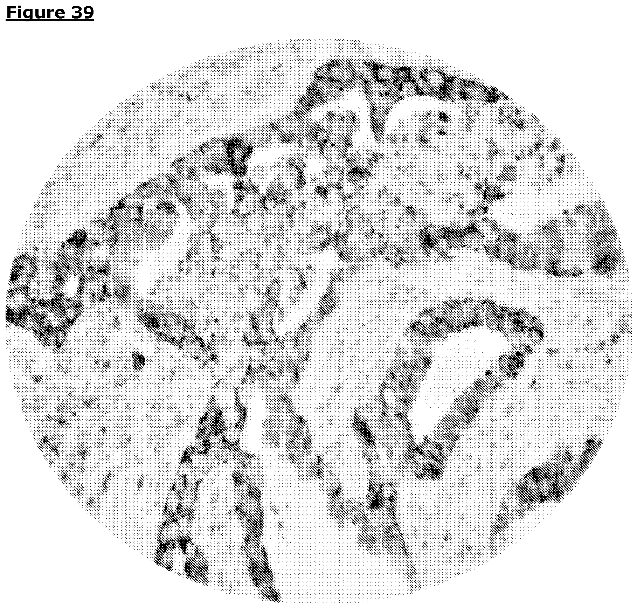

Granular SPAG5 protein is expressed in lung cancer (see Figure 39; n = 215).

Example 55 - Granular SPAG5 protein expression in lung cancer (Clinical outcome measurement: risk of death)

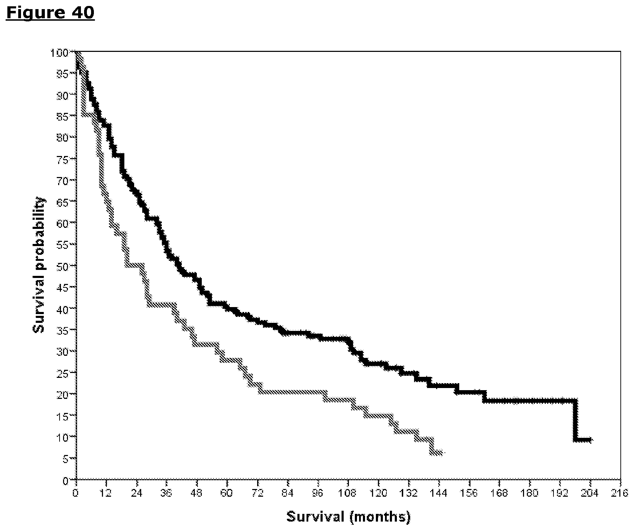

In lung cancer, SPAG5 protein overexpression in lung cancer tissue at the time of surgery was associated with higher risk of death at 10 years of follow up after surgically removing the primary lung cancer compared to low SPAG5 protein expression (HR (95% CI): 1.17 (1.04-1.30), p=0.007) (see Figure 40).

Example 56 - SPAG5 mRNA overexpression in liver cancer (Clinical outcome measurement: risk of relapse)

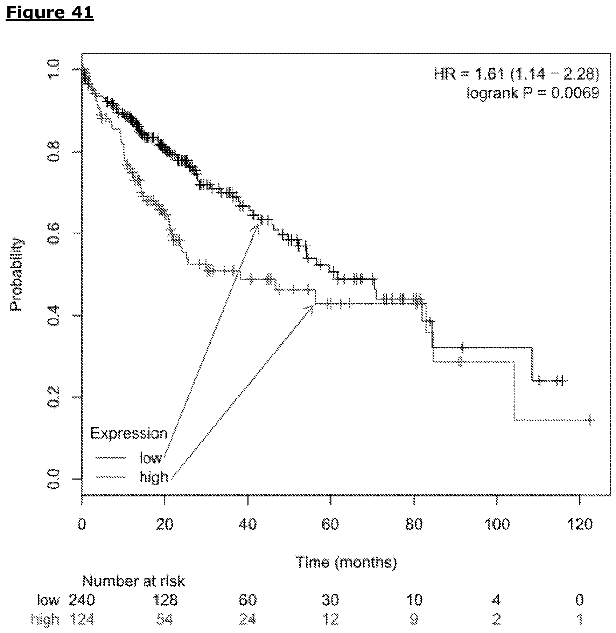

In liver cancer, SPAG5 mRNA overexpression in liver cancer tissue at the time of surgery was associated with 2-fold increase in the risk of relapse at 5 years of follow up after surgically removing the primary liver cancer compared to low SPAG5 mRNA expression (HR (95% CI): 1.61(1.14-2.28), p=0.0069; n=364) (see Figure 41).

Example 57 - Cisplatinum and taxane treatment of colon cancer that expresses granular SPAG5 protein (Clinical outcome measurement: risk of death)

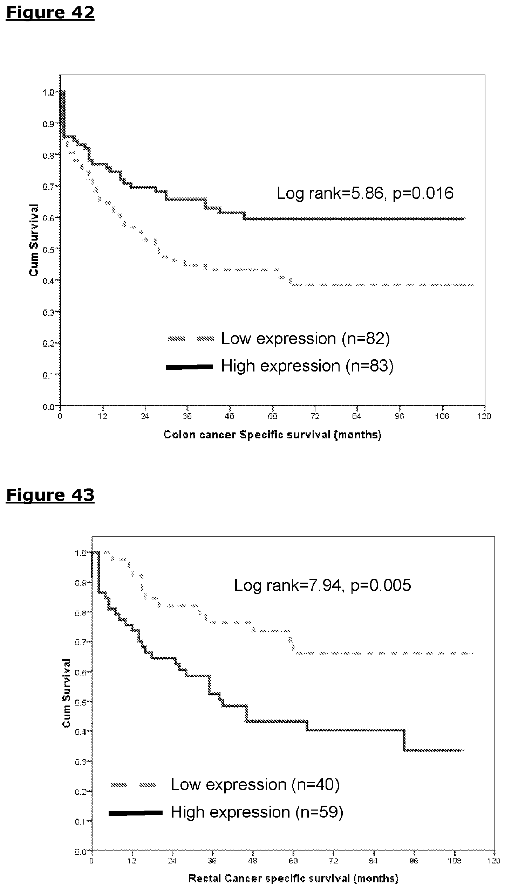

In colon cancer, SPAG5 protein overexpression in colon cancer tissue at the time of surgery was associated with 20% reduction in the risk of death after receiving taxane and cisplatin at 5 years of follow up after surgically removing the primary colon cancer compared to low SPAG5 protein expression (p=0.016) (see Figure 42).

Example 58 - Granular SPAG5 protein expression in rectal cancer (Clinical outcome measurement: risk of death)

In rectal cancer, SPAG5 protein overexpression in rectal cancer tissue at the time of surgery was associated with 2-fold increase in the risk of death after receiving radiotherapy at 5 years of follow up after surgically removing the primary rectal cancer compared to low SPAG5 protein expression (p=0.005) (see Figure 43).

Example 59 - Granular SPAG5 protein expression in early stage HER2+ breast cancer that has not been treated with chemotherapy or HER2 target therapy (Clinical outcome measurement: risk of distant relapse)

In HER2+ early stage BC patients (i.e. patients that have a breast cancer that express the human epidermal growth factor receptor 2 (HER+2) who did not receive any systemic chemotherapy and HER2 target therapy, SPAG5 protein overexpression in the cancerous tissue at the time of surgery was associated with increase of distant relapse risk at 5 years follow up compared to low SPAG5 protein expression (i.e. no staining or faint or diffuse staining (no intracytoplasmic punctate expression of SPAG5 protein) (see Figure 44).

Example 60 - Granular SPAG5 protein expression in early stage HER2+ breast cancer that has been treated with anthracycline based chemotherapy but no Trastuzumab (Clinical outcome measurement: risk of distant relapse)

In HER2+ early stage BC patients (i.e. patients that have a breast cancer that express the human epidermal growth factor receptor 2 (HER+2) who received anthracycline based chemotherapy but no Trastuzumab, SPAG5 protein overexpression in the cancerous tissue at the time of surgery was associated with increase of distant relapse risk at 5 years follow up compared to low SPAG5 protein expression (i.e., no staining or faint or diffuse staining (no intracytoplasmic punctate expression of SPAG5 protein) (see Figure 45).

Example 61 - Granular SPAG5 protein expression in early stage HER2+ breast cancer that has been treated with anthracycline based chemotherapy plus Trastuzumab (Clinical outcome measurement: risk of distant relapse)

In HER2+ early stage BC patients (i.e., patients that have a breast cancer that express the human epidermal growth factor receptor 2 (HER+2) who received anthracycline based chemotherapy plus Trastuzumab, SPAG5 protein overexpression in the cancerous tissue at the time of surgery was associated with reduction of distant relapse risk at 5 years follow up compared to low SPAG5 protein expression (i.e., no staining or faint or diffuse staining (no intracytoplasmic punctate expression of SPAG5 protein) (see Figure 46).

Example 62 - Pathological complete response (pCR) of locally advanced HER+ breast cancer (with increased granular SPAG5 protein expression at time of surgery) to preoperative treatment with anthracycline based chemotherapy with or without Taxane and with or without Trastuzumab therapy (SPAG5 protein analysis)

In HER2 positive locally advanced breast cancer with low SPAG5 protein expression (no intracytoplasmic punctate expression of SPAG5 protein) in the tumour biopsy that had been taken from patients at the time of the diagnosis and before receiving any treatment, receiving anthracycline based chemotherapy plus Taxane plus HER2 targeting Neo-adjuvant therapy was associated with higher pCR compared to anthracycline based chemotherapy with or without taxane (21% vs., 4%; OR (95% CI): 6.6 (1.4-32.6), p=0.01). Whereas receiving anthracycline-based chemotherapy with or without taxane was associated with similar pCR to anthracycline based chemotherapy plus Taxane plus HER2 targeting Neo-adjuvant therapy in patients with HER2+ SPAG5+ protein (49% vs., 53%; OR (95% CI): 1.2 (0.5-3.2), p=0.702). Receiving anthracycline-based chemotherapy plus Taxane plus HER2 targeting Neo-adjuvant therapy was associated with lower DRR compared to anthracycline based chemotherapy plus Taxane Neo-adjuvant therapy [HR (95% CI): 0.82 (0.08-0.97); p=0.045] in patients with SPAG5- protein expression but not in those with SPAG5+ protein [HR (95% CI): 1.08 (0.34-3.36); p=0.901].

Example 63 - SPAG5 transcript expression in early stage HER2+ breast cancer that has not been treated with chemotherapy or HER2 target therapy (Clinical outcome measurement: risk of distant relapse)

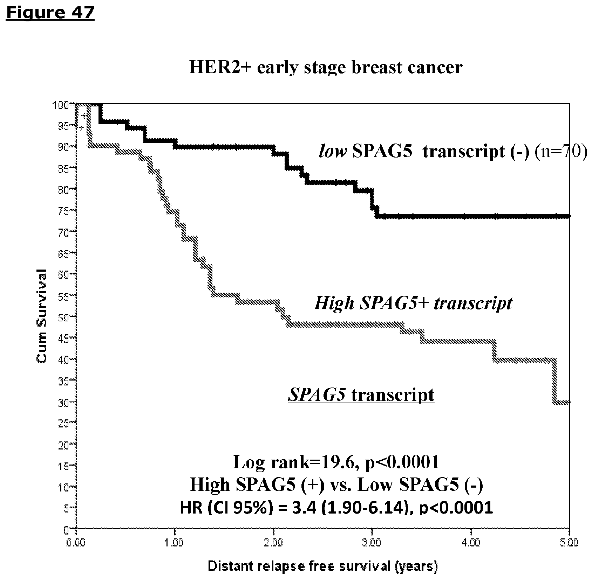

In HER2+ early-stage BC patients (i.e., patients that have a breast cancer that express the human epidermal growth factor receptor 2 (HER+2) who did not receive any systemic chemotherapy and HER2 target therapy, SPAG5 transcript overexpression in the cancerous tissue at the time of surgery was associated with increase of distant relapse risk at 5 years follow up compared to low SPAG5 transcript expression (see Figure 47).

Example 64 - SPAG5 transcript expression in early stage HER2+ breast cancer that has been treated with anthracycline based chemotherapy but no Trastuzumab (Clinical outcome measurement: risk of distant relapse)

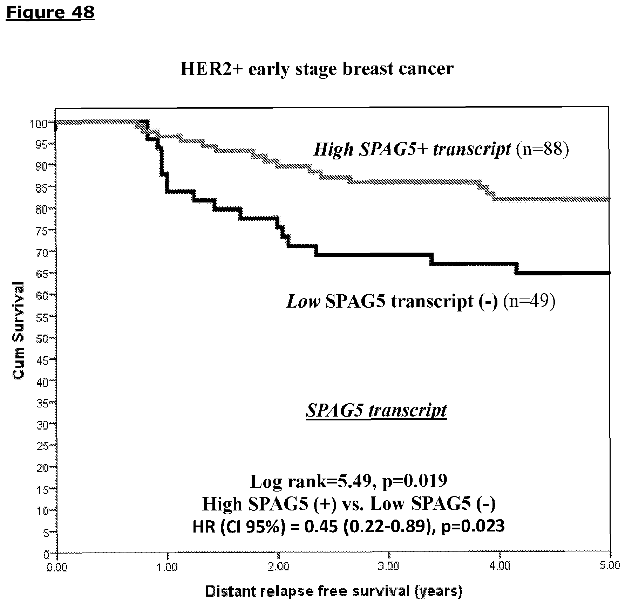

In HER2+ early-stage BC patients (i.e., patients that have a breast cancer that express the human epidermal growth factor receptor 2 (HER+2) who treated with anthracycline based chemotherapy but no Trastuzumab, SPAG5 transcript overexpression in the cancerous tissue at the time of surgery was associated with reduced distant relapse risk at 5 years follow up compared to low SPAG5 transcript expression (see Figure 48).

Example 65 - SPAG5 transcript expression in early stage HER2+ breast cancer that has been treated with anthracycline based chemotherapy plus Trastuzumab (Clinical outcome measurement: risk of distant relapse)

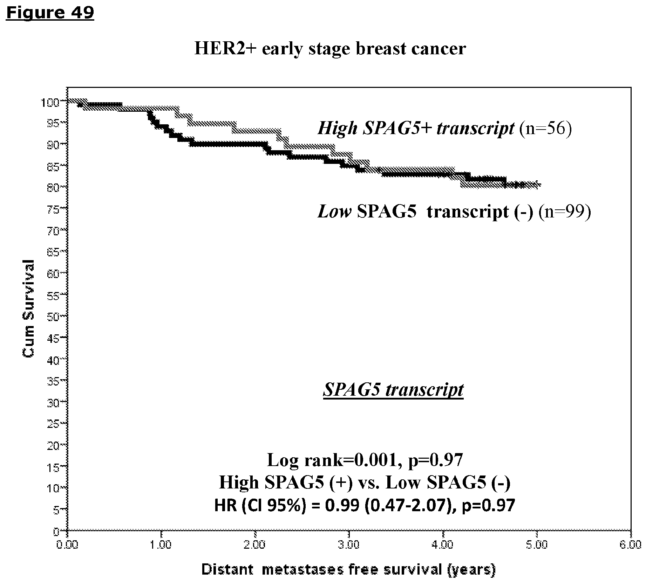

In HER2+ early-stage BC patients (i.e., patients that have a breast cancer that express the human epidermal growth factor receptor 2 (HER+2) who treated with anthracycline based chemotherapy plus Trastuzumab, SPAG5 transcript overexpression in the cancerous tissue at the time of surgery was associated with similar distant relapse risk at 5 years follow up compared to low SPAG5 transcript expression (see Figure 49).

Example 66- Pathological complete response (pCR) of locally advanced HER+ breast cancer (with increased SPAG5 transcript expression at time of surgery) to preoperative treatment with anthracycline based chemotherapy with or without Taxane and with or without Trastuxumab therapy (SPAG5 transcript analysis)

In HER2 positive locally advanced breast cancer, patients with SPAG5 mRNA overexpression (+;> median), those who had received anthracycline based chemotherapy with or without taxane Neo-adjuvant therapy alone achieved similar pCR to those who had received anthracycline based chemotherapy plus Taxane plus HER2 targeting Neo-adjuvant therapy (38% vs., 37%; OR (95% CI): 1.0 (0.6-1.6), p=0.923) in either ER- (46% vs., 52%; OR (95% CI): 1.3 (0.5-3.0), p=0.58) or ER+ subgroups (25% vs., 26%; OR (95% CI): 1.1 (0.4-3.4), p=0.88). Whereas in patients with low SPAG5 mRNA (-), those who had received anthracycline based chemotherapy plus Taxane plus HER2 targeting Neo-adjuvant therapy had achieved 2.5 fold increase in pCR compared to those who received anthracycline based chemotherapy with or without taxane alone (47% vs., 26%; OR (95% CI): 2.5 (1.4-4.4), p=0.001) in either ER- (60% vs., 31%; OR (95% CI): 3.4 (1.7-6.7), p<0.001) or ER+ subgroups (42% vs., 16%; OR (95% CI): 4.0 (1.2-12.9), p=0.018).

Example 67 - HER2 expression in paired post neoadjuvant chemotherapy samples of locally advanced pre systemic therapy HER2 negative breast cancer that (n= 361; Clinical outcome measurement: risk of relapse)

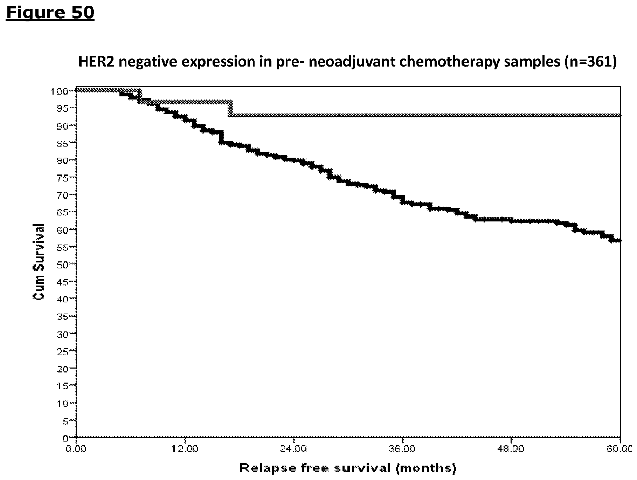

Eight percent (8%; 30/361) of pre neoadjuvant chemotherapy HER2- samples had been converted into HER2+ in post neoadjuvant chemotherapy samples had achieved 92% 5-year relapse free survival compared to those who remained HER-in post neoadjuvant chemotherapy (58% 5-year DFS); (HR (95% CI) = 0.18 (0.05-0.74); p=0.017). Noteworthy 19 out of 30 HER2 converted cases (63%) have subsequently received adjuvant Trastuzumab while 11 out of the 30 converted cases did not receive either adjuvant chemotherapy or HER2 target therapy (see Figure 50).

Example 68 - HER2 positive expression cases in the paired post neoadiuvant chemotherapy samples of locally advanced pre systemic therapy HER2 negative breast cancer that (n= 30; Clinical outcome measurement: risk of relapse)

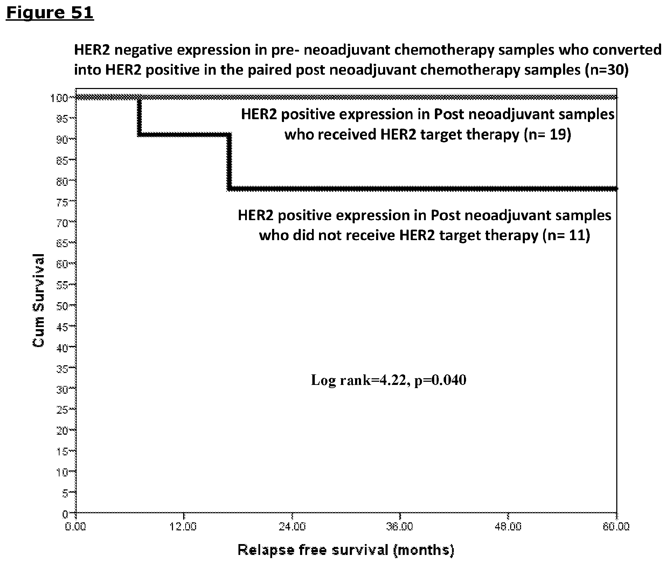

Eight percent (8%; 30/361) of pre neoadjuvant chemotherapy HER2- samples had been converted into HER2+ in post neoadjuvant chemotherapy samples. The 19 out of 30 HER2 converted cases who have subsequently received adjuvant Trastuzumab had achieved 100 % 5-year relapse free survival compared to those who did not receive either adjuvant chemotherapy or HER2 target therapy (58% 5-year DFS); p=0.040) (see Figure 50) while 11 out of the 30 converted cases (see Figure 51).

Example 69 - Granular SPAG5 protein expression in the post neoadiuvant chemotherapy of the pre systemic therapy HER2- breast cancer samples that has remained HER2- in the paired post neoadiuvant chemotherapy samples (n=343; Clinical outcome measurement: risk of relapse)

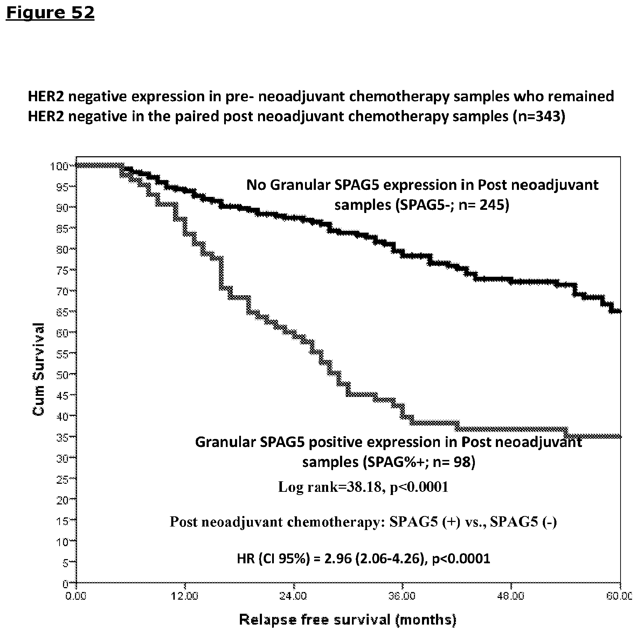

In pre systemic therapy chemotherapy locally advanced BC patients that were HER2- (i.e. patients that have a breast cancer that doesn't express the human epidermal growth factor receptor 2 (HER+2) in both pre and post neoadjuvant chemotherapy samples, the presence of granular SPAG5 protein expression was reported in 26% of the post neoadjuvant chemotherapy residual cancerous tissue and was associated with 3 fold in increase in the risk of relapse at 5 years follow up compared to the absence of granular SPAG5 protein expression (65% vs., 35% 5-year DFS); (HR (95% CI)= 2.96 (2.06-4.26); p<0.0001) (see Figure 52).

Example 70 - Granular SPAG5 protein expression in the post neoadiuvant chemotherapy of the pre systemic therapy HER2-/ER- breast cancer samples that has remained HER2- in the paired post neoadjuvant chemotherapy samples (n=123; Clinical outcome measurement: risk of relapse)

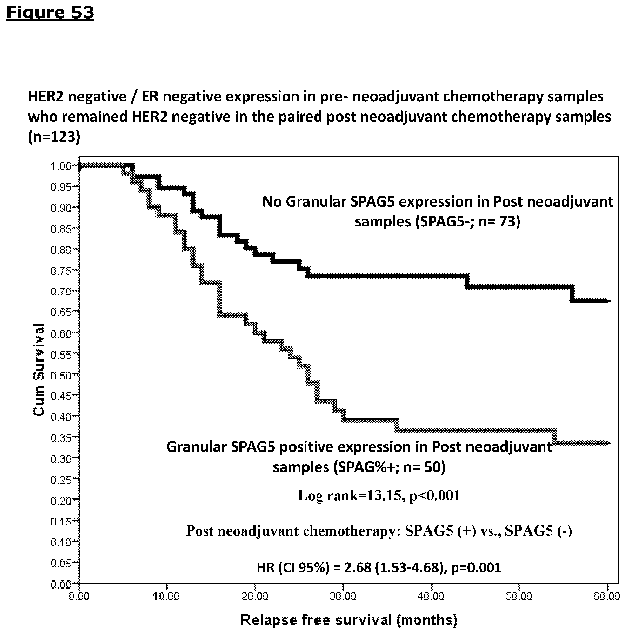

In pre systemic therapy chemotherapy locally advanced BC patients that were HER2-/ER- (i.e. patients that have a breast cancer that doesn't express the human epidermal growth factor receptor 2 (HER+2) and oestrogen receptor) in the pre neoadjuvant chemotherapy samples and remained HER2- in the post chemotherapy samples, the presence of granular SPAG5 protein expression was reported in 41% of the post neoadjuvant chemotherapy residual cancerous tissue samples and was associated with 3 fold in increase in the risk of relapse at 5 years follow up compared to the absence of granular SPAG5 protein expression (67% vs., 34% 5-year DFS); (HR (95% CI)= 2.68 (1.53-4.68); p=0.001) (see Figure 53).

Example 71 - Granular SPAG5 protein expression in the post neoadjuvant chemotherapy of the pre systemic therapy HER2-/ER- breast cancer samples that has remained HER2- in the paired post neoadjuvant chemotherapy samples (n=220; Clinical outcome measurement: risk of relapse)

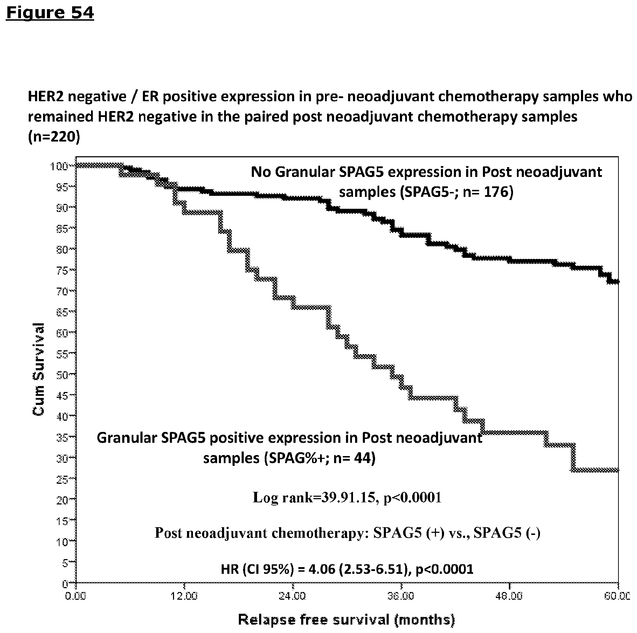

In pre systemic therapy chemotherapy locally advanced BC patients that were HER2-/ER+ (i.e. patients that have a breast cancer that doesn't express the human epidermal growth factor receptor 2 (HER+2) but expresses oestrogen receptor) in the pre neoadjuvant chemotherapy samples and remained HER2- in the post chemotherapy samples, the presence of granular SPAG5 protein expression was reported in 25% of the post neoadjuvant chemotherapy residual cancerous tissue samples and was associated with four fold in increase in the risk of relapse at 5 years follow up compared to the absence of granular SPAG5 protein expression (72% vs., 32% 5-year DFS); (HR (95% CI)= 4.06 (2.53-6.51); p<0.001) (see Figure 54).

Example 72 - HER2 expression in paired post neoadjuvant chemotherapy samples of locally advanced pre systemic therapy HER2 positive breast cancer that (n= 92; Clinical outcome measurement: risk of relapse)

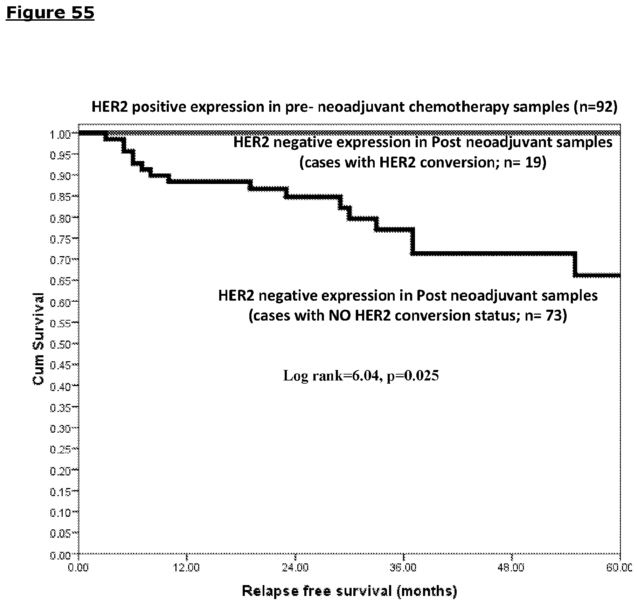

Twenty-one percent (21%; 19/92) of pre neoadjuvant chemotherapy HER2+ samples had been converted into HER2- in post neoadjuvant chemotherapy residual samples and had achieved 100% 5-year relapse free survival compared to those who remained HER+ in post neoadjuvant chemotherapy residual sample (66% 5-year DFS); p=0.025). Noteworthy 15 out of 19 HER2 converted cases (79%) have received HER2 target neoadjuvant therapy in addition to anthracycline based combination plus taxane chemotherapy followed by adjuvant Trastuzumab for one year (see Figure 55).

Example 73 - HER2 positive expression cases in the paired post neoadjuvant chemotherapy samples of locally advanced pre systemic therapy HER2 positive breast cancer that (n= 73; Clinical outcome measurement: risk of relapse)

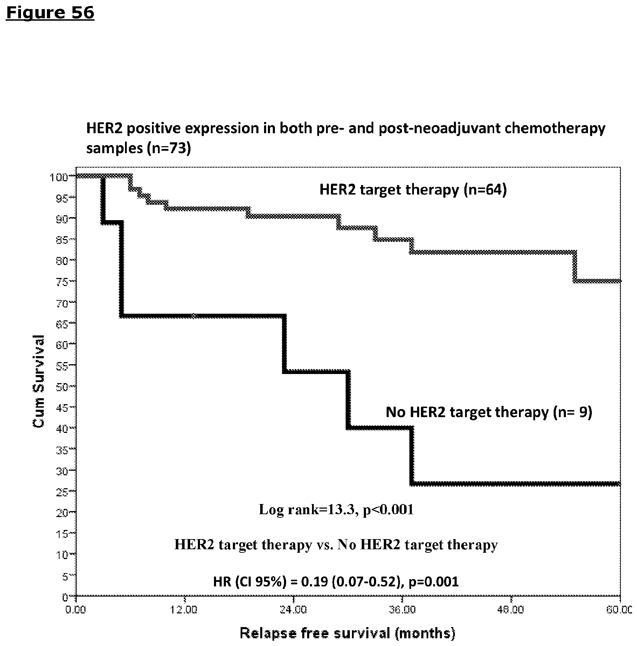

Seventy-nine percent (79%; 73/92) of pre neoadjuvant chemotherapy HER2+ samples had remained HER2+ in post neoadjuvant chemotherapy samples. The majority of these cases 88%; (64/73) have received adjuvant HER2 Target therapy after the neoadjuvant chemotherapy and achieved a better prognosis (75% 5-year DFS) compared to those who did not receive any HER2 Target therapy (n=9/73 (12%); 26% 5-year DFS; p<0.001 (see Figure 56).

Example 74 - Granular SPAG5 protein expression in the post neoadjuvant chemotherapy of the pre systemic therapy HER2+ breast cancer samples that has remained HER2+ in the paired post neoadjuvant chemotherapy samples and received HER2 target therapy for one year (n=64; Clinical outcome measurement: risk of relapse)

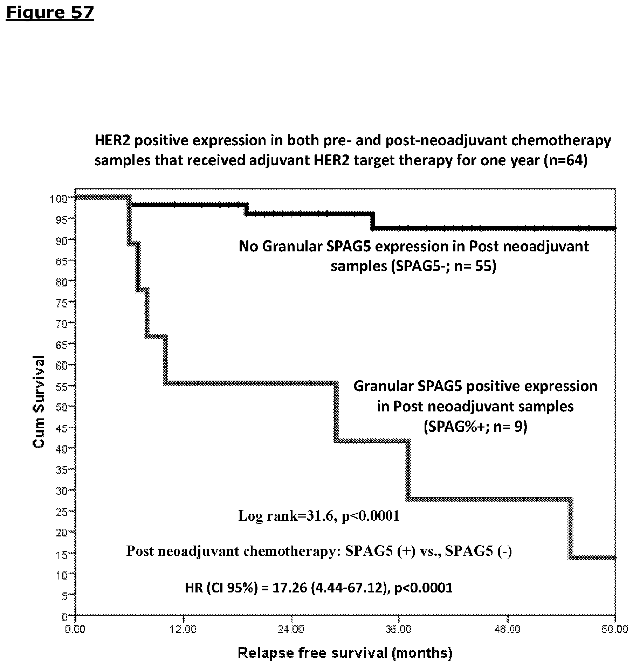

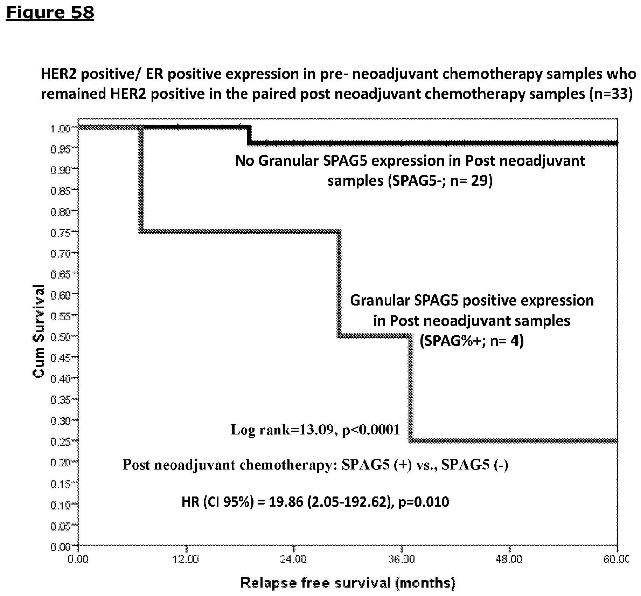

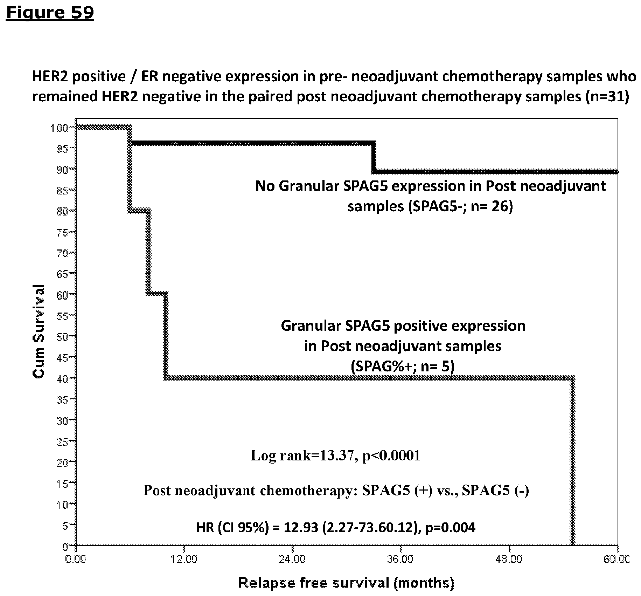

In pre systemic therapy chemotherapy locally advanced BC patients that were HER2+ (i.e., patients that have a breast cancer that express the human epidermal growth factor receptor 2 (HER+2) in both pre and post neoadjuvant chemotherapy samples, the presence of granular SPAG5 protein expression was reported in 16% of the post neoadjuvant chemotherapy residual cancerous tissue. In cases that has received HER2 target therapy. The presence of the granules of SPAG5 protein was associated with increase in the risk of relapse at 5 years follow up compared to the absence of granular SPAG5 protein expression either in ER negative or ER positive breast cancer (see Figure 57-59).

Example 75 - Cox regression analysis for 5-year relapse free survival showed that the presence of the granules of SPAG5 protein expression in the post neoadjuvant chemotherapy samples was an independent biomarker for predicting 5- relapse free survival.

| Variables | HR | 95% CI | | P value |
|---|---|---|---|---|
| | | Lower | Upper | |
| SPAG5 post NACT (high) | 2.58 | 1.19 | 5.63 | 0.017* |
| Tumour volume reduction (MRI measurements >30%) | 0.38 | 0.16 | 0.89 | 0.026* |
| Lymphovascular invasion | 2.92 | 1.33 | 6.40 | 0.008* |
| Fibrosis or evidence of response | 0.41 | 0.19 | 0.91 | 0.029 |
| Histological grade (high grade) | 2.62 | 1.19 | 5.74 | 0.016* |

**Summary**

✔ SPAG5 protein overexpression level in the tumour tissue at the time of surgery predicts higher death, relapse and or distant relapse risks at 5 and or 10 years of follow up after removing the primary tumour surgically for breast cancer patients particularly the ER+ breast cancer patients.

✔ SPAG5 protein overexpression level in the tumour tissue specimen at the time of surgery predicts lower death, relapse and or distant relapse risks at 5 years of follow up after removing the primary tumour surgically and receiving adjuvant anthracycline based combination chemotherapy for ER negative breast cancer patients.

✔ SPAG5 protein overexpression level in the tumour tissue biopsy at the time of diagnosis predicts higher pathological complete response rate (complete disappearance of tumour cells in both surgically removed specimens of the breast and lymph node) after receiving the preoperative anthracycline based combination chemotherapy with or without taxane and with or without Trastuzumab chemotherapy for breast cancer patients either ER positive or ER negative breast cancer patients.

✔ SPAG5 protein overexpression level in the tumour tissue specimen at the time of surgery predicts lower death and relapse risks at 5 years of follow up after removing the tumour surgically and receiving cisplatin with or without taxane for ovarian cancer patients.

✔ SPAG5 protein overexpression in the tumour tissue specimen at the time of surgery predicts higher response and lower relapse risk at 5 years of follow up after preoperative cisplatin with or without taxane and removing the tumour surgically for gastric cancer patients.

✔ SPAG5 protein overexpression in the tumour tissue specimen at the time of surgery predicts lower response and higher relapse risk at 5 years of follow up after removing the tumour surgically and receiving adjuvant 5-Flu chemotherapy for gastric cancer patients.

✔ SPAG5 protein overexpression level in the tumour tissue specimen at the time of surgery predicts lower death risk at 5 years of follow up after removing the tumour surgically and receiving cisplatin with or without taxane for colon cancer patients.

✔ SPAG5 protein overexpression level in the tumour tissue specimen at the time of surgery predicts higher death risk at 5 years of follow up after removing the tumour surgically and receiving radiotherapy for rectal cancer patients.

✔ SPAG5 protein overexpression level in the tumour tissue specimen at the time of surgery predicts higher death risk at 5 years of follow up after removing the tumour surgically for lung cancer patients.

✔ SPAG5 mRNA overexpression were independent predictive biomarker for poor survival, relapse and distant relapse risks in ER+ breast cancer.

✔ SPAG5 mRNA overexpression was an independent predictive biomarkers for response to anthracycline based combination chemotherapy with or without taxane and with or without Trastuzumab in ER positive breast cancer.

✔ SPAG5 mRNA overexpression were predictive biomarker for improved relapse risk and response to cisplatin with or without taxane in ovarian cancer

✔ SPAG5 mRNA overexpression were predictive biomarker for improved relapse risk and response to preoperative cisplatin with or without taxane followed by surgery in gastric cancer.

✔ SPAG5 mRNA overexpression were predictive biomarker for higher death and relapse risks of gastric cancer that treated with 5-FU based adjuvant chemotherapy.

✔ The analysis revealed that SPAG5 mRNA overexpression were predictive biomarkers for higher death and relapse risks in Lung cancer.

✔ SPAG5 mRNA overexpression was predictive biomarker for higher death and relapse risks in hepatocellular cancer.

**Claims**

1. A method for determining if an ER+ breast cancer of a subject is expected to respond to endocrine therapy or chemotherapy, the method comprising:

   (i) determining if a cell in a sample of the ER+ breast cancer expresses granular SPAG5 protein; or
   (ii) determining if cells in a sample of the ER+ breast cancer overexpress a *SPAG5* gene;

   wherein if the sample does not comprise a cell that expresses granular SPAG5 protein and/or if cells in the sample do not overexpress *SPAG5* transcript, the subject has a cancer that is expected to respond to endocrine therapy, and
   wherein if the sample comprises a cell that expresses granular SPAG5 protein and/or if cells in the sample overexpress the *SPAG5* transcript, the subject has a cancer that is expected to respond to chemotherapy, wherein the chemotherapy is one or more selected from the group consisting of: cisplatinum, taxane, cyclophosphamide, methotrexate, 5-fluorouracil, an anthracycline, epirubicin, doxorubicin and anthracycline-based combination chemotherapy, wherein the endocrine therapy is tamoxifen and/or an aromatase inhibitor.

2. A method according to claim 1, wherein if the ER+ sample does not comprise a cell that expresses granules of SPAG5 protein and/or cells in the sample do not overexpress the *SPAG5* transcript, the cancer is expected to be resistant to chemotherapy.

3. A method according to claim 1 or claim 2, wherein if the ER+ sample comprises a cell that expresses granules of SPAG5 protein and/or if cells in the sample overexpress the *SPAG5* transcript, the cancer is expected to be resistant to endocrine therapy.

4. An endocrine therapy for use in treating an ER+ breast cancer in a subject, wherein the cancer cells of the cancer do not comprise granules of SPAG5 protein or do not overexpress *SPAG5* transcript, wherein the endocrine therapy is tamoxifen and/or an aromatase inhibitor.

**Patentansprüche**

1. Verfahren zur Bestimmung, ob ein ER+-Brustkrebs bei einem Individuum voraussichtlich auf eine endokrine Therapie oder Chemotherapie anspricht, wobei das Verfahren Folgendes umfasst:

   (i) Bestimmen, ob eine Zelle in einer Probe des ER+-Brustkrebses granulöses SPAG5-Protein exprimiert; oder
   (ii) Bestimmen, ob Zellen in einer Probe des ER+-Brustkrebses ein *SPAG5-Gen* überexprimieren;

   wobei, falls die Probe keine Zelle umfasst, die granulöses SPAG5-Protein exprimiert, und/oder falls Zellen in der Probe *SPAG5*-Transkript nicht überexprimieren, bei dem Individuum ein Brustkrebs vorliegt, der voraussichtlich auf eine endokrine Therapie anspricht, und
   wobei, falls die Probe eine Zelle umfasst, die granulöses SPAG5-Protein exprimiert, und/oder falls Zellen in der Probe das *SPAG5*-Transkript überexprimieren, bei dem Individuum ein Brustkrebs vorliegt, der voraussichtlich auf eine Chemotherapie anspricht, wobei es sich bei der Chemotherapie um eine oder mehrere ausgewählt aus der Gruppe bestehend aus Cisplatin, Taxan, Cyclophosphamid, Methotrexat, 5-Fluoruracil, einem Anthracyclin, Epirubicin, Doxorubicin und einer Kombinationschemotherapie auf Anthracyclinbasis handelt, wobei es sich bei der endokrinen Therapie um Tamoxifen und/oder einen Aromatase-Inhibitor handelt.

2. Verfahren nach Anspruch 1, wobei, falls die Probe keine Zelle umfasst, die granulöses SPAG5-Protein exprimiert, und/oder Zellen in der Probe das *SPAG5*-Transkript nicht überexprimieren, der Krebs voraussichtlich resistent gegen eine Chemotherapie ist.

**3.** Verfahren nach Anspruch 1 oder Anspruch 2, wobei, falls die Probe eine Zelle umfasst, die granulöses SPAG5-Protein exprimiert, und/oder falls Zellen in der Probe das *SPAG5*-Transkript überexprimieren, der Krebs voraussichtlich resistent gegen eine endokrine Therapie ist.

**4.** Endokrine Therapie zur Verwendung beim Behandeln eines ER+-Brustkrebses bei einem Individuum, wobei die Krebszellen des Krebses kein Granulat von SPAG5-Protein umfassen oder *SPAG5*-Transkript nicht überexprimieren, wobei es sich bei der endokrinen Therapie um Tamoxifen und/oder einen Aromatase-Inhibitor handelt.

**Revendications**

**1.** Procédé pour la détermination du fait qu'un cancer du sein ER+ d'un sujet est censé répondre ou pas à une thérapie endocrinienne ou une chimiothérapie, le procédé comprenant :

(i) la détermination du fait qu'une cellule dans un échantillon du cancer du sein ER+ exprime ou pas une protéine SPAG5 granulaire ; ou
(ii) la détermination du fait que des cellules dans un échantillon du cancer du sein ER+ surexpriment ou pas un gène de *SPAG5 ;*

dans lequel, si l'échantillon ne comprend pas une cellule qui exprime une protéine SPAG5 granulaire et/ou si des cellules dans l'échantillon ne surexpriment pas un transcrit de *SPAG5,* le sujet à un cancer qui est censé répondre à une thérapie endocrinienne, et
dans lequel, si l'échantillon comprend une cellule qui exprime une protéine SPAG5 granulaire et/ou si des cellules dans l'échantillon surexpriment le transcrit de *SPAG5,* le sujet a un cancer qui est censé répondre à une chimiothérapie,
la chimiothérapie étant l'une ou plusieurs choisies dans le groupe constitué par : une chimiothérapie au cisplatine, au taxane, au cyclophosphamide, au méthotrexate, au 5-fluorouracile, à une anthracycline, à l'épirubicine, à la doxorubicine et une chimiothérapie combinée à base d'anthracycline,
la thérapie endocrinienne étant le tamoxifène et/ou un inhibiteur d'aromatase.

**2.** Procédé selon la revendication 1, dans lequel si l'échantillon ER+ ne comprend pas une cellule qui exprime des granules de protéine SPAG5 et/ou des cellules dans l'échantillon ne surexpriment pas le transcrit de *SPAG5,* le cancer est censé être résistant à une chimiothérapie.

**3.** Procédé selon la revendication 1 ou la revendication 2, dans lequel si l'échantillon ER+ comprend une cellule qui exprime des granules de protéine SPAG5 et/ou si des cellules dans l'échantillon surexpriment le transcrit de *SPAG5,* le cancer est censé être résistant à une thérapie endocrinienne.

**4.** Thérapie endocrinienne pour une utilisation dans le traitement d'un cancer du sein ER+ chez un sujet, les cellules cancéreuses du cancer ne comprenant pas de granules de protéine SPAG5 ou ne surexprimant pas de transcrit de *SPAG5,* la thérapie endocrinienne étant le tamoxifène et/ou un inhibiteur d'aromatase.

## Figure 1

## Figure 2

## Figure 3

## Figure 4

## Figure 5A and 5B

## Figure 6

Early stage breast cancer

**Figure 7**

**Figure 8**

ER+ Early stage breast cancer

**Figure 9**

ER positive QFU

Survival Probability (y-axis)
Breast cancer specific survival (years) (x-axis)

Low SPAG5 (=259)

High SPAG5 (=28)

Log rank= p<0.033
High SPAG5 (+) vs. Low SPAG5 (-)
HR (CI 95%) = 2.57 (1.49-4.42), p=0.023

## Figure 10

**ER positive NUH ESBC subgroup (n=2253)**

SPAG5- (n=1935)

SPAG5+ (n=318)

**SPAG5 protein**

**Log rank=12.76, p<0.0001**
**High SPAG5 (+) vs. Low SPAG5 (-)**
**HR (CI 95%) = 1.52 (1.21-1.92), p <0.0001**

Survival

DRFS (Years)

**Figure 11**

ER+ Lymph node negative NUH-ESBC cohort with NO systemic adjuvant therapy (n=655)

SPAG5- (n=596)

SPAG5+ (n=59)

**SPAG5 protein**

Log rank=10.33, p=0.001
High SPAG5 (+) vs. Low SPAG5 (-)
HR (CI 95%) = 2.95 (1.48-5.88), p =0.002

DFRFS (Years)

**Figure 12**

ER+ Lymph node negative NUH-ESBC cohort with 5-year adjuvant endocrine therapy but no chemotherapy (n=347))

SPAG5- (n=287)

SPAG5+ (n=60)

Log rank=14.12, p<0.0001
High SPAG5 (+) vs. Low SPAG5 (-)
HR (CI 95%) = 2.52 (1.53-4.16), p <0.0001

DRFS (Years)

## Figure 13

ER+ Lymph node negative NUH-ESBC cohort with 5-year adjuvant
endocrine therapy and anthracycline chemotherapy (n=51))

SPAG5+ (n=13)

SPAG5- (n=37)

Log rank=1.18, p=0.276
High SPAG5 (+) vs. Low SPAG5 (-)
HR (CI 95%) = 0.33 (0.04-2.67), p =0.302

DRFS (Years)

## Figure 14

ER+ Lymph node positive NUH-ESBC cohort with 5-year adjuvant
endocrine therapy but no chemotherapy (n=373)

SPAG5- (n=317)

SPAG5+ (n=56)

SPAG5 protein

Log rank=8.57, p=0.003
High SPAG5 (+) vs. Low SPAG5 (-)
HR (CI 95%) = 2.24 (1.29-3.90), p =0.004

DRFS (Years)

## Figure 15

**ER+ Lymph node positive NUH-ESBC cohort with 5-year adjuvant endocrine therapy and CMF chemotherapy (n=70)**

## Figure 16

ER+ Lymph node positive NUH-ESBC cohort with 5-year adjuvant endocrine therapy and anthracycline based chemotherapy (n=225)

SPAG5- (n=172)

SPAG5+ (n=53)

SPAG5 protein

Log rank=0.00, p=1.00
High SPAG5 ( +) vs. Low SPAG5 (-)
HR (CI 95%) = 1.00 (0.61-1.64), p =1.00

**Figure 17A**

**Figure 17B**

## Figure 17C

Pathological complete response (pCR) to preoperative anthrycyline based combination therapy (AC) alone, with taxane (AC+T) or with Taxane and Herceptin (AC+T+H) of ER positive locally advanced breast cancer

## Figure 18

**LN- adjuvant therapy naive (MC-AT (n=455))**

SPAG5- (n=240)

SPAG5+ (n=205)

*SPAG5 mRNA*

**Log rank=32.68, p<0.0001**
*High SPAG5 (+)* vs. Low *SPAG5 (-)*
**HR (CI 95%) = 2.77 (1.92-3.99), p<0.0001**

Survival

DRFS (Years)

## Figure 19

**LN- ET alone (MC-AT (n=400))**

SPAG5- (n=255)

SPAG5+ (n=145)

*SPAG5 mRNA*

Log rank=18.54, p<0.0001
*High SPAG5 (+)* vs. Low *SPAG5 (-)*
HR (CI 95%) = 2.63 (1.66-4.16), p<0.0001

Survival

DRFS (Years)

**Figure 20**

LN-/SPAG5- transcript (AT-MCC (n=584))

*ET+ACT* (n=89)

*No Adjuvant therapy* (n=240)

*ET alone* (n=255)

*ET+ACT* vs. No adjuvant therapy
HR (CI 95%) = 0.99 (0.62-1.57), p =0.966
*ET+ACT* vs. ET alone
HR (CI 95%) = 0.80 (0.37-1.72), p=0.57

DRFS (Years)

**Figure 21**

LN negative /SPAG5 overexpression transcript

*Tamoxifen + anthracycline* (n=51)

*Tamoxifen alone* (n=145)

*No systemic Adjuvant therapy* (n=205)

*ET+ACT* vs. No adjuvant therapy
HR (CI 95%) = 0.28 (0.11-0.68), p =0.005
*ET+ACT* vs. ET alone
HR (CI 95%) = 0.34 (0.14-0.87), p=0.028

DRFS (Years)

## Figure 22

**LN positive Tamoxifen alone**

## Figure 23

**LN positive received Tamoxifen + anthracycline chemotherapy (n=319)**

## Figure 24

### LN+/SPAG5 overexpression transcript (n=324))

*Tamoxifen +anthracycline chemotherapy* (n=135)

*Tamoxifen alone* (n=189)

Log rank= 15.51, p<0.0001
*ET+ACT* vs. ET alone
HR (CI 95%) = 0.43 (0.28-0.66), p<0.0001

## Figure 25

**LN+/ lowSPAG5- transcript (n=376)**

DRFS (Years)

## Figure 26A

**SPAG5 transcript expression and clinical response to neoadjuvant endocrine therapy**

## Figure 26B

**SPAG5 transcript expression and clinical response to neoadjuvant endocrine therapy**

## Figure 27

SPAG5 transcript expression and the pathological complete response (pCR) after pre-operative anthracycline based chemotherapy (AC), AC+Taxane (T) or AC+T+ Herceptin (H) chemotherapy in ER+ BC

**Figure 28**

## Figure 29

## Figure 30

Overall Cancer Specific Survival (months)

**Figure 31**

**Figure 32**

**Figure 33**

**Figure 34**

**Figure 35**

Log rank=6.84, p=0.009

Cum Survival

Low expression (n=72)
High expression (n=24)

Gastric Specific survival (months)

**Figure 36**

Log rank=5.11, p=0.024

Cum Survival

Low expression (n=72)
High expression (n=24)

Progression Free survival (months)

## Figure 37

**Figure 38**

**Figure 39**

**Figure 40**

**Figure 41**

## Figure 42

Log rank=5.86, p=0.016

Low expression (n=82)
High expression (n=83)

Colon cancer Specific survival (months)

## Figure 43

Log rank=7.94, p=0.005

Low expression (n=40)
High expression (n=59)

Rectal Cancer specific survival (months)

**Figure 44**

HER2+ early stage breast cancer

No granular SPAG5 (-) (n=941)

Granular SPAG5+ staining (n=225)

*SPAG5 protein*

Log rank=7.15, p=0.007
High SPAG5 (+) vs. Low SPAG5 (-)
HR (CI 95%) = 1.43 (1.10-1.87), p=0.008

Cum Survival

Distant Metastases Free Survival (months)

## Figure 45

**HER2+ early stage breast cancer treated with anthracycline based chemotherapy**

*Granular SPAG5+ staining* (n=175)

*No granular* SPAG5 (-) (n=100)

SPAG5 protein

Log rank=9.3, p=0.002
High SPAG5 (+) vs. Low SPAG5 (-)
HR (CI 95%) = 0.51 (0.33-0.79), p=0.003

Distant Metastases Free Survival (months)

**Figure 46**

HER2+ early stage breast cancer treated with
anthracycline based chemotherapy plus Trastuzumab

## Figure 47

### HER2+ early stage breast cancer

*low* SPAG5 transcript (-) (n=70)

*High SPAG5+ transcript*

SPAG5 transcript

Log rank=19.6, p<0.0001
High SPAG5 (+) vs. Low SPAG5 (-)
HR (CI 95%) = 3.4 (1.90-6.14), p<0.0001

Cum Survival

Distant relapse free survival (years)

## Figure 48

**HER2+ early stage breast cancer**

*High SPAG5+ transcript* (n=88)

*Low* SPAG5 transcript (-) (n=49)

*SPAG5 transcript*

Log rank=5.49, p=0.019
High SPAG5 (+) vs. Low SPAG5 (-)
HR (CI 95%) = 0.45 (0.22-0.89), p=0.023

Distant relapse free survival (years)

Cum Survival

**Figure 49**

HER2+ early stage breast cancer

## Figure 50

**HER2 negative expression in pre- neoadjuvant chemotherapy samples (n=361)**

Relapse free survival (months)

## Figure 51

**HER2 negative expression in pre- neoadjuvant chemotherapy samples who converted into HER2 positive in the paired post neoadjuvant chemotherapy samples (n=30)**

**HER2 positive expression in Post neoadjuvant samples who received HER2 target therapy (n= 19)**

**HER2 positive expression in Post neoadjuvant samples who did not receive HER2 target therapy (n= 11)**

Log rank=4.22, p=0.040

Relapse free survival (months)

## Figure 52

HER2 negative expression in pre- neoadjuvant chemotherapy samples who remained
HER2 negative in the paired post neoadjuvant chemotherapy samples (n=343)

No Granular SPAG5 expression in Post neoadjuvant samples (SPAG5-; n= 245)

Granular SPAG5 positive expression in Post neoadjuvant samples (SPAG%+; n= 98)

Log rank=38.18, p<0.0001

Post neoadjuvant chemotherapy: SPAG5 (+) vs., SPAG5 (-)

HR (CI 95%) = 2.96 (2.06-4.26), p<0.0001

Relapse free survival (months)

## Figure 53

HER2 negative / ER negative expression in pre- neoadjuvant chemotherapy samples who remained HER2 negative in the paired post neoadjuvant chemotherapy samples (n=123)

## Figure 54

HER2 negative / ER positive expression in pre- neoadjuvant chemotherapy samples who remained HER2 negative in the paired post neoadjuvant chemotherapy samples (n=220)

No Granular SPAG5 expression in Post neoadjuvant samples (SPAG5-; n= 176)

Granular SPAG5 positive expression in Post neoadjuvant samples (SPAG%+; n= 44)

Log rank=39.91.15, p<0.0001

Post neoadjuvant chemotherapy: SPAG5 (+) vs., SPAG5 (-)

HR (CI 95%) = 4.06 (2.53-6.51), p<0.0001

Cum Survival

Relapse free survival (months)

## Figure 55

HER2 positive expression in pre- neoadjuvant chemotherapy samples (n=92)

HER2 negative expression in Post neoadjuvant samples
(cases with HER2 conversion; n= 19)

HER2 negative expression in Post neoadjuvant samples
(cases with NO HER2 conversion status; n= 73)

Log rank=6.04, p=0.025

Cum Survival

Relapse free survival (months)

## Figure 56

HER2 positive expression in both pre- and post-neoadjuvant chemotherapy samples (n=73)

HER2 target therapy (n=64)

No HER2 target therapy (n= 9)

Log rank=13.3, p<0.001

HER2 target therapy vs. No HER2 target therapy

HR (CI 95%) = 0.19 (0.07-0.52), p=0.001

## Figure 57

HER2 positive expression in both pre- and post-neoadjuvant chemotherapy samples that received adjuvant HER2 target therapy for one year (n=64)

## Figure 58

HER2 positive/ ER positive expression in pre- neoadjuvant chemotherapy samples who remained HER2 positive in the paired post neoadjuvant chemotherapy samples (n=33)

No Granular SPAG5 expression in Post neoadjuvant samples (SPAG5-; n= 29)

Granular SPAG5 positive expression in Post neoadjuvant samples (SPAG%+; n= 4)

Log rank=13.09, p<0.0001

Post neoadjuvant chemotherapy: SPAG5 (+) vs., SPAG5 (-)

HR (CI 95%) = 19.86 (2.05-192.62), p=0.010

## Figure 59

HER2 positive / ER negative expression in pre- neoadjuvant chemotherapy samples who remained HER2 negative in the paired post neoadjuvant chemotherapy samples (n=31)

No Granular SPAG5 expression in Post neoadjuvant samples (SPAG5-; n= 26)

Granular SPAG5 positive expression in Post neoadjuvant samples (SPAG%+; n= 5)

Log rank=13.37, p<0.0001

Post neoadjuvant chemotherapy: SPAG5 (+) vs., SPAG5 (-)

HR (CI 95%) = 12.93 (2.27-73.60.12), p=0.004

Relapse free survival (months)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015185219 A

**Non-patent literature cited in the description**

- **THOMPSON et al.** *Nucleic Acids Research,* 1994, vol. 22, 4673-4680

- **THOMPSON et al.** *Nucleic Acids Research,* 1997, vol. 24, 4876-4882